(19) Europäisches Patentamt
European Patent Office
Office européen des brevets

(11) Publication number: 0 472 062 A1

# (12) EUROPEAN PATENT APPLICATION

(21) Application number: 91113384.1

(22) Date of filing: 09.08.91

(51) Int. Cl.5: C07D 499/00, C07D 487/04, C07D 477/00, A61K 31/43

(30) Priority: 10.08.90 JP 212102/90
27.12.90 JP 415862/90
28.12.90 JP 416070/90

(43) Date of publication of application:
26.02.92 Bulletin 92/09

(84) Designated Contracting States:
AT BE CH DE DK ES FR GB GR IT LI LU NL SE

(71) Applicant: SUMITOMO PHARMACEUTICALS COMPANY, LIMITED
2-8, Dosho-Machi 2-Chome, Chuo-Ku
Osaka-Shi Osaka-Fu(JP)

(72) Inventor: Sunagawa, Makoto
29-1, Yamada, Aza-nohata
Itami-shi, Hyogo-ken(JP)
Inventor: Sasaki, Akira
4-2-301, Ryodo-cho
Nishinomiya-shi, Hyogo-ken(JP)
Inventor: Yamaga, Hiroshi
2-1-117, Kuwata-cho
Ibaraki-shi, Osaka-fu(JP)
Inventor: Shinagawa, Hisatoshi
1-12-8, Dekijima, Nishiyodogawa-ku
Osaka-shi, Osaka-fu(JP)
Inventor: Fukasawa, Masatomo
4-27-10, Gotenyama
Takarazuka-shi, Hyogo-ken(JP)
Inventor: Sumita, Yoshihiro
5-4-12-403, Kudo, Ohji-cho, Kitakatsuragi-gun
Nara-ken(JP)

(74) Representative: Vossius & Partner
Siebertstrasse 4 P.O. Box 86 07 67
W-8000 München 86(DE)

## (54) Beta-lactam compounds, and their production and use.

(57) A compound of the formula:

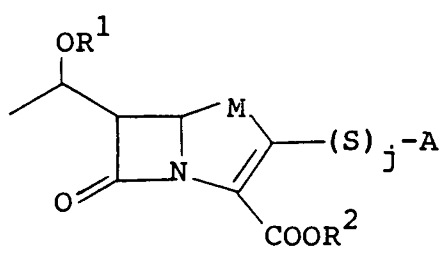

(I)

which is useful as an antimicrobial agent.

EP 0 472 062 A1

The present invention relates to β-lactam compounds, and their production and use. More particularly, it relates to novel penem or carbapenem compounds bearing a catechol or hydroxypyridone moiety, and their production and use.

Among β-lactam compounds showing an excellent antimicrobial spectrum over a wide range of Gram-positive and Gram-negative bacteria, there are known some compounds having a penem or carbapenem skeleton. Of these compounds, imipenem is available on the market, but the appearance of imipenem-resistant bacteria in clinics is already reported. It is thus highly demanded to provide novel β-lactam compounds which exert an antimicrobial activity against imipenem-resistant bacteria, especially against imipenem-resistant strains of Pseudomonas aeruginosa.

As the result of an extensive study, it has now been found that penem or carbapenem compounds having a certain catechol or hydroxypyridone moiety exert a strong antimicrobial activity against various bacteria including imipenem-resistant strains of P. aeruginosa. The present invention is based on this finding.

Accordingly, a basic object of the present invention is to provide a novel β-lactam compound of the formula:

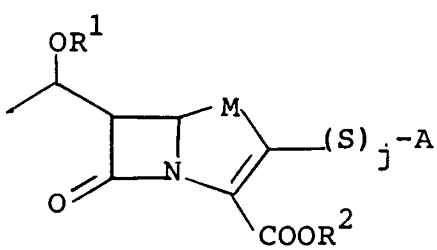

(I)

wherein:

j is an integer of 0 or 1;

$R^1$ is a hydrogen atom, a protective group for hydroxyl or either one of the below-mentioned groups (1) to (3);

$R^2$ is a hydrogen atom, a protective group for carboxyl or a negative charge when A has any moiety with a positive charge;

M is a sulfur atom, a methylene group or a methylene group substituted with lower alkyl; and

A varies depending on $R^1$ and represents the followings:

(i) when $R^1$ is a hydrogen atom or a protective group for hydroxyl, A is either one of the groups (a) to (g):

(a)

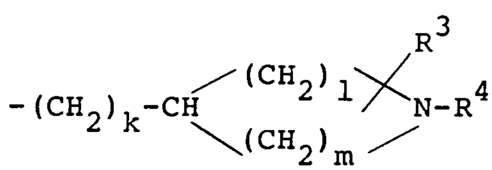

wherein k, l and m are each an integer of 0 to 3 but l and m are not simultaneously 0, $R^3$ is a hydrogen atom, a lower alkoxycarbonyl group, a carbamoyl group, a mono- or di(lower)-alkylaminocarbonyl group, a cyclic aminocarbonyl group, a lower alkyl group or a substituted lower alkyl group and $R^4$ is either one of the groups (1) to (3):

(1)

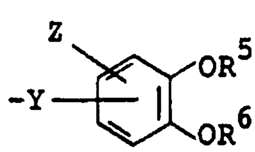

wherein Y is a single bond, a lower alkylene group, a lower alkenylene group, a carbonyl group, a

lower alkylene group having at least one linkage chosen from -CO-, -O- and $-NR^0-$ (in which $R^0$ is a hydrogen atom or a lower alkyl group) in the alkylene chain or a lower alkenylene group having at least one linkage chosen from -CO-, -O- and $-NR^0-$ (in which $R^0$ is as defined above) in the alkenylene chain, $R^5$ and $R^6$ are each a hydrogen atom or a protective group for hydroxyl, Z is a hydrogen atom, a halogen atom, a nitro group, a cyano group, a lower alkoxycarbonyl group, a carbamoyl group or a mono- or di(lower)alkylaminocarbonyl group,

(2)

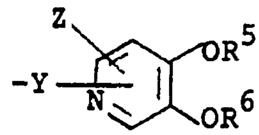

wherein Y, Z, $R^5$ and $R^6$ are each as defined above, and

(3)

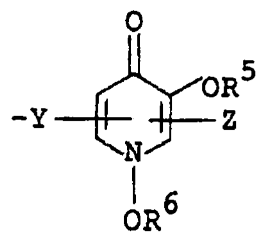

wherein Y, Z, $R^5$ and $R^6$ are each as defined above;

(b)

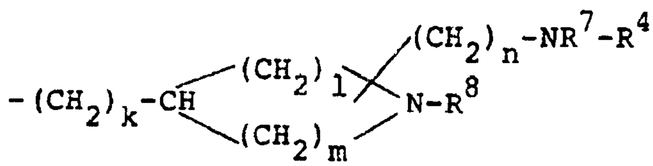

wherein k, l, m and $R^4$ are each as defined above, n is an integer of 0 to 3, $R^7$ is a hydrogen atom, a lower alkyl group or a substituted lower alkyl group and $R^8$ is a hydrogen atom, a protective group for amino, a lower alkyl group or a substituted lower alkyl group;

(c)

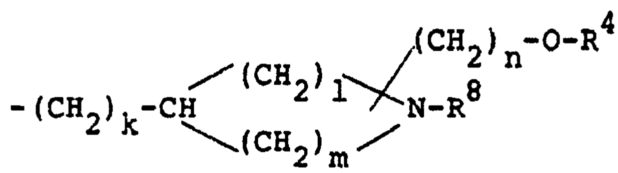

wherein k, l, m, n, $R^4$ and $R^8$ are each as defined above;

(d)

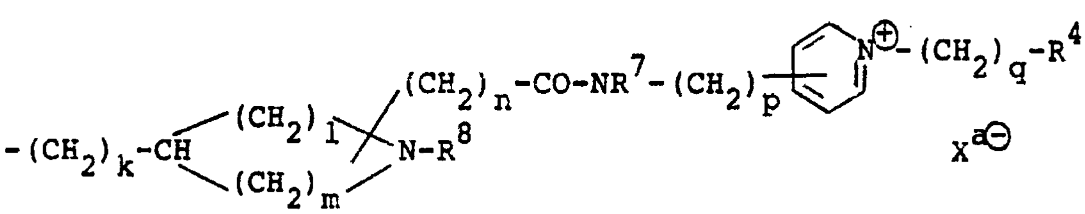

wherein k, l, m, n, $R^4$, $R^7$ and $R^8$ are each as defined above and p is an integer of 0 to 5, q is an integer of 1 to 5 and $X^a$ is an acid residue or an intramolecular COO when $R^2$ is a negative charge;
(e)

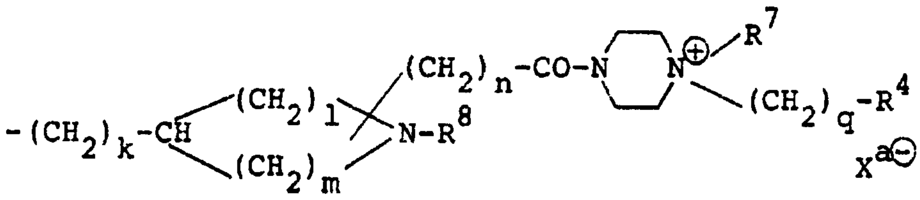

wherein k, l, m, n, q, $R^4$, $R^7$, $R^8$ and $X^a$ are each as defined above;
(f)

$(CH_2)_k$-O-$R^4$

wherein k and $R^4$ are each as defined above;

(g) -$(CH_2)_k$-$NR^7$-$R^4$

wherein k, $R^4$ and $R^7$ are each as defined above;
(ii) when $R^1$ is either one of the groups (1) to (3), A is either one of the groups (h) to (p) or any other organic group:
(h)

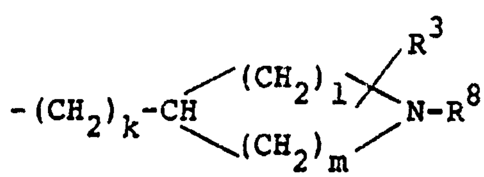

wherein k, l, m, $R^3$ and $R^8$ are each as defined above;
(i)

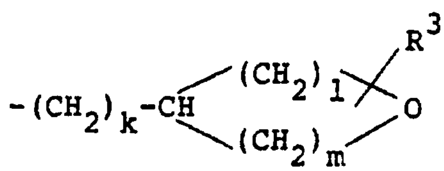

wherein k, l, m and $R^3$ are each as defined above;
(j)

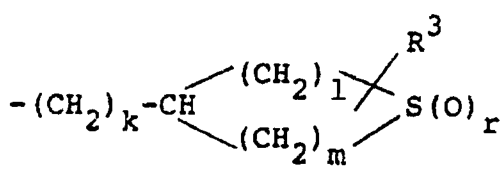

wherein k, l, m and $R^3$ are each as defined above and r is an integer of 0 to 2;
(k)

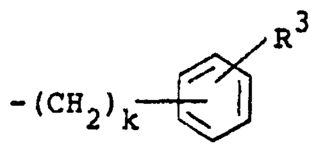

wherein k and $R^3$ are each as defined above;
(l)

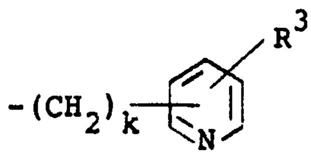

wherein k and $R^3$ are each as defined above;
(m)

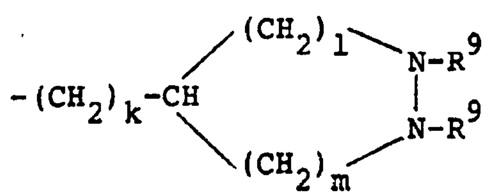

wherein k, l and m are each as defined above and $R^9$ is a hydrogen atom or a protective group for amino;
(n)

$$-(CH_2)_k-R^{10}$$

wherein k is as defined above and $R^{10}$ is a lower alkyl group or a substituted lower alkyl group;
(o)

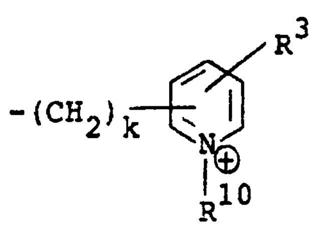

wherein k, $R^3$ and $R^{10}$ are each as defined above; and
(p)

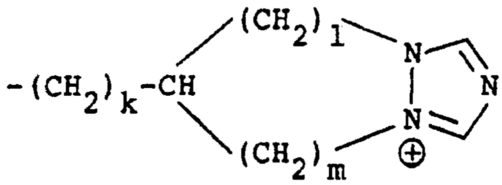

wherein k, l and m are each as defined above, and its salts.

Among various β-lactam compounds which fall within the formula (I), preferred are those wherein $R^2$ is a hydrogen atom or a negative charge in case of A comprising a positive charge, and their salts. More preferred are those wherein $R^1$ is a hydrogen atom and A is either one of the groups (a) to (e), and their salts, and those wherein $R^1$ is either one of the groups (1) to (3), and their salts. The most preferred are those wherein A comprises a pyrrolidine ring, and their salts.

Further, in addition to the groups (a) to (p), A may be any other organic group such as a $C_3$-$C_8$ cycloalkylgroup (e.g. cyclopropyl, cyclobutyl, cyclopentyl), a $C_3$-$C_8$ cycloalkyl group-substituted $C_1$-$C_3$ alkyl group, in which the cycloalkyl group is as defined above, a mono or bicyclic heterocyclic group having 5 to 11 ring atoms, of which 1 to 5 atoms are hetero atoms chosen from nitrogen, oxygen and sulfur (e.g. 2-pyrrolyl, 4-imidazolyl, 2-thiazolyl), a mono or bicyclic heterocyclic group-substituted $C_1$-$C_3$ alkyl group, in which the heterocyclic group is as defined above, etc.

Another object of the invention is to provide a process for preparing the β-lactam compound (I) and its salts. Namely, the β-lactam compound (I) is produced according to the process as set forth below.

(A) When j is 1 and A is either one of the groups (a) to (c), the process comprises reacting a compound of the formula:

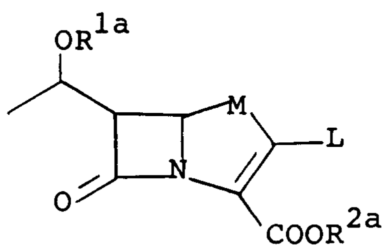

(II)

wherein M is as defined above, $R^{1a}$ is a hydrogen atom or a protective group for hydroxyl, $R^{2a}$ is a protective group for carboxyl and L is a reactive ester group of hydroxyl or a substituted or unsubstituted lower alkylsulfinyl group with a mercaptan compound of the formula:

$A^a$-SH (III-a)

wherein $A^a$ is either one of the groups (a) to (c) to give a β-lactam compound of the formula:

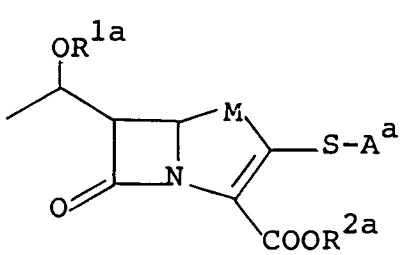

(I-a)

wherein $R^{1a}$, $R^{2a}$, $A^a$ and M are each as defined above, or reacting the compound (II) with a compound of the formula:

$Q^1$-SH (III-b-1)

wherein $Q^1$ is the group (h) to give a compound of the formula:

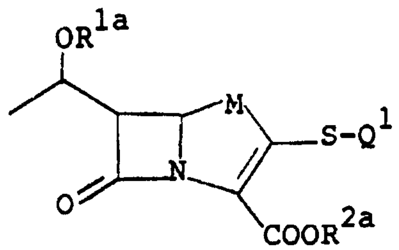

(IV-a)

wherein $R^{1a}$, $R^{2a}$, $Q^1$ and M are each as defined above and, in case of $R^8$ in the group (h) being a protective group for amino, then eliminating the protecting group and reacting the resultant compound with a compound of the formula:

$R^4$-$X^c$ (V)

wherein $R^4$ is as defined above and $X^c$ is an acid residue or an isocyanate group to give the β-lactam compound (I-a);

(B) When j is 1 and A is either one of the groups (d) to (e), the process comprises reacting the compound (II) with a mercaptan compound of the formula:

$Q^2$-SH (III-b-2)

wherein $Q^2$ is either one of the groups (d') and (e') of the formulas:

(d')

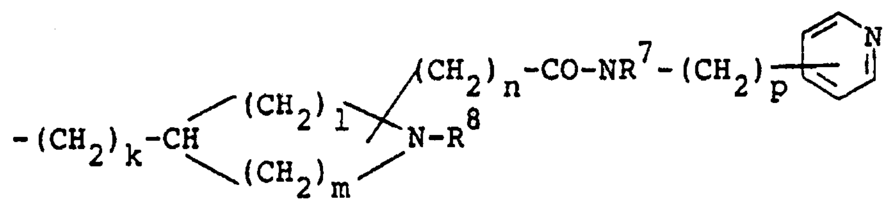

wherein k, l, m, n, p, $R^7$ and $R^8$ are each as defined above and

(e')

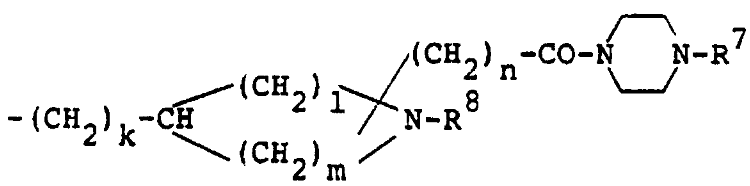

wherein k, l, m, n, $R^7$ and $R^8$ are each as defined above and reacting the resultant compound of the formula:

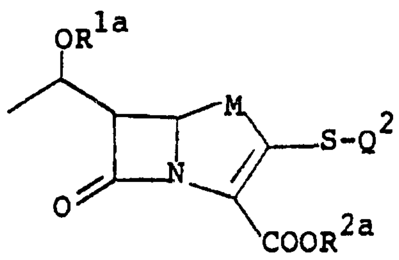

(IV-b)

wherein $R^{1a}$, $R^{2a}$, $Q^2$ and M are each as defined above with a compound of the formula:

$X^b-(CH_2)_q-R^4$ (VI)

wherein q and $R^4$ are each as defined above and $X^b$ is an acid residue to give a β-lactam compound of the formula:

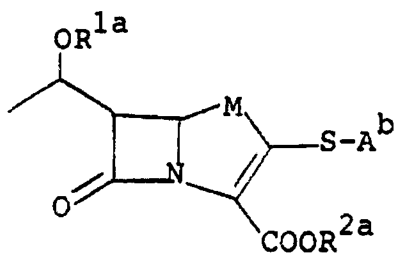

(I-b)

wherein $R^{1a}$, $R^{2a}$ and M are each as defined above and $A^b$ is either one of the groups (d) or (e);
(C) When j is 1 and A is either one of the groups (f) to (g), the process comprises reacting a compound of the formula:

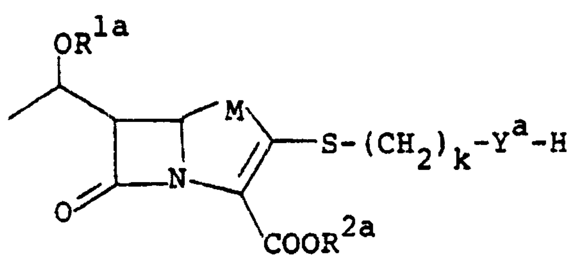

(VII)

wherein k, $R^{1a}$, $R^{2a}$ and M are each as defined above and $Y^a$ is an oxygen atom or $-NR^7$ (in which $R^7$ is as defined above) with a compound of the formula:

$R^4-X^c$ (V)

wherein $R^4$ and $X^c$ are each as defined above to give a β-lactam compound of the formula:

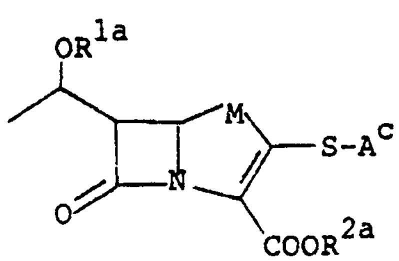

(I-c)

wherein $R^{1a}$, $R^{2a}$ and M are each as defined above and $A^c$ is either one of the groups (f) or (g);
(D) When j is 0 and A is either one of the groups (a) to (c), the process comprises heating a compound of the formula:

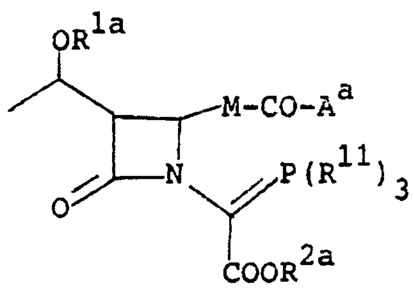

(VIII)

wherein $R^{1a}$, $R^{2a}$, M and $A^a$ are each as defined above and $R^{11}$ is a lower alkyl group, an aryl group or a lower alkoxy group to give a β-lactam compound of the formula:

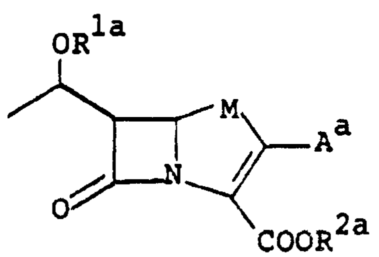

(I-d)

wherein $R^{1a}$, $R^{2a}$, M and $A^a$ are each as defined above;
(E) When j is 0 and A is either one of the groups (d) to (e), the process comprises heating a compound of the formula:

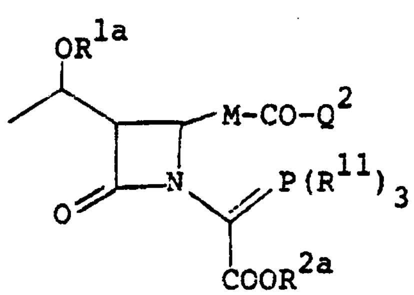

(IX)

wherein $R^{1a}$, $R^{2a}$, $R^{11}$, M and $Q^2$ are each as defined above to give a compound of the formula:

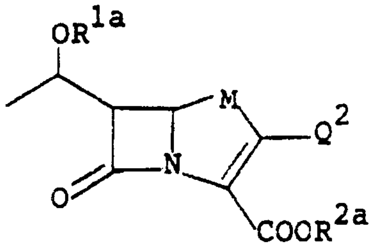

(X)

wherein $R^{1a}$, $R^{2a}$, M and $Q^2$ are each as defined above and reacting the latter with a compound of the formula:

$X^b$-$(CH_2)_q$-$R^4$ (VI)

wherein q, $R^4$ and $X^b$ are each as defined above to give a β-lactam compound of the formula:

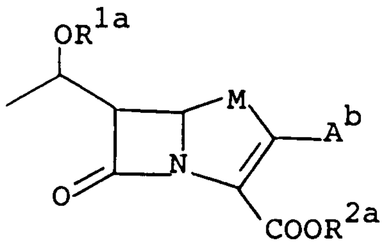

(I-e)

wherein $R^{1a}$, $R^{2a}$, M and $A^b$ are each as defined above;
(F) When j is 0 and A is either one of the groups (f) to (g), the process comprises reacting a compound of the formula:

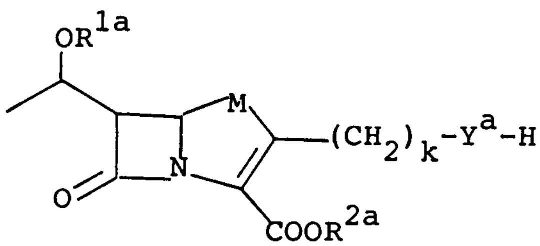

(XI)

wherein k, $R^{1a}$, $R^{2a}$ and M are each as defined above and $Y^a$ is an oxygen atom or -$NR^7$ (in which $R^7$ is as defined above) with a compound of the formula:

$R^4$-$X^c$ (V)

wherein $R^4$ and $X^c$ are each as defined above to give a β-lactam compound of the formula:

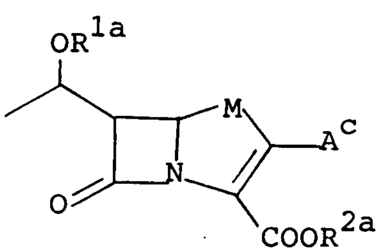

(I-f)

wherein $R^{1a}$, $R^{2a}$ and M are each as defined above and $A^c$ is either one of the groups (f) to (g).
(G) When j is 1 and A is either one of the groups (h) to (n), the process comprises reacting the compound (II) with a mercaptan compound of the formula:

$A^d$-SH (III-c)

wherein $A^d$ is either one of the groups (h) to (n) to give a compound of the formula:

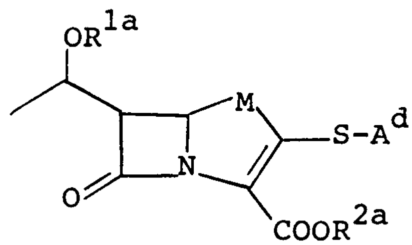

(XII)

wherein $R^{1a}$, $R^{2a}$, M and $A^d$ are each as defined above and, in case of $R^{1a}$ being a protective group for hydroxyl, then eliminating the protecting group and reacting the resultant compound of the formula:

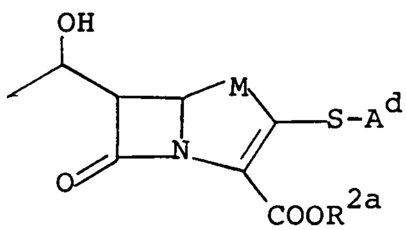

(XIII)

wherein $R^{2a}$, M and $A^d$ are each as defined above with a compound of the formula:

$R^{1b}$-$X^d$ (XIV)

wherein $R^{1b}$ is either one of the groups (1) to (3) and $X^d$ is an acid residue to give a β-lactam compound of the formula:

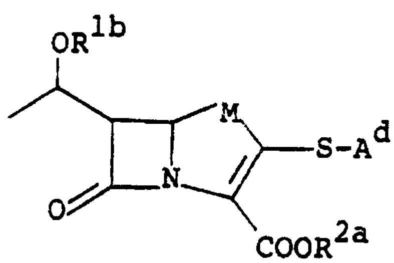

(I-g)

wherein $R^{1b}$, $R^{2a}$, M and $A^d$ are each as defined above.

(H) When j is 1 and A is either one of the groups (o) to (p), the process comprises reacting a compound of the formula:

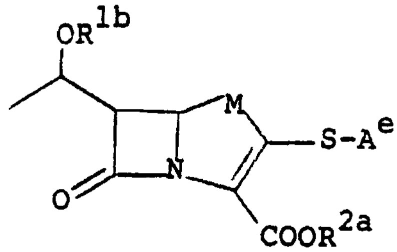

(I-g')

wherein $R^{1b}$, $R^{2a}$ and M are each as defined above and $A^e$ is either one of the above-mentioned groups (l) or (m) with a compound of the formula:

$R^{10}$-$X^e$ (XV)

wherein $R^{10}$ is as defined above and $X^e$ is an acid residue, or eliminating the amino-protecting group represented by $R^9$ and reacting the resultant compound with a $C_1$-$C_5$ alkyl ester of formimidic acid to give a $\beta$-lactam compound of the formula:

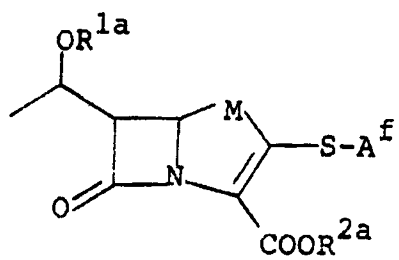

(I-h)

wherein $R^{1a}$, $R^{2a}$ and M are each as defined above and $A^f$ is either one of the groups (o) or (p).

(l) When j is 0 and A is either one of the formulas (h) to (n), the process comprises heating a compound of the formula:

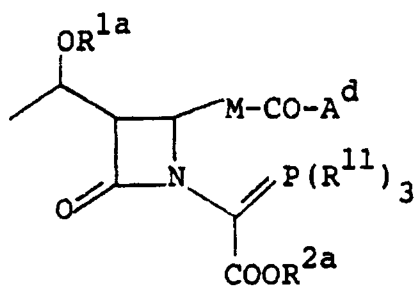

(XVI)

wherein $R^{1a}$, $R^{2a}$, M and $A^d$ are each as defined above and $R^{11}$ is a lower alkyl group or an aryl group or a lower alkoxy group to give a compound of the formula:

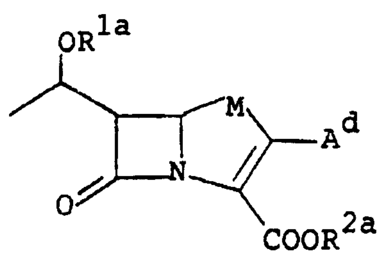

(XVII)

wherein $R^{1a}$, $R^{2a}$, M and $A^d$ are each as defined above and, in case of $R^{1a}$ being a protective group for hydroxyl, eliminating the protecting group, reacting the resultant compound of the formula:

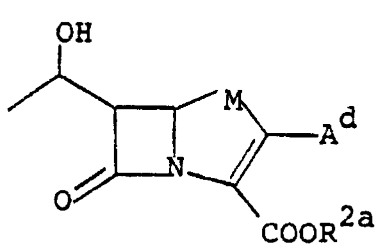

(XVIII)

wherein $R^{2a}$, M and $A^d$ are each as defined above with a compound of the formula:

$R^{1b}$-$X^d$ (XIV)

wherein $R^{1b}$ and $X^d$ are each as defined above to give a $\beta$-lactam compound of the formula:

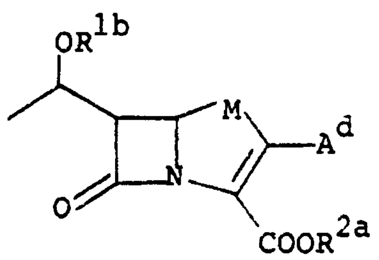

(I-i)

wherein $R^{1b}$, $R^{2a}$, M and $A^d$ are each as defined above.

(J) When j is 0 and A is either one of the groups (o) to (p), the process comprises reacting a compound of the formula:

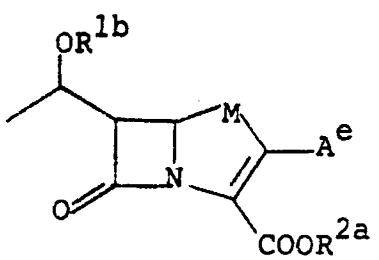

(I-i')

wherein $R^{1b}$, $R^{2a}$ and M are each as defined above and $A^e$ is either one of the groups (l) or (m) with a compound of the formula:

$R^{10}$-$X^e$ (XV)

wherein $R^{10}$ is as defined above and $X^e$ is an acid residue, or eliminating the amino-protecting group represented by $R^9$ and reacting the resultant compound with a $C_1$-$C_5$ alkyl ester of formimidic acid to give a $\beta$-lactam compound of the formula:

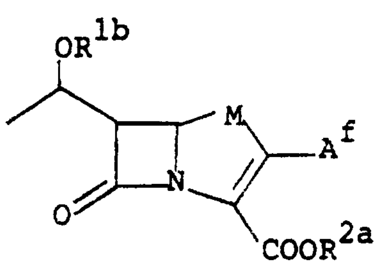

(I-j)

wherein $R^{1b}$, $R^{2a}$ and M are each as defined above and $A^f$ is either one of the groups (o) or (p).

When the $\beta$-lactam compound (I) wherein $R^1$, $R^5$, $R^6$, $R^8$ and $R^9$ are each a hydrogen atom and $R^2$ is a hydrogen atom or a negative charge is desired, the $\beta$-lactam compound (I-a) to (I-i) may be further subjected to reaction for elimination of the hydroxyl-protecting group, the carboxyl-protecting group and/or the amino-protecting group.

The protective group for hydroxyl (i.e. hydroxyl-protecting group) represented by $R^1$, $R^5$, $R^6$ or $R^{1a}$ and the protective group for amino (i.e. amino-protecting group) represented by $R^8$ or $R^9$ in the formulas (I), (I-a), (I-b), (I-c), (I-d), (I-e), (I-f), (I-g), (I-g'), (I-h), (I-i), (I-i'), (I-j), (II), (III-a), (III-b-1), (IIII-b-2), (III-c), (IV-a), (IV-b), (V), (VI), (VII), (VIII), (IX), (X), (XI), (XII), (XIII), (XIV), (XV), (XVI), (XVII) and (XVIII) may be the ones as conventionally used in the related art field. Their preferred examples are $C_1$-$C_5$ alkoxycarbonyl (e.g. t-butyloxycarbonyl), halo($C_1$-$C_5$)alkoxycarbonyl (e.g. 2-iodoethyloxycarbonyl, 2,2,2-trichloroethyloxycarbonyl), substituted or unsubstituted $C_3$-$C_7$ alkenyloxycarbonyl (e.g. allyloxycarbonyl), ar($C_1$-$C_3$)alkyloxycarbonyl

such as phenyl($C_1$-$C_3$)alkyloxycarbonyl (e.g. benzyloxycarbonyl) or substituted phenyl($C_1$-$C_3$)-alkyloxycarbonyl (e.g. p-methoxybenzyloxycarbonyl, o-nitrobenzyloxycarbonyl, p-nitrobenzyloxycarbonyl) and tri($C_1$-$C_5$)alkylsilyl (e.g. trimethylsilyl, t-butyldimethylsilyl).

Preferred examples of the protective group for hydroxyl (e.g. hydroxyl-protective group) represented by $R^5$ or $R^6$ may be, in addition to the above, a straight or branched $C_1$-$C_5$ alkyl group (e.g. methyl, t-butyl), a $C_2$-$C_5$ alkoxymethyl group (e.g. methoymethyl, ethoxymethyl, isobutoxymethyl), a substituted or unsubstituted phenyl($C_1$-$C_3$)alkyl group (e.g. benzyl, p-methoxybenzyl, o-nitrobenzyl, p-nitrobenzyl) and a $C_3$-$C_7$ alkenyl group (e.g. allyl, 2-methylallyl, 3-methylallyl).

The protective group for carboxyl (e.g. carboxyl-protective group) represented by $R^2$ or $R^{2a}$ may also be the one as conventionally used. Preferred examples are straight or branched $C_1$-$C_5$ alkyl (e.g. methyl, ethyl, isopropyl, t-butyl), halo($C_1$-$C_5$)alkyl (e.g. 2-iodoethyl, 2,2,2-trichloroethyl), $C_1$-$C_5$ alkoxymethyl (e.g. methoxyethyl, ethoxymethyl, isobutoxymethyl), $C_1$-$C_5$ aliphatic acyloxymethyl (e.g. acetoxymethyl, propionyloxymethyl, butyryloxymethyl, pivaloyloxymethyl), 1-($C_1$-$C_5$)alkoxycarbonyloxyethyl (e.g. 1-ethoxycarbonyloxyethyl), ar($C_1$-$C_3$)alkyl such as phenyl($C_1$-$C_3$) alkyl (e.g. benzyl) or substituted phenyl($C_1$-$C_3$)alkyl(e.g. p-methoxybenzyl, o-nitrogenzyl, p-nitrobenzyl), $C_3$-$C_7$ alkenyl (e.g. allyl, 2-methylallyl, 3-methylallyl), benzhydryl and phthalidyl.

The lower alkyl group optionally present on the methylene group represented by M may be, for instance, $C_1$-$C_5$ alkyl (e.g. methyl, ethyl, n-propyl, isopropyl, n-butyl, n-pentyl). The lower alkyl group represented by $R^0$, $R^3$, $R^7$, $R^8$, $R^{10}$ or $R^{11}$ may be also $C_1$-$C_5$ alkyl (e.g. methyl, ethyl, n-propyl, isopropyl, n-butyl, n-pentyl). The lower alkoxy group represented by $R^{11}$ may be, for instance, $C_1$-$C_5$ alkoxy (e.g. methoxy, ethoxy, n-propoxy, isopropoxy, n-butoxy, n-pentoxy). Examples of the aryl group include phenyl, o-tolyl and p-tolyl.

Examples of the substituted lower alkyl group represented by $R^3$, $R^7$ or $R^8$ are hydroxy($C_1$-$C_5$)alkyl (e.g. hydroxymethyl, 1-hydroxyethyl, 2-hydroxyethyl), $C_1$-$C_5$ alkoxy($C_1$-$C_5$)alkyl (e.g. metoxyethyl, 1-methoxyethyl, 2-ethoxyethyl), amino($C_1$-$C_5$)alkyl (e.g. aminomethyl), N-($C_1$-$C_5$)alkylamino($C_1$-$C_5$)alkyl (e.g. N-methylaminomethyl, N-methylaminoethyl), N,N-di($C_1$-$C_5$)alkylamino($C_1$-$C_5$)alkyl (e.g. N,N-dimethylaminomethyl, N,N-dimethylaminoethyl), aminocarbonyl($C_1$-$C_5$)alkyl (e.g. carbamoylmethyl, carbamoylethyl), N-($C_1$-$C_5$)alkylaminocarbonyl($C_1$-$C_5$)alkyl (e.g. methylaminocarbonylmethyl) and N,N-di($C_1$-$C_5$)alkylaminocarbonyl($C_1$-$C_5$)alkyl (e.g. dimethylaminocarbonylmethyl, 2-(dimethylaminocarbonyl)ethyl). The substituted lower alkyl group represented by $R^{10}$ may be, for instance, a $C_1$-$C_5$ alkyl group bearing a substituent such as carboxyl, lower alkanoyl (e.g. acetyl, propionyl), carbamoyl, lower alkylaminocarbonyl (e.g. methylaminocarbonyl), di(lower)alkylaminocarbonyl (e.g. dimethylaminocarbonyl), cyano, lower alkoxy (e.g. methoxy, ethoxy), lower alkanoylamino (e.g. acetylamino), iminomethylamino or hydroxyl, and its specific examples are carboxylmethyl, acetylmethyl, propionylmethyl, carbamoylmethyl, N-methylaminocarbonylmethyl, N,N-dimethylaminocarbonylmethyl, 2-cyanomethyl, 2-methoxyethyl, 2-ethoxyethyl, 2-carboxyethyl, 2-hydroxyethyl, 2-carbamoylethyl, 2-N-methylaminocarbonylethyl, 2-N,N-dimethylaminocarbonylethyl, 3-carboxylpropyl, 4-hydroxybutyl, 5-hydroxypentyl, 2-(acetylamino)ethyl and 2-(iminomethylamino)ethyl.

The lower alkoxycarbonyl group represented by $R^3$ may be, for instance, $C_2$-$C_5$ alkoxycarbonyl (e.g. methoxycarbonyl, ethoxycarbonyl, n-propyloxycarbonyl, t-butyloxycarbonyl). The mono(lower)-alkylaminocarbonyl or di(lower)alkylaminocarbonyl group may be, for instance, $C_1$-$C_5$ alkylaminocarbonyl or di($C_1$-$C_5$)alkylaminocarbonyl (e.g. methylaminocarbonyl, dimethylaminocarbonyl, ethylaminocarbonyl, methylethylaminocarbonyl, diethylaminocarbonyl). The cyclic aminocarbonyl group includes 3 to 6-membered cyclic aminocarbonyl such as aziridinocarbonyl, azetidinocarbonyl, pyrrolidinocarbonyl and piperidinocarbonyl.

The halogen atom represented by Z includes fluorine, chlorine, bromine, iodine, etc. The lower alkoxycarbonyl group may be $C_1$-$C_5$ alkoxycarbonyl (e.g. methoxycarbonyl, ethoxycarbonyl, n-propyloxycrbonyl, t-butyloxycarbonyl). The mono(lower)alkylaminocarbonyl or di(lower)alkylaminocarbonyl group may be $C_1$-$C_5$ alkylaminocarbonyl (e.g. methylaminocarbonyl, ethylaminocarbonyl) or di($C_1$-$C_5$)-alkylaminocarbonyl (e.g. dimethylaminocarbonyl, methylethylaminocarbonyl, diethyiaminocarbonyl).

The reactive ester group of hydroxyl group represented by L may be, for instance, a substituted or unsubstituted arylsulfonic ester (e.g. benzenesulfonate, p-toluenesulfonate, p-nitrobenzenesulfonate, p-bromobenzenesulfonate), $C_1$-$C_5$ alkanesulfonic ester (e.g. methanesulfonate, ethanesulfonate), halo($C_1$-$C_5$)-alkanesulfonic ester (e.g. trifluoromethanesulfonate), diarylphosphoric ester (e.g. diphenylphosphate) or an ester with hydrogen halide (e.g. chloride, bromide, iodide).

Preferred are p-toluenesulfonate, methanesulfonate, diphenylphosphate, etc. The substituted or unsubstituted lower alkylsulfinyl group includes $C_1$-$C_5$ alkylsulfinyl (e.g. methylsulfinyl, ethylsulfinyl, n-propylsulfinyl, n-butylsulfinyl, 2-(acetylamino)ethylsulfinyl).

Examples of the acid residue represented by $X^a$, $X^b$, $X^c$, $X^d$ or $X^e$ are an inorganic acid residue such as halogen (e.g. chlorine, bromine, fluorine, iodine) and an organic acid residue (e.g. benzenesulfonyloxy, p-toluenesulfonyloxy, methanesulfonyloxy, trifluoromethanesulfonyloxy). In case of a carbonyl group being present at the terminal position in Y, $X^c$ or $X^d$ may additionally be $C_2$-$C_6$ alkoxycarbonyloxy such as ethoxycarbonyloxy or isopropyloxycarbonyloxy.

The lower alkylene group and the lower alkenylene group in Y may be $C_1$-$C_4$ alkylene (e.g. methylene, ethylene, propylene, butylene) and $C_2$-$C_4$ alkenylene (e.g. vinylene, propenylene, butynylene), respectively. When Y indicates a lower alkylene group having at least one linkage chosen from -CO-, -O- and $-NR^0-$ in the alkylene chain, its specific examples are $-COCH_2-$, $-COCH_2CH_2-$, $-CH_2CO-$, $-CH_2COCH_2-$, $-CH_2CONH-$, $-CH_2CONCH_3-$, $-CH_2CONHCH_2-$, $-CH_2CH_2CONH-$, $-CH_2CH_2CONCH_3-$, $-CH_2CH_2CONCH_3CH_2-$, $-COOCH_2-$, $-CH_2COO-$ and $-CH_2COOCH_2CH_2-$. Likewise, examples of the lower alkenylene group having at least one linkage chosen from -CO-, -O- and $-NR^0-$ are $-COCH=CH-$, $-CH_2COCH=CH-$, $-CH_2CONHCH=CHCH_2-$, $-CH_2CONCH_3CH=CHCH_2-$, $-COOCH_2CH=CH-$ and $-CH_2COOCH=CH-$.

The β-lactam compound (I) may be either in a free form or in a salt (preferably non-toxic salt) form. Examples of the salt are inorganic base salts (e.g. sodium, potassium, calcium, magnesium, ammonium), organic base salts (e.g. triethylammonium, pyridinium, diosopropylammonium), inorganic acid addition salts (e.g. hydrochloride, sulfate, phosphate) and organic acid addition salts (e.g. formate, acetate, methanesulfonate, benzenesulfonate).

Production of the β-lactam compounds (I), i.e. (I-a) to (I-l), according to the invention are summarizedly shown in the following reaction scheme:

Process (A)

1)

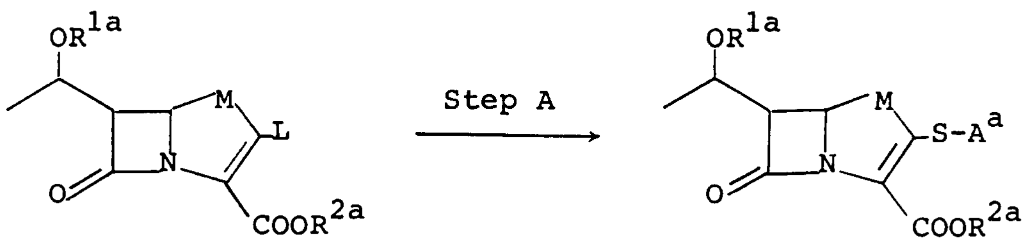


(II) (I-a)

2)

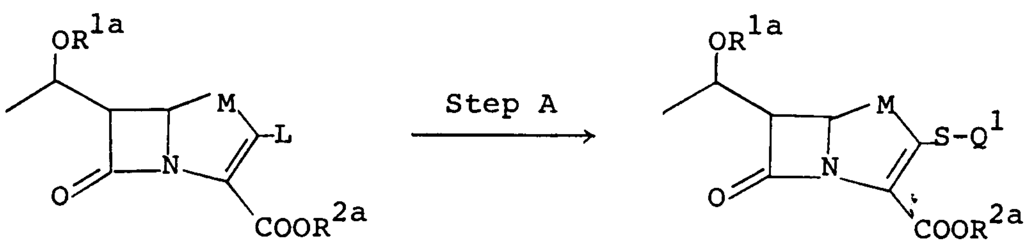


(II) (IV-a)

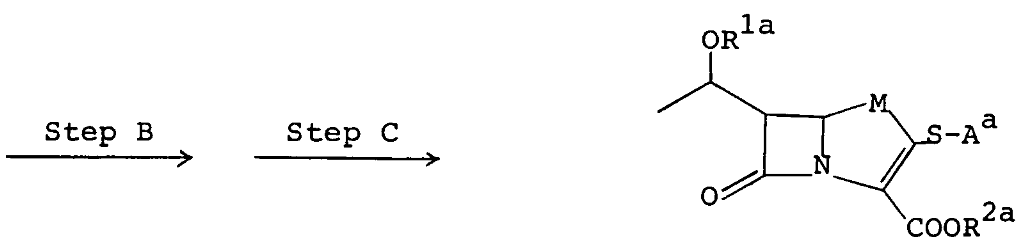


(I-a)

Process (B)

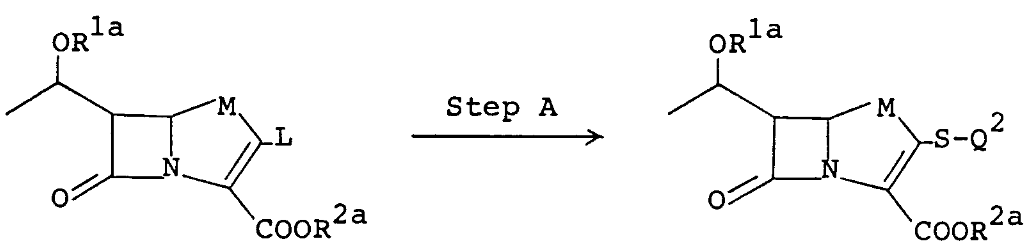


(II) (IV-b)

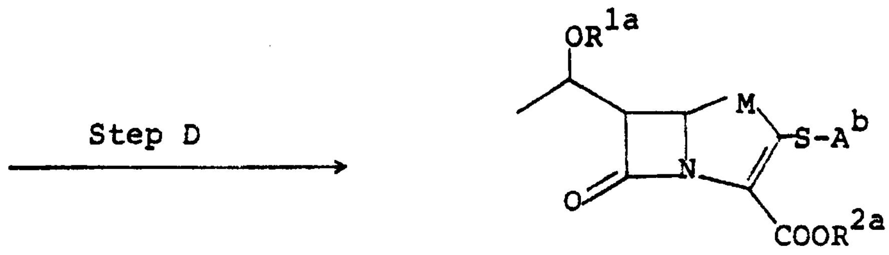


(I-b)

Process (C)

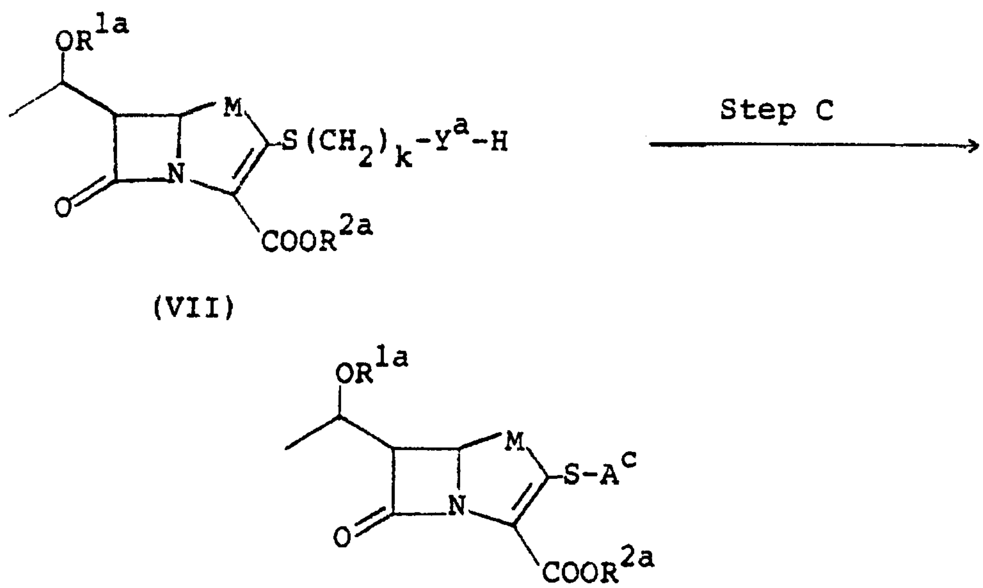


(VII)

(I-c)

Process (D)

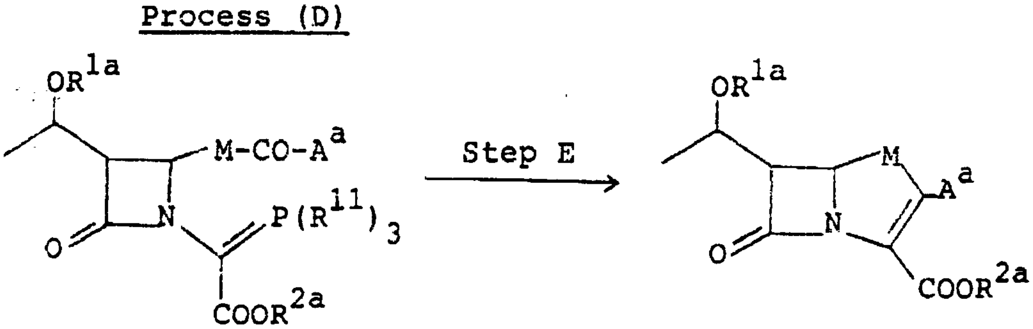


(VIII)

(I-d)

Process (E)

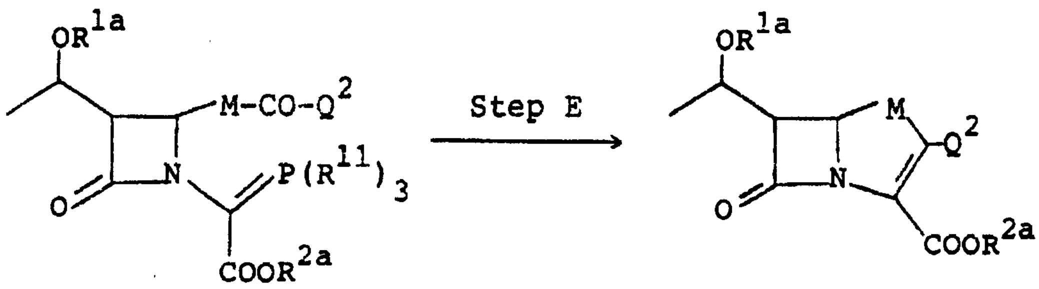


(IX) (X)

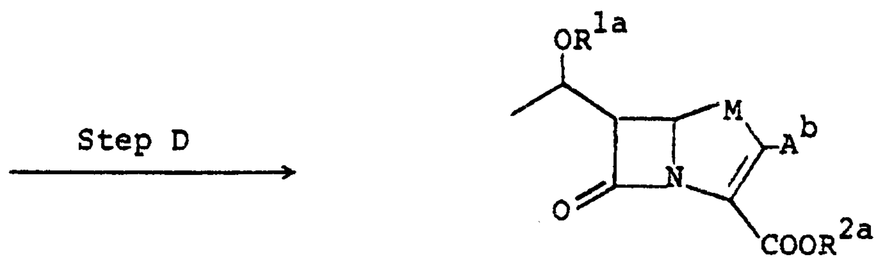


(I-e)

Process (F)

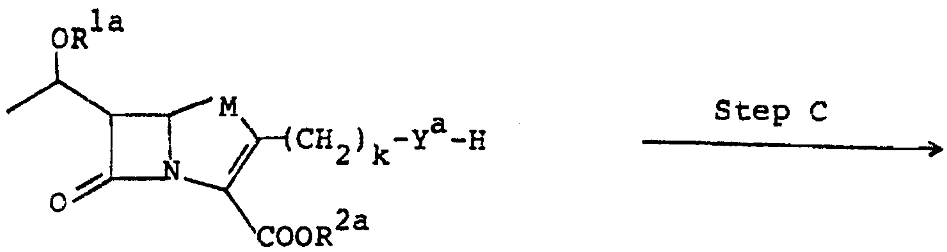


(XI)

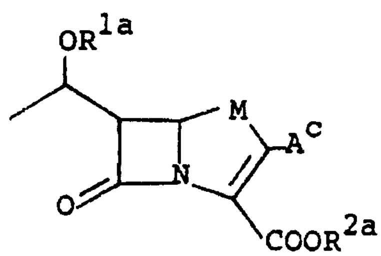

(I-f)

Process (G)

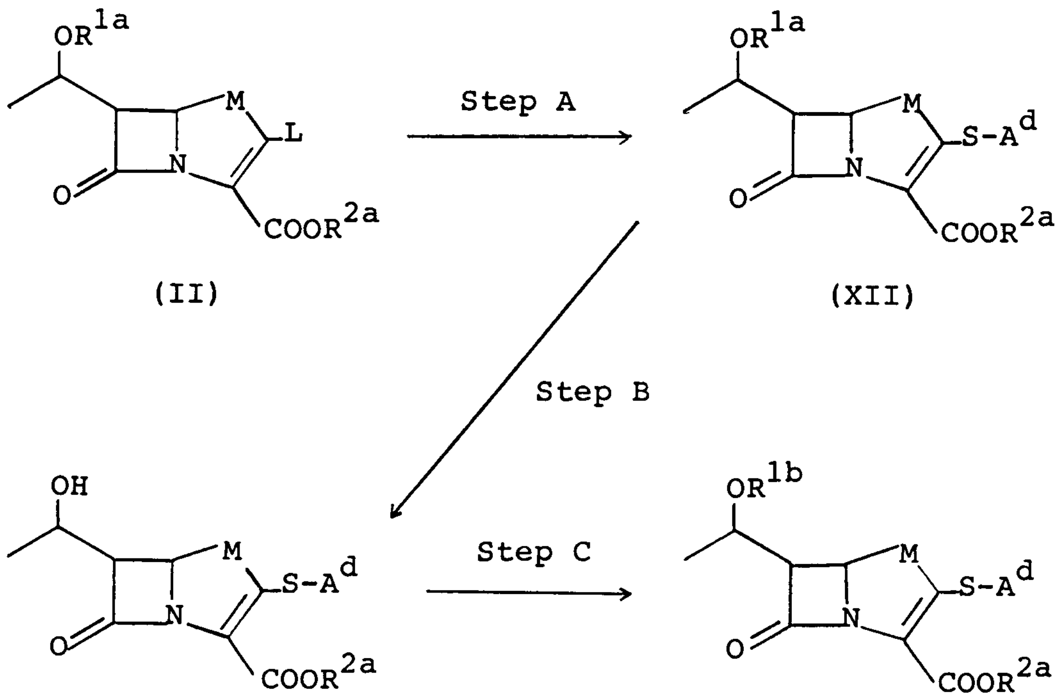


(II)

(XII)

(XIII)

(I-g)

Process (H)

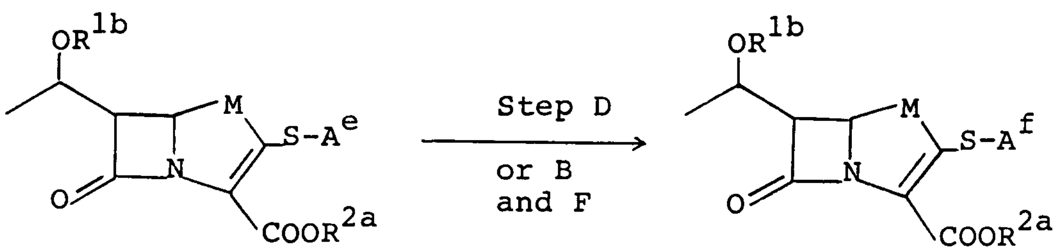


(I-g')

(I-h)

Process (I)

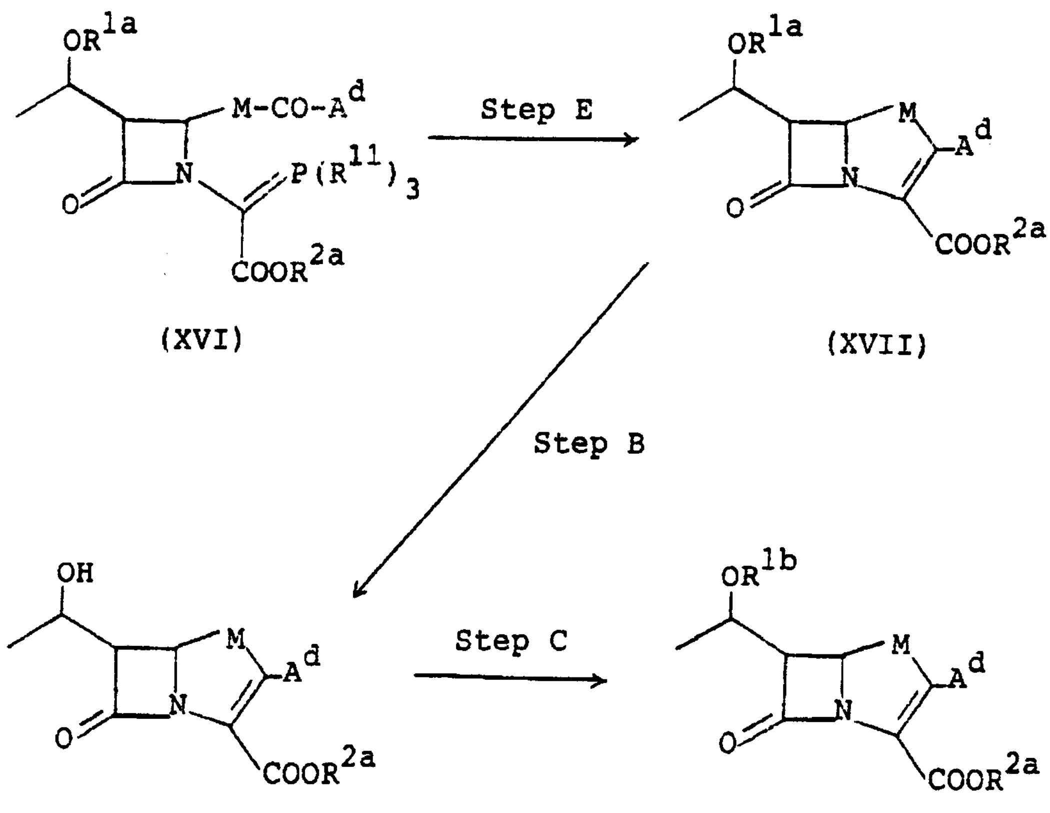


(XVI) (XVII)

(XVII) (I-i)

Process (J)

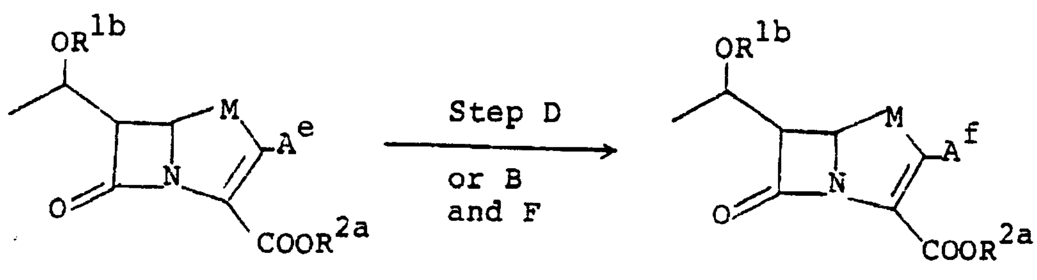


(I-i') (I-j)

Each of the above processes will be hereinafter explained in detail.

Process (A) - Production of β-Lactam Compound (I-a)

The β-lactam compound (I-a) is obtainable by reacting the compound (II) with the mercaptan compound (III-a) in an inert solvent in the presence of a base (Step A).

As the solvent, there may be used, e.g. dioxane, tetrahydrofuran, dimethylsulfoxide, acetonitrile or hexamethylphosphoramide. Examples of the base are an inorganic base (e.g. sodium carbonate, potassium carbonate, sodium hydride, potassium hydride, potassium t-butoxide) and an organic base (e.g. pyridine, dimethylaminopyridine, triethylamine, diisopropylethylamine, 1,8-diazabicyclo[5.4.0]-7-undecene (DBU), among which preferred are diisopropylethylamine and DBU. The base is to be used in such an amount as can assure the smooth proceeding of the reaction, normally in 1 to 3 equimolar amount to the mercaptan compound (III-a). Similarly, the mercaptan compound (III-a) may be used in an excess amount, normally in

1 to 2 equimolar amount to the compound (II). The reaction is usually carried out at a temperature of -78 ° C to 60 ° C, preferably at a temperature of -40 ° C to 40 ° C.

Upon terminatin of the reaction, the reaction mixture may be subjected to post-treatment in a per se conventional manner so as to obtain the objective compound (I-a), if necessary, followed by purification.

The β-lactam compound (I-a) may also be produced by reacting the compound (II) with the mercaptan compound (III-b-1) according to Step A, subjecting the resulting compound (IV-a) to elimination of the amino-protecting group represented by $R^8$ therefrom (Step B) and reacting the deprotected compound with the compound (V) in an inert solvent, if necessary, in the presence of a base (Step C), thereby giving the β-lactam compound (I-a).

The elimination of the amino-protecting group may be effected by a per se conventional procedure such as treatment with an acid, a base or a reducing agent (T.W.Greene: Protective Groups in Organic Synthesis, J. Wiley & Sons Inc., 1981). As the acid, there are exemplified trifluoroacetic acid, formic acid, boron trifluoride and aluminium chloride. As the base, there are exemplified alkali metal carbonate (e.g. sodium carbonate, potassium carbonate), alkali metal sulfide (e.g. sodium sulfide, potassium sulfide) and tetrabutylammonium fluoride. When the elimination is conducted through reduction, there may be adopted any procedure using zinc and acetic acid, hydrogen and palladium-carbon or platinum. The elimination with tetrakistriphenylphosphine palladium is also avilable. Any particular limitation is not present on the solvent to be used, and it may be chosen from water, alcohols (e.g. methanol, ethanol), ethers (e.g. tetrahydrofuran, dioxane), aliphatic acids (e.g. acetic acid), halogenated hydrocarbons (dichloromethane, dichloroethane, chloroform, chlorobenzene), and its mixture. The reaction temperature may be appropiately decided so as to control or accelerate the proceeding of the reaction, and a preferred temperture is normally from -30 to 40 ° C.

The solvent in the latter reaction, i.e. Step C, may be appropriately chosen from any solvent which does not give any adverse effect to the reaction. Specific examples are water, ketones (e.g. acetone, methylethylketone), ethers (e.g. tetrahydrofuran, dioxane), acetonitrile, dimethylformamide, halogenated hydrocarbons (e.g. dichloromethane, dichloroethane, chloroform), and its mixture . Examples of the base are an inorganic base (e.g. sodium carbonate, potassium carbonate, sodium hydride, sodium hydroxide) or an organic base (e.g. pyridine, dimethylaminopyridine, triethylamine, diisopropylethylamine, 1,8-diazabicyclo[5.4.0]-7-undecene (DBU)). This reaction normally proceeds at a temperature of -40 ° C to 60 ° C.

Upon completion of the reaction, the objective product is isolated from the reaction mixture by a per se conventional procedure.

Process (B) - Production of β-Lactam Compound (I-b)

The β-lactam compound (I-b) is obtainable by reacting the compound (II) with the mercaptan compound (III-b-2) according to Step A to give the compound (VI-b), followed by reacting the same with the compound (VI) in an inert solvent (Step D).

The solvent to be used may be appropriately chosen from any solvent which does not give any adverse effect to the reaction. Specific examples are water, ketones (e.g. acetone, methylethylketone), ethers (e.g. tetrahydrofuran, dioxane), acetonitrile, dimethylformamide and halogenated hydrocarbons (e.g. dichloromethane, dichloroethane, chloroform). This reaction normally proceeds at a temperature of -40 ° C to 60 ° C.

Upon completion of the reaction, the objective product is isolated from the reaction mixture by a per se conventional procedure.

Process (C) - Production of β-Lactam Compound (I-c)

The β-lactam compound (I-c) is produced by reacting the compound (VII) with the compound (V) as in Step C.

Process (D) - Production of β-Lactam Compound (I-d)

The β-lactam compound (I-d) is obtainable by heating the compound (VIII) in an inert solvent (Step E).

The solvent may be chosen from aromatic hydrocarbons (e.g. benzene, toluene, xylene), ethers (e.g. dioxane, tetrahydrofuran), cyclohexane and chloroform. Of these, aromatic hydrocarbons are preferred. The reaction temperature may be appropriately decided to control or accelerate the reaction. Thus, a temperature ranging from 20 to 200 ° C is preferred.

The reaction itself in Step E is known, and the operation may be effected, for instance, in the manner as

disclosed in The Journal of Antibiotics, 36, pp.938-941, 1983; ibid., 41, pp.780-787, 1988; and Journal of Medicinal Chemistry, 30, pp.871-880, 1987.

Process (E) - Production of β-Lactam Compound (I-e)

The β-lactam compound (I-e) is produced by subjecting the compound (IX) to Step E to give the compound (X) and reacting the latter with the compound (VI) according to Step D.

Process (F) - Production of β-Lactam Compound (I-f)

The β-lactam compound (I-f) is produced by reacting the compound (XI) with the compound (V) according to Step C.

Process (G) - Production of β-Lactam Compound (I-g)

The β-lactam compound (I-g) is obtainable by reacting the compound (II) with the mercaptan compound (III-c) according to Step A to give the compound (XII), subjecting the latter to elimination of the hydroxy-protecting group represented by $R^{1a}$ (Step B) and reacting the deprotected compound (XIII) with the compound (XIV) in an inert solvent, if necessary, in the presence of a base (Step C), thereby giving the objective compound.

Process (H) - Production of β-Lactam Compound (I-h)

The β-lactam compound (I-h) can be obtained by reacting the compound (I-g') with the compound (XV) according to Step D. Alternatively, the compound (I-g') is subjected to elimination of the amino-protecting group represented by $R^9$ (Step B), followed by reaction of the deprotected compound with $C_1$-$C_3$ alkyl ester of formimidic acid by a per se known procedure, i.e. EP-A-0289801, (Step F) to give the objective compound.

Process (I) - Production of β-Lactam Compound (I-i)

The β-lactam compound (I-i) can be obtained by converting the compound (XVI) to the compound (XVII) according to Step E, if necessary, followed by elimination of the hydroxyl-protecting group represented by $R^{1a}$ (Step B), and reacting the deprotected compound with the compound (XIV) according to Step C to give the objective compound.

Process (J) - Production of β-Lactam Compound (I-j)

The β-lactam compound (I-j) can be obtained by reacting the compound (I-i') with the compound (XV) according to Step D. Alternatively, the compound (I-i') is subjected to elimination of the amino-protecting group represented by $R^9$ (Step B), followed by reaction of the deprotected compound with $C_1$-$C_3$ alkyl ester of formimidic acid (Step F) to give the objective compound.

The thus obtained product, i.e. any of the β-lactam compounds (I-a) to (I-j), may be optionally subjected to elimination of the carboxyl-protecting group represented by $R^{2a}$, the amino-protecting group represented by $R^8$ or $R^9$ and/or the hydroxyl-protecting group represented by $R^{1a}$, $R^5$ or $R^6$ in one or more steps to give the β-lactam compound (I) wherein at least one of $R^8$, $R^9$, $R^{1a}$, $R^5$ and $R^6$ is a hydrogen atom and $R^{2a}$ is a hydrogen atom or a negative charge.

Recovery of the product from the reaction mixture may be accomplished by a per se conventional post-treatment procedure. When desired, the product may be subjected to column chromatography on an adsorptive resin (e.g. CHP-20P polymer) for purification. The fraction comprising the β-lactam compound (I) may be then subjected to freeze-drying.

Various starting and intermediary compounds employed in the above Processes are known or can be produced by the procedures as described in the prior art. Some typical examples of such prior art are as follows:

| Compound | Literature |
|---|---|
| (II) | Tetrahedron Letters, 23, pp.897-900, 1982<br>Heterocyclces, 21, pp.29-40, 1984<br>EP-A-0046363 |
| (VII) or (XI) | Chemical & Pharmaceutical Bulletin, 29, pp.3158-3172, 1981<br>The Journal of Antibiotics, 36, pp.938-941, 1983<br>The Journal of Antibiotics, 41, pp.780-787, 1988 |
| (VIII), (IX) or ((XVI) | The Journal of Antibiotics, 36, pp.938-941, 1983<br>The Journal of Antibiotics, 41, pp.780-787, 1988<br>Journal of Medicinal Chemistry, 30, pp.871-880, 1987 |

The compounds (III-a), (III-b-1), (III-b-2) or (III-c) are obtainable by the known procedures, for instance, those as disclosed in JP-A-60-58987 (Kokai 58987/1985), Japanese Patent Applns. Nos. 34952/1990 and 212102/1990.

The β-lactam compound (I) of the invention includes asymmetric carbon atoms at the 5-, 6- and 8-positions in the penem skeleton as well as at the 4-, 5-, 6- and 8-positions in the carbapenem skeleton and has optical and steric isomers due to those asymmetric carbon atoms. While all these optical and steric isomers and their mixtures are represented by the general formula (I) and fall within the scope of this invention, some certain isomers are particularly preferred. When, for instance, M is a sulfur atom or a methylene group, those having an R-configuration at the 5-position, i.e. (5R,6S) or (5R,6R) isomers, are favorable. When M is a methylene group substituted with lower alkyl and j is 1, those having an S-configuration at the 5-position, i.e. (5S,6S) or (5S,6R) isomers, are favorable. When M is a methylene group substituted with lower alkyl and j is 0, those having an R-configuration at the 5-position, i.e. (5R,6S) or (5R,6R) isomers, are favorable. With respect to the 8-position, it is favored to have an R-configuration.

More preferred are the β-lactam compounds of the following formula (I-k) or (I-l):

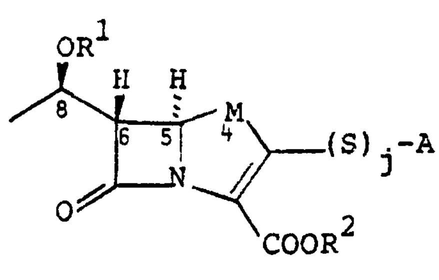

(I-k)

and

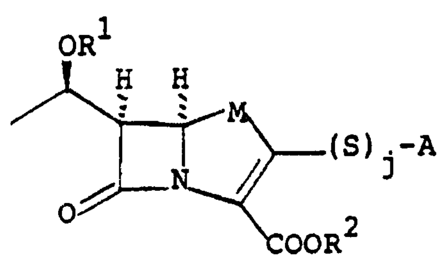

(I-l)

wherein $R^1$, $R^2$, M, A and j are each as defined above.

The most preferred is the β-lactam compound of the above formula (I-k).

Production of the isomers having a certain specific configuration as stated above can be achieved by the use of the corresponding isomers of the starting compounds (II), (VII), (VIII), (IX), (XI) and (XVI).

Still, when a hydroxypyridone moiety, i.e. the group (2) or (3), is present in the structure of the β-lactam compound (I), there can be present tautomeric isomers represented by the following formulas (i) and (ii), and all these are included within the scope of this invention (their nomenclatures and chemical formulas being based on the pyridone type for the sake of convenience):

(i) When the 1-substition is a hydrogen atom, i.e. the group (2) being included:-

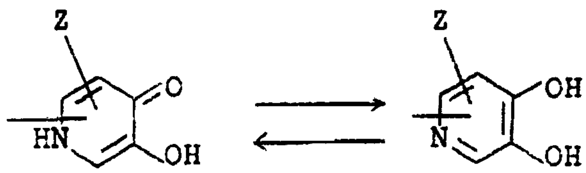

wherein Z is as defined above;

(ii) When the 1-substition is a hydroxyl group, i.e. the group (3) being included and $R^6$ being a hydrogen atom:-

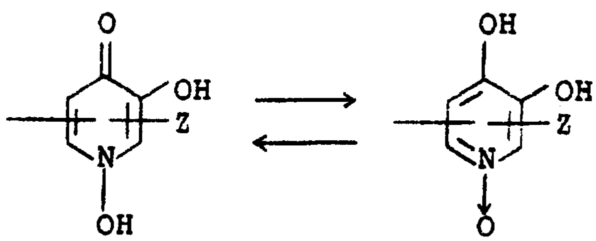

wherein Z is as defined above.

The β-lactam compounds (I) according to the invention are pepem or carbapenem compounds characteristic in having a certain catechol or hydroxypyridone moiety and showing an excellent antimicrobial activity. They are thus useful as antimicrobial agents or as intermediates for preparation of compounds exerting an antimicrobial activity by themselves.

Typical examples of the β-lactam compounds (I) are shown in Tables 1-(1) and 1-(2), in which Me and Et indicate respectively methyl and ethyl.

Table 1-(1)

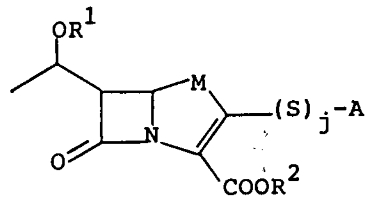

(I)

| Compound No. | $R^1$ | $R^2$ | M | j | A = 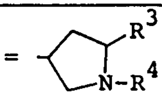 | |
|---|---|---|---|---|---|---|
| | | | | | $R^3$ | $R^4$ |
| 1 | H | H | -CHMe- | 1 | 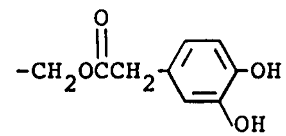 | H |
| 2 | H | H | -CHMe- | 1 | 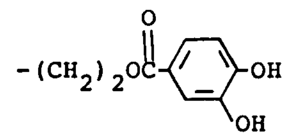 | H |
| 3 | H | H | -CHMe- | 1 | 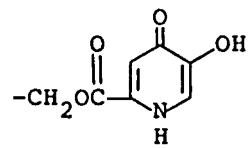 | H |
| 4 | H | H | -CHMe- | 1 | 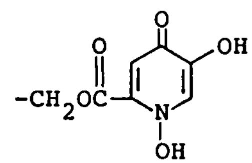 | H |

| | | | | | | |
|---|---|---|---|---|---|---|
| 5 | H | H | $-CH_2-$ | 1 | 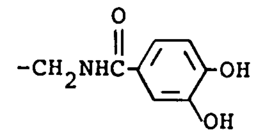 | H |
| 6 | H | H | -CHMe- | 1 | 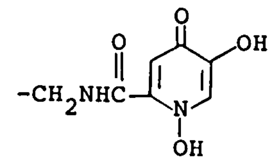 | H |
| 7 | H | H | -CHMe- | 1 | 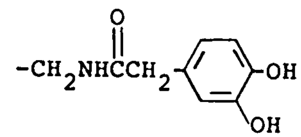 | H |
| 8 | H | H | -CHMe- | 1 | 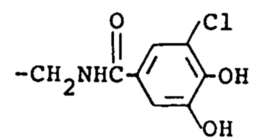 | H |
| 9 | H | H | -CHMe- | 1 | 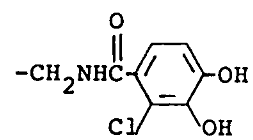 | H |
| 10 | H | H | -CHMe- | 1 | 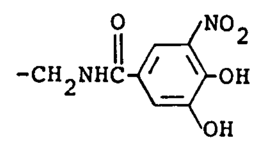 | H |
| 11 | H | H | -CHMe- | 1 | 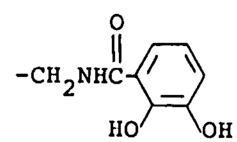 | H |

| | | | | | | |
|---|---|---|---|---|---|---|
| 12 | H | H | -CHMe- | 1 | 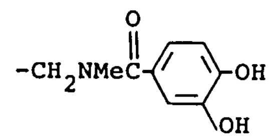 | H |
| 13 | H | H | -CHMe- | 1 | 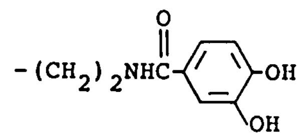 | H |
| 14 | H | H | -CHMe- | 1 | 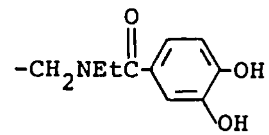 | H |
| 15 | H | ⊖ | -CHMe- | 1 | 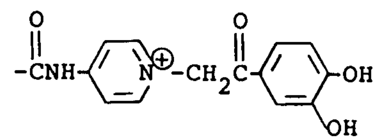 | H |
| 16 | H | ⊖ | -CHMe- | 1 | 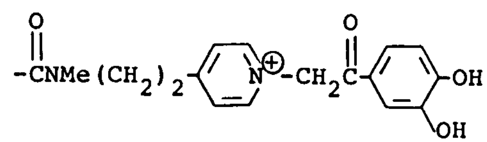 | H |
| 17 | H | ⊖ | -CHMe- | 1 | 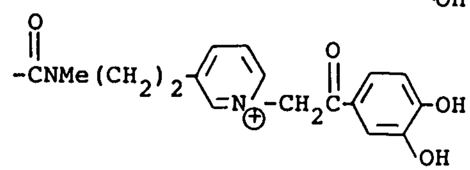 | H |
| 18 | H | ⊖ | -CHMe- | 1 | 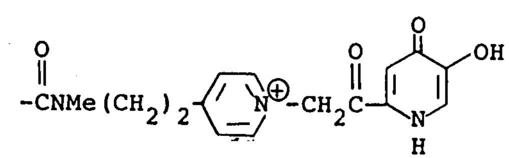 | H |

| | | | | | | |
|---|---|---|---|---|---|---|
| 19 | H | ⊖ | -CHMe- | 1 | 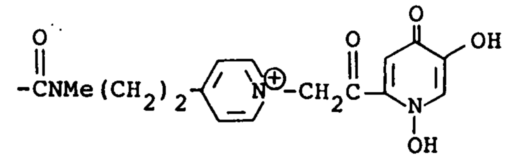 | H |
| 20 | H | ⊖ | -CHMe- | 1 | 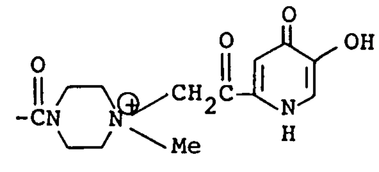 | H |
| 21 | H | ⊖ | -CHMe- | 1 | 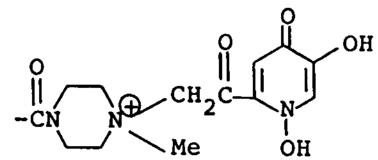 | H |
| 22 | H | ⊖ | -CHMe- | 1 | 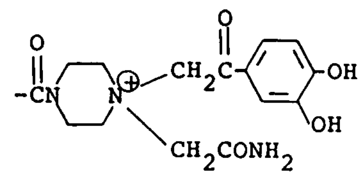 | H |
| 23 | H | ⊖ | -CHMe- | 1 | 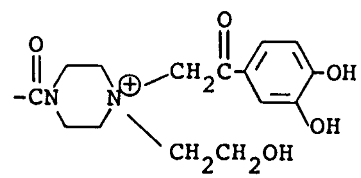 | H |
| 24 | H | H | -CHMe- | 1 | $-CONMe_2$ | 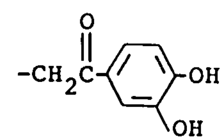 |

| | | | | | | |
|---|---|---|---|---|---|---|
| 25 | H | H | -CHMe- | 1 | $-CONH_2$ | 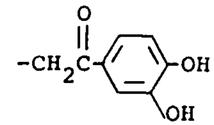 |
| 26 | H | H | -CHMe- | 1 | H | 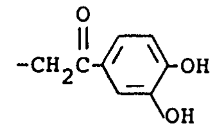 |
| 27 | H | H | -CHMe- | 1 | -CON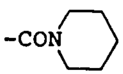 | 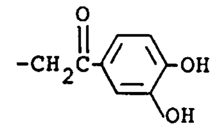 |
| 28 | H | H | -CHMe- | 1 | $-CH_2OH$ | 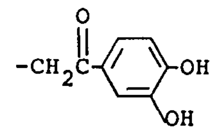 |
| 29 | H | H | -CHMe- | 1 | $-CH_2OMe$ | 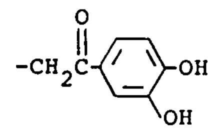 |
| 30 | H | H | -CHMe- | 1 | -CONHMe | 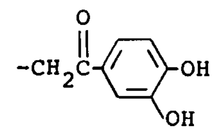 |
| 31 | H | H | $-CH_2-$ | 1 | $-CONMe_2$ | 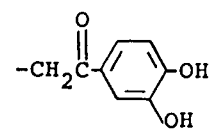 |
| 32 | H | H | -CHMe- | 1 | $-CONMe_2$ | 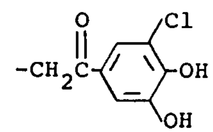 |

| | | | | | | |
|---|---|---|---|---|---|---|
| 33 | H | H | -CHMe- | 1 | $-CONMe_2$ | 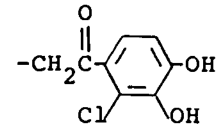 |
| 34 | H | H | -CHMe- | 1 | $-CONMe_2$ | 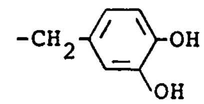 |
| 35 | H | H | -CHMe- | 1 | $-CONMe_2$ | 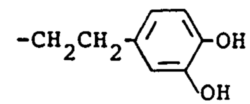 |
| 36 | H | H | -CHMe- | 1 | $-CONMe_2$ | 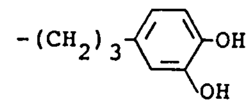 |
| 37 | H | H | -CHMe- | 1 | -CONHMe | 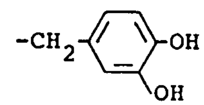 |
| 38 | H | H | -CHMe- | 1 | $-CONH_2$ | 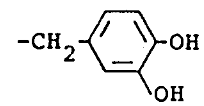 |
| 39 | H | H | -CHMe- | 1 | H | 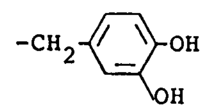 |
| 40 | H | H | -CHMe- | 1 | -CON 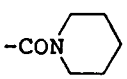 | 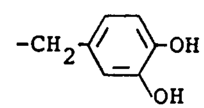 |
| 41 | H | H | -CHMe- | 1 | $-CH_2OH$ | 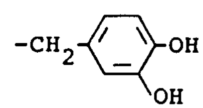 |

| | | | | | | |
|---|---|---|---|---|---|---|
| 42 | H | H | -CHMe- | 1 | $-CH_2OMe$ | 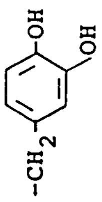 |
| 43 | H | H | -CHMe- | 1 | $-CONMe_2$ | 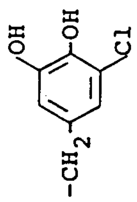 |
| 44 | H | H | -CHMe- | 1 | -CONHMe | 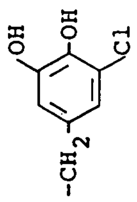 |
| 45 | H | H | -CHMe- | 1 | $-CONH_2$ | 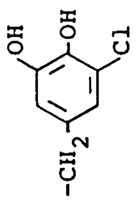 |
| 46 | H | H | -CHMe- | 1 | H | 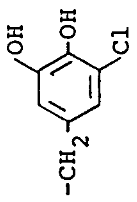 |
| 47 | H | H | $-CH_2-$ | 1 | $-CONMe_2$ | 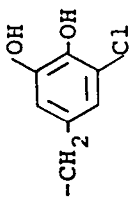 |
| 48 | H | H | -CHMe- | 1 | $-CONMe_2$ | 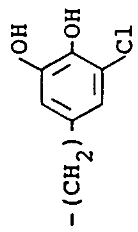 |

| | | | | | | |
|---|---|---|---|---|---|---|
| 49 | H | H | -CHMe- | 1 | $-CONMe_2$ | 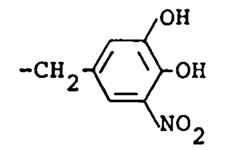 |
| 50 | H | H | -CHMe- | 1 | $-CONMe_2$ | 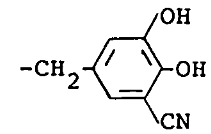 |
| 51 | H | H | -CHMe- | 1 | $-CONMe_2$ | 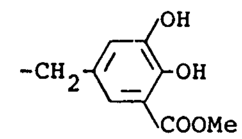 |
| 52 | H | H | -CHMe- | 1 | $-CONMe_2$ | 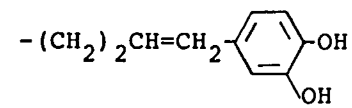 |
| 53 | H | H | -CHMe- | 1 | $-CONMe_2$ | 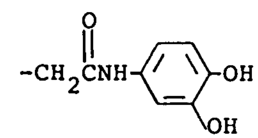 |
| 54 | H | H | -CHMe- | 1 | $-CONMe_2$ | 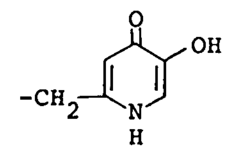 |
| 55 | H | H | -CHMe- | 1 | $-CONMe_2$ | 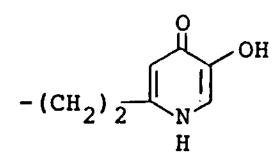 |

| | | | | | | |
|---|---|---|---|---|---|---|
| 56 | H | H | -CHMe- | 1 | $-CONMe_2$ | 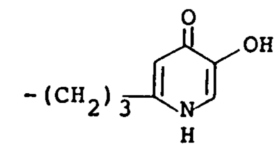 |
| 57 | H | H | -CHMe- | 1 | $-CONMe_2$ | 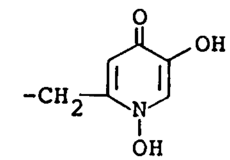 |
| 58 | H | H | -CHMe- | 1 | $-CONMe_2$ | 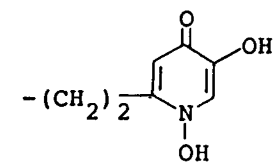 |
| 59 | H | H | -CHMe- | 1 | $-CONMe_2$ | 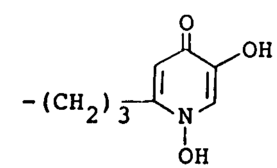 |
| 60 | H | H | -CHMe- | 1 | $-CONMe_2$ | 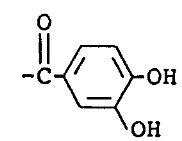 |
| 61 | H | H | -CHMe- | 1 | $-CONMe_2$ | 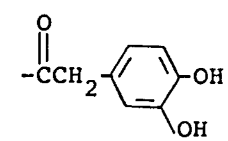 |

| | | | | | | |
|---|---|---|---|---|---|---|
| 62 | H | H | -CHMe- | 1 | $-CONMe_2$ | 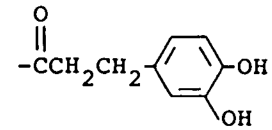 |
| 63 | H | H | -CHMe- | 1 | $-CONMe_2$ | 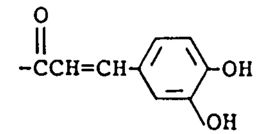 |
| 64 | H | H | -CHMe- | 1 | $-CONH_2$ | 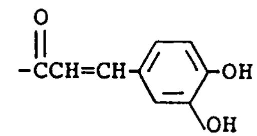 |
| 65 | H | H | -CHMe- | 1 | H | 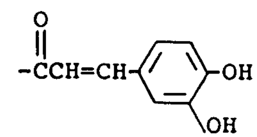 |
| 66 | H | H | -CHMe- | 1 | $-CH_2OH$ | 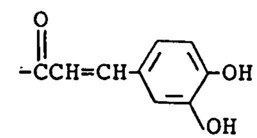 |
| 67 | H | H | -CHMe- | 1 | $-CONMe_2$ | 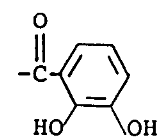 |
| 68 | H | H | -CHMe- | 1 | $-CONMe_2$ | 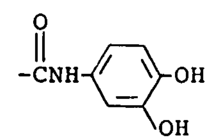 |

| | | | | | | |
|---|---|---|---|---|---|---|
| 69 | H | H | $-CHMe-$ | 1 | $-CONMe_2$ | 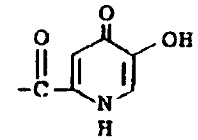 |
| 70 | H | H | $-CHMe-$ | 1 | $-CONMe_2$ | 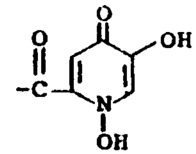 |
| 71 | H | H | $-CHMe-$ | 1 | $-CONMe_2$ | 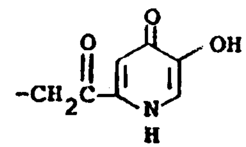 |
| 72 | H | H | $-CHMe-$ | 1 | $-CONMe_2$ | 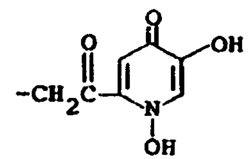 |
| 73 | H | H | $-CHMe-$ | 1 | $-CONMe_2$ | 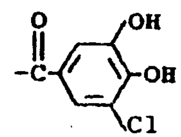 |
| 74 | H | H | $-CHMe-$ | 1 | $-CONMe_2$ | 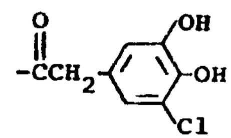 |

| | | | | | | |
|---|---|---|---|---|---|---|
| 75 | 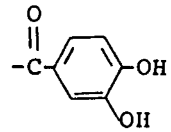 | H | -CHMe- | 1 | $-CONMe_2$ | H |
| 76 | 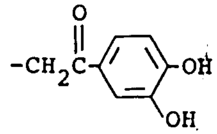 | H | -CHMe- | 1 | $-CONMe_2$ | H |
| 77 | 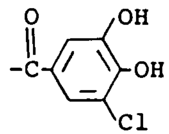 | H | -CHMe- | 1 | $-CONMe_2$ | H |
| 78 | 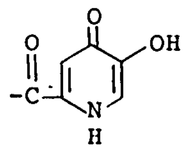 | H | -CHMe- | 1 | $-CONMe_2$ | H |
| 79 | 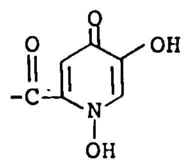 | H | -CHMe- | 1 | $-CONMe_2$ | H |
| 80 | H | H | -CHMe- | 1 | $-CH_2NH_2$ | 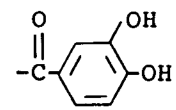 |
| 81 | H | H | -CHMe- | 1 | $-CH_2NH_2$ | 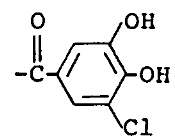 |

| | | | | | | |
|---|---|---|---|---|---|---|
| 82 | H | H | -CHMe- | 1 | $-CH_2NH_2$ | 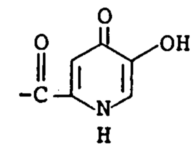 |
| 83 | H | H | -CHMe- | 1 | $-CH_2NH_2$ | 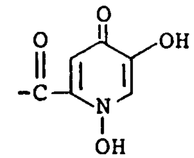 |
| 84 | H | H | -CHMe- | 0 | $-CONMe_2$ | 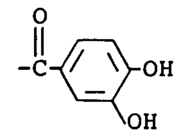 |
| 85 | H | H | -CHMe- | 0 | $-CONMe_2$ | 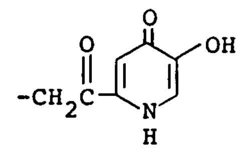 |
| 86 | H | H | -CHMe- | 0 | 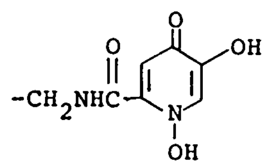 | H |
| 87 | H | H | -S- | 0 | $-CONMe_2$ | 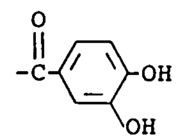 |

| | | | | | | |
|---|---|---|---|---|---|---|
| 88 | H | H | -S- | 0 | $-CONMe_2$ | 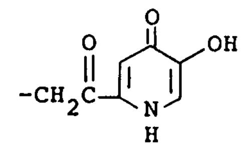 |
| 89 | H | H | -S- | 0 | 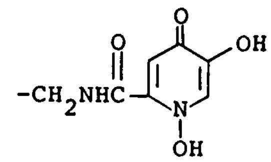 | H |
| 90 | H | H | -S- | 0 | $-CH_2NH_2$ | 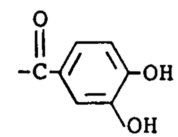 |
| 91 | H | H | -S- | 0 | $-CH_2OH$ | 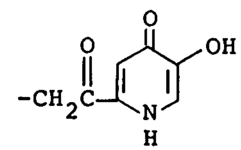 |
| 92 | H | H | -S- | 0 | 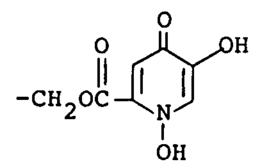 | Me |

Table 1-(2')

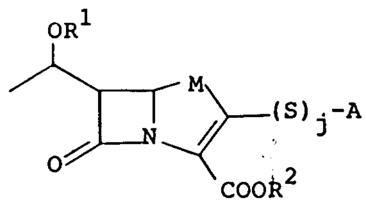

(I)

| Compound No. | $R^1$ | $R^2$ | M | j | A |
|---|---|---|---|---|---|
| 93 | 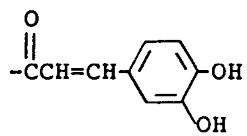 | H | -CHMe- | 1 | 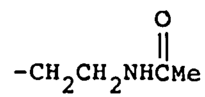 |
| 94 | 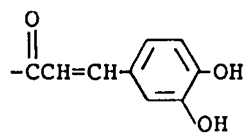 | H | -CHMe- | 1 | $-CH_2CH_2NH_2$ |
| 95 | 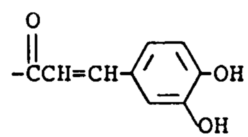 | H | -CHMe- | 1 | $-CH_2CH_2NMe_2$ |
| 96 | 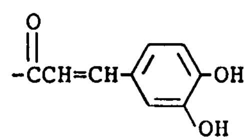 | H | -CHMe- | 1 | $-CH_2CH_2OH$ |
| 97 | 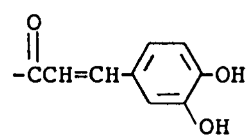 | H | $-CH_2-$ | 1 | 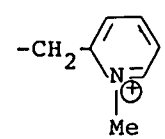 |

| | | | | | |
|---|---|---|---|---|---|
| 98 | 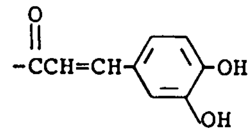 | H | $-CH_2-$ | 1 | 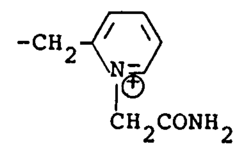 |
| 99 | 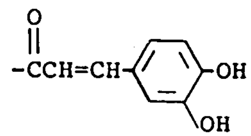 | H | $-CH_2-$ | 1 | 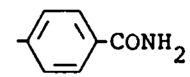 |
| 100 | 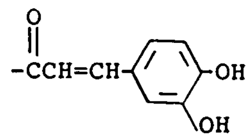 | H | -S- | 0 | 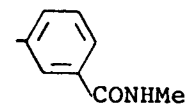 |
| 101 | H | H | -CHMe- | 1 | 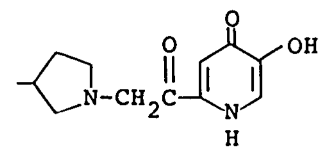 |
| 102 | H | H | -CHMe- | 1 | 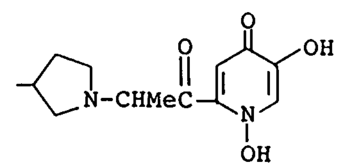 |
| 103 | H | H | -CHMe- | 1 | 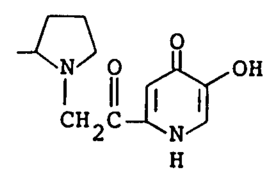 |

| | | | | | |
|---|---|---|---|---|---|
| 104 | H | H | -S- | 0 | 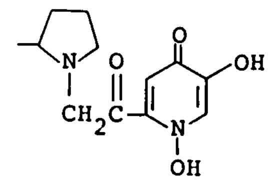 |
| 105 | H | H | -CHMe- | 1 | 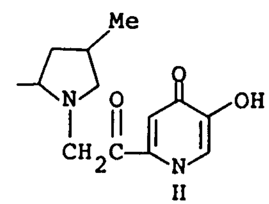 |
| 106 | H | H | -S- | 0 | 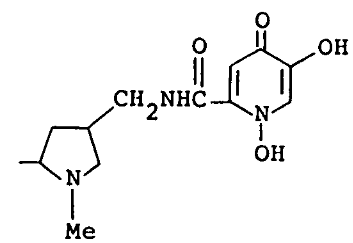 |
| 107 | H | H | -CHMe- | 1 | 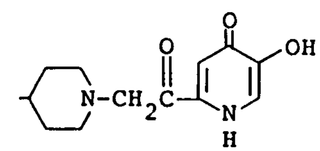 |
| 108 | H | H | -S- | 0 | 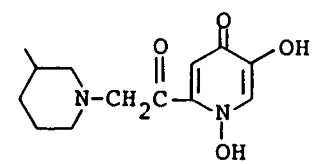 |

| | | | | | |
|---|---|---|---|---|---|
| 109 | H | H | -CHMe- | 1 | 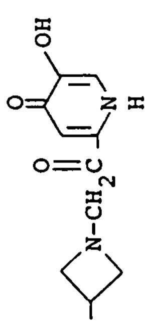 |
| 110 | H | H | -S- | 0 | 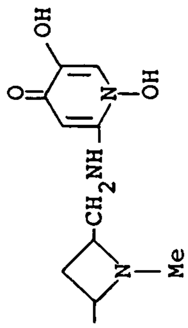 |
| 111 | 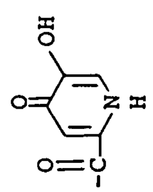 | H | -CHMe- | 1 | 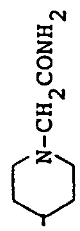 |
| 112 | 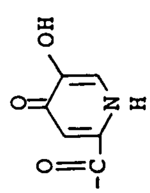 | H | -S- | 1 | 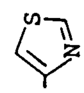 |
| 113 | 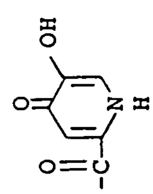 | H | -S- | 1 | 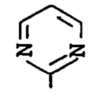 |
| 114 | 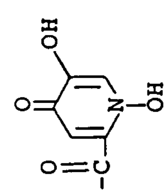 | H | -S- | 0 | 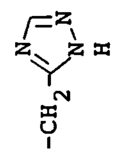 |

The β-lactam compounds (I) as exemplified in Table 1 have their optical and steric isomers, and all of them are included within the scope of this invention.

The β-lactam compounds (I) according to the invention exert an excellent antimicrobial activity against Gram-positive and Gram-negative bacteria including Staphylococcus aureus, Streptococcus pyogenes, Escherichia coli and Pseudomonas aeruginosa. It is notable that while conventional carbapenem compounds such as imipenem are generally unstable in a living body, especially sensitive to renal DHP-I, the β-lactam compounds (I) are in general resistant or stable to renal DHP-I. It is also notable that the half life time ($T\frac{1}{2}$) of the β-lactam compounds (I) in a living body is generally longer than that of conventional carbapenem compounds such as imipenem. It is further notable that the β-lactam compounds (I) produce strong antimicrobial potency against imipenem-resistant Pseudomonas aeruginosa. The β-lactam compounds (I) are thus useful as antimicrobial drugs, especially against Pseudomonas aeruginosa infection, and also as intermediates in the synthesis of such antimicrobial drugs.

For the practical usage of the β-lactam compounds (I) as antimicrobial drugs, they may be formulated into conventional preparation forms together with excipients or additives such as carriers, diluents, binders

and stabilizers and administered in various modes, of which examples are oral administration in the form of tablets, capsules, dispersants and syrups, non-oral administration in the form of injection through vein, muscle or rectum, etc. When they are applied in injection forms, the preparations may additionally include buffering agents, solubilizing agents and isotonic agents. The daily dosage may vary depending upon the state of disease, the age and body weight of patients, the administration mode and time and the normal daily dosage to a human adult is between about 100 to 3000 mg, optinally divided in one to several times per day. If necessary, the dosage may be increased or decreased appropriately.

Practical and presently preferred embodiments of the invention are illustratively shown in the following Examples, which are not intended to limit the scope of the invention thereto. Further, the abbreviations used therein show the following meanings: AOC, allyloxycarbonyl, BOC, t-butyloxycarbonyl, Me, methyl; Et, ethyl; Ph, phenyl; PNB, p-nitrobenzyl; PNZ, p-nitrobenzyloxycarbonyl; TBDMS, t-butyldimethylsilyl; TMS, trimethylsilyl.

Example 1

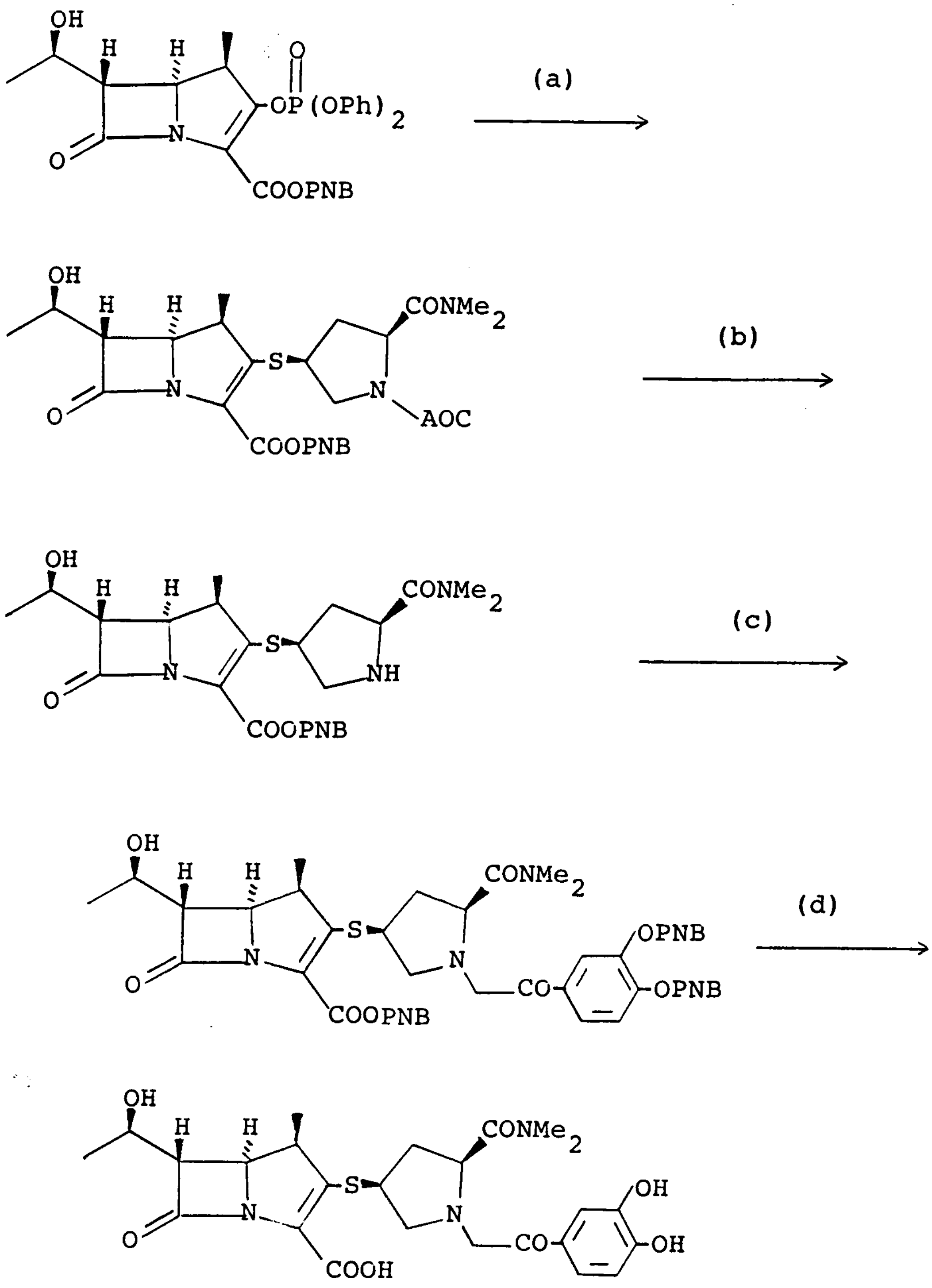


(a) To a solution of (4R,5R,6S,8R)-p-nitrobenzyl-3-diphenylphosphoryloxy-4-methyl-6-(1-hydroxyethyl)-1-azabicyclo[3.2.0]hept-2-en-7-one-2-carboxylate (1.52 g) in dry acetonitrile (10 ml), a solution of (2S,4S)-1-allyloxycarbonyl-2-dimethylaminocarbonyl-4-mercaptopyrrolidine (877 mg) in dry acetonitrile (2 ml) was added in nitrogen stream while ice-cooling. Diisopropylethylamine (0.49 ml) was added to the mixture, followed by stirring for 1 hour. The reaction mixture was diluted with ethyl acetate, washed with a 2.5 % aqueous potassium dihydrogenphosphate solution and an aqueous sodium chloride solution in order, dried over magnesium sulfate and concentrated. The residue was chromatographed on silica gel column to give (4R,5S,6S,8R,2'S,4'S)-p-nitrobenzyl-3-[(1-allyloxycarbonyl-2-dimethylaminocarbonylpyrrolidin)-4-ylthio]-4-methyl-6-(1-hydroxyethyl)-1-azabicyclo[3.2.0]hept-2-en-7-one-2-carboxylate (1.0 g).

$IR_{max}$ $cm^{-1}$ (neat): 3307 (br), 1770, 1702, 1655 (sh), 1650, 1522, 1412, 1347, 1140.

NMR δ ppm ($CDCl_3$): 1.28 (3H, m), 1.37 (3H, m), 2.68 (1H, m), 2.97, 2.98, 3.06, 3.11 (6H in total, each s), 3.27 (1H, m), 3.47 (2H, m), 3.64 (1H, m), 4.12 (1H, m), 4.26 (2H, m), 4.74 (1H, m), 5.1 - 5.6 (4H, m), 5.90 (1H, m), 7.65 (2H, d, J = 8.8 Hz), 8.23 (2H, d, J = 8.8 Hz).

(b) (4R,5S,6S,8R,2'S,4'S)-p-Nitrobenzyl-3-[(1-allyloxycarbonyl-2-dimethylaminocarbonylpyrrolidin)-4-ylthio]-4-methyl-6-(1-hydroxyethyl)-1-azabicyclo[3.2.0]-hept-2-en-7-one-2-carboxylate (1.65 g) was dissolved in dry tetrahydrofuran (30 ml), followed by addition of dimedone (1.66 g) in nitrogen stream. Under ice-cooling, tetrakistriphenylphosphine palladium (342 mg) was added to the resultant mixture, followed by stirring for 10 minutes. After removal of the solvent, the residue was chromatographed on silica gel column to give (4R,5S,6S,8R,2'S,4'S)-p-nitrobenzyl-3-[(2-dimethylaminocarbonylpyrrolidin)-4-ylthio]-4-methyl-6-(1-hydroxyethyl)-1-azabicyclo[3.2.0]-hept-2-en-7-one-2-carboxylate (0.94 g).

$IR_{max}$ $cm^{-1}$ (neat): 3370 (br), 1763, 1698, 1638, 1518, 1381, 1342, 1203, 1137.

NMR δ ppm ($CDCl_3$): 1.28 (3H, d, J = 7.3 Hz), 1.36 (3H, d, J = 6.3 Hz), 1.59 (1H, m), 2.59 (1H, m), 3.00 (3H, s), 3.02 (3H, s), 3.10 (1H, dd, J = 11.6 & 4.6 Hz), 3.27 (2H, m), 3.39 (1H, m), 3.75 (1H, m), 3.94 (1H, t, J = 7.9 Hz), 4.25 (2H, m), 5.23 (1H, d, J = 13.8 Hz), 5.50 (1H, d, J = 13.8 Hz), 7.67 (2H, d, J = 8.6 Hz), 8.24 (2H, d, J = 8.6 Hz).

(c) To a solution of (4R,5S,6S,8R,2'S,4'S)-p-nitrobenzyl-3-[(2-dimethylaminocarbonylpyrrolidin)-4-ylthio]-4-methyl-6-(1-hydroxyethyl)-1-azabicyclo[3.2.0]-hept-2-en-7-one-2-carboxylate (137 mg) in acetone (3 ml) and methylene chloride (2 ml), 1-bromoacetyl-3,4-di(p-nitrobenzyloxy)benzene (200 mg) was added, and the resultant mixture was stirred at room temperature for 1 day. The reaction mixture was washed with 0.02M phosphate buffer (pH, 7.0), dried over magnesium sulfate and concentrated. The residue was chromatographed on silica gel column to give (4R,5S,6S,8R,2'S,4'S)-p-nitrobenzyl-3-[(1-(3,4-di-p-nitrobenzyloxybenzoyl)methyl-2-dimethylaminocarbonylpyrrolidin)-4-ylthio]-4-methyl-6-(1-hydroxyethyl)-1-azabicyclo[3.2.0]-hept-2-en-7-one-2-carboxylate (129 mg).

$IR_{max}$ $cm^{-1}$ (neat): 3400 (br), 1763, 1694, 1672, 1640, 1602, 1505, 1417, 1344, 1271, 1207, 1177, 1133.

NMR δ ppm ($CDCl_3$): 1.20 (3H, d, J = 7.3 Hz), 1.35 (3H, d, J = 5.9 Hz), 1.87 (1H, m), 2.74 (1H, m), 2.95 (3H, s), 3.03 (3H, s), 3.20 (3H, m), 3.37 (1H, m), 3.75 (1H, d, J = 16.2 Hz), 3.81 (1H, m), 3.99 (1H, t, J = 7.9 Hz), 4.23 (1H, t, J = 6.3 Hz), 4.32 (1H, d, J = 16.2 Hz), 5.17 (1H, d, J = 13.9 Hz), 5.32 (2H, s), 5.37 (2H, s), 5.44 (1H, d, J = 13.9 Hz), 6.93 (1H, d, J = 8.6 Hz), 7.56 - 7.77 (7H, m), 7.96 (1H, d, J = 2.0 Hz), 8.13 - 8.33 (6H, m).

(d) (4R,5S,6S,8R,2'S,4'S)-p-Nitrobenzyl-3-[(1-(3,4-di-p-nitrobenzyloxybenzoyl)methyl-2-dimethylaminocarbonylpyrrolidin)-4-ylthio]-4-methyl-6-(1-hydroxyethyl)-1-azabicyclo[3.2.0]hept-2-en-7-one-2-carboxylate (120 mg) was dissolved in tetrahydrofuran (6 ml) and 0.1M phosphate buffer (pH, 7.0; 6 ml), followed by addition of 10 % palladium-carbon (120 mg). Catalytic hydrogenation was performed at room temperature under atmospheric pressure for 2 hours. After removal of the catalyst by filtration, the filtrate was washed with methylene chloride three times. The organic solvent in the aqueous phase was removed by distillation under reduced pressure. The residue was filtered through a membrane filter, and the filtrate was purified by column chromatography (CHP-20P polymer) with a 2 to 4 % aqueous tetrahydrofuran solution as an eluent. The eluted fractions were collected and freeze-dried to give (4R,5S,6S,8R,2'S,4'S)-3-[(1-(3,4-dihydroxybenzoyl)methyl-2-dimethylaminocarbonylpyrrolidin)-4-ylthio]-4-methyl-6-(1-hydroxyethyl)-1-azabicyclo[3.2.0]hept-2-en-7-one-2-carboxylic acid as a white amorphous substance.

$UV_{max}$ nm ($H_2O$): 220, 263, 297.

$IR_{max}$ $cm^{-1}$ (KBr): 3400 (br), 1754, 1620, 1594, 1388, 1289.

NMR δ ppm ($D_2O$): 1.19 (3H, d, J = 6.9 Hz), 1.30 (3H, d, J = 6.3 Hz), 1.72 (3H, m), 2.80 (1H, m), 2.90 (3H, s), 3.01 (3H, s), 3.24 (3H, m), 3.40 (2H, m), 3.87 (1H, m), 3.95 - 4.30 (5H, m), 6.91 (1H, d, J = 8.3 Hz), 7.45 (1H, s), 7.52 (1H, d, J = 8.3 Hz).

Example 2

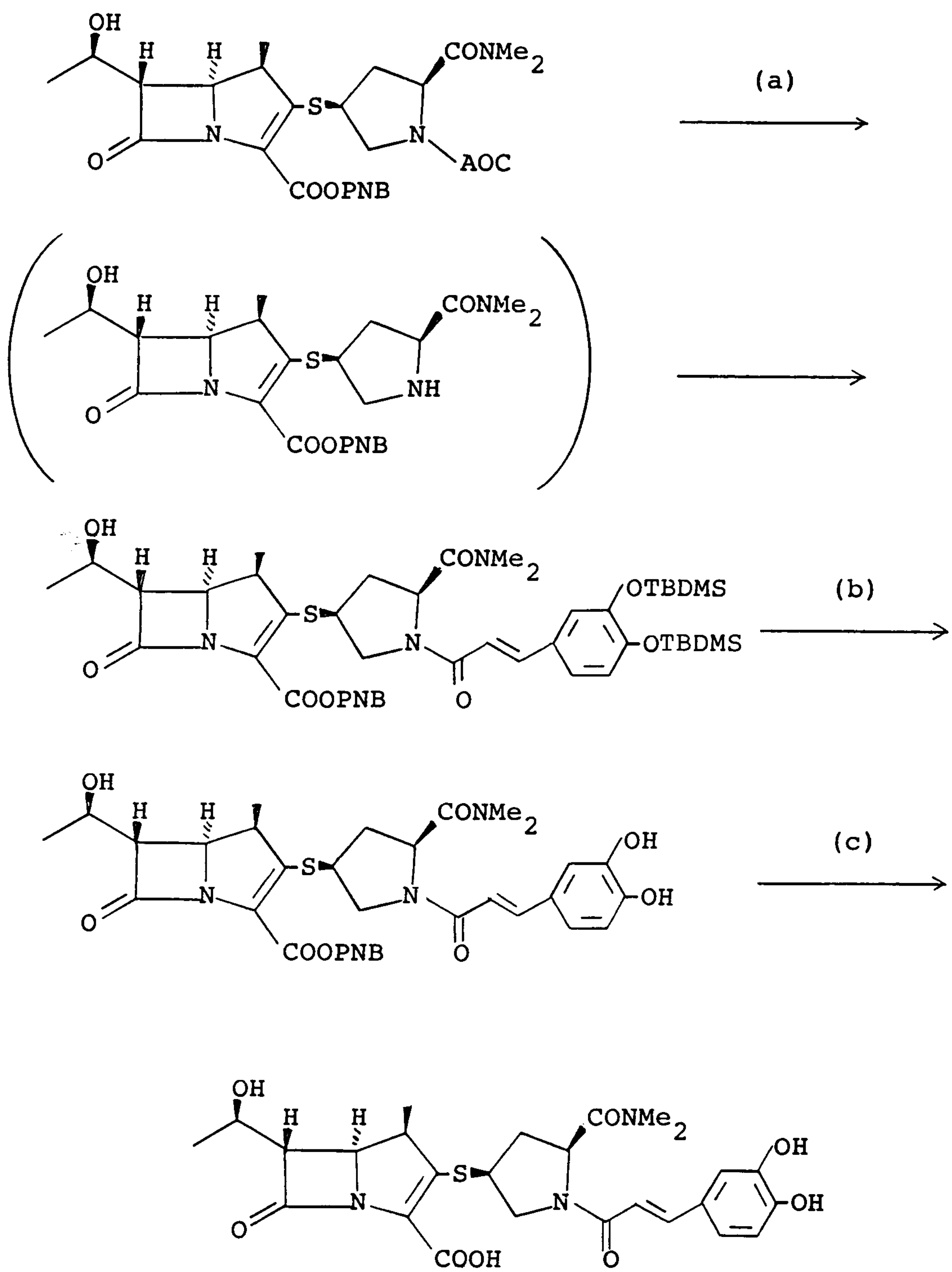


(a) To a solution of (4R,5S,6S,8R,2'S,4'S)-p-nitrobenzyl-3-[(1-allyloxycarbonyl-2-dimethylaminocarbonylpyrrolidin)-4-ylthio]-4-methyl-6-(1-hydroxyethyl)-1-azabicyclo[3.2.0]hept-2-en-7-one-2-carboxylate (574 mg) in dry methylene chloride (18 ml), dimedone (560 mg) was added in nitrogen stream, and tetrakistriphenylphosphine palladium (116 mg) was added thereto, followed by stirring for 20 minutes. The reaction mixture was cooled to 0 °C, and a solution of diisopropylethylamine (115 mg) in dry methylene chloride (0.5 ml) was added thereto. To the resultant mixture, a solution of 3,4-di(t-butyldimethylsilyloxy)-cinnamoyl chloride (approx. 1 mmol) in dry methylene chloride (3 ml) was dropwise added, and the reaction mixture was washed with an aqueous sodium chloride solution, dried over magnesium sulfate and sodium carbonate and concentrated. The residue was chromatographed on silica gel column to give (4R,5S,6S,8R,2'S,4'S)-p-nitrobenzyl-3-[{1-(3,4-di(t-butyldimethylsilyloxy)-

cinnamoyl)-2-dimethylaminocarbonylpyrrolidin}-4-ylthio]-4-methyl-6-(1-hydroxyethyl)-1-azabicyclo[3.2.0]-hept-2-en-7-one-2-carboxylate (446 mg).

$IR_{max}$ $cm^{-1}$ (neat): 3400 (br), 1770, 1707, 1655 (sh), 1642, 1602, 1593, 1513, 1347, 1302, 1253, 1132.

NMR δ ppm ($CDCl_3$): 0.19 (6H, s), 0.21 (6H, s), 0.98 (18H, s), 1.27 (3H, d, J = 6.9 Hz), 1.37 (3H, d, J = 6.3 Hz), 2.63 (1H, m), 3.00 (3H, s), 3.21 (3H, s), 3.25 - 3.55 (3H, m), 3.60 - 3.85 (2H, m), 4.20 - 4.40 (3H, m), 4.98 (1H, t, J = 8.4 Hz), 5.20 - 5.60 (2H, m), 6.46 (1H, d, J = 16 Hz), 6.81 (1H, d, J = 8.3 Hz), 6.94 (1H, d, J = 2.0 Hz), 7.05 (1H, dd, J = 2.0 & 8.3 Hz), 7.56 (1H, d, J = 16 Hz), 7.66 (2H, d, J = 8.9 Hz), 8.24 (2H, d, J = 8.9 Hz).

(b) To a solution of (4R,5S,6S,8R,2'S,4'S)-p-nitrobenzyl-3-[{1-(3,4-di(t-butyldimethylsilyloxy)-cinnamoyl)-2-dimethylaminocarbonylpyrrolidin}-4-ylthio]-4-methyl-6-(1-hydroxyethyl)-1-azabicyclo[3.2.0]hept-2-en-7-one-2-carboxylate (446 mg) in tetrahydrofuran (6.8 ml), acetic acid (187 mg) was added at room temperature, followed by dropwise addition of a 1M tetrahydofuran solution of tetrabutylammonium fluoride (1 ml). The resultant mixture was stirred for 1 hour, diluted with ethyl acetate and neutralized with an aqueous solution of sodium hydrogen carbonate (262 mg). The organic layer was washed with an aqueous sodium chloride solution and dried over magnesium sulfate. After removal of the solvent, the residue was chromatographed on silica gel column to give (4R,5S,6S,8R,2'S,4'S)-p-nitrobenzyl-3-[{1-(3,4-dihydroxycinnamoyl)-2-dimethylaminocarbonylpyrrolidin}-4-ylthio]-4-methyl-6-(1-hydroxyethyl)-1-azabicyclo[3.2.0]hept-2-en-7-one-2-carboxylate (220 mg).

$IR_{max}$ $cm^{-1}$ (KBr): 3430 (br), 1768, 1710, 1642, 1600, 1521, 1440, 1350, 1286.

NMR δ ppm ($CDCl_3$-$CD_3OD$ (9:1)): 1.25 (3H, d, J = 7.3 Hz), 1.33 (3H, d, J = 6.3 Hz), 2.66 (1H, m), 3.00 (3H, s), 3.38 (3H, m), 3.70 (2H, m), 4.0 - 4.4 (3H, m), 4.95 (1H, t, J = 8.6 Hz), 5.2 - 5.6 (2H, m), 6.49 (1H, d, J = 15 Hz), 6.80 (1H, d, J = 8.3 Hz), 6.92 (1H, d, J = 7.3 Hz), 7.04 (1H, s), 7.49 (1H, d, J = 15 Hz), 7.67 (2H, d, J = 8.9 Hz), 8.24 (2H, d, J = 8.9 Hz).

(c) (4R,5S,6S,8R,2'S,4'S)-p-Nitrobenzyl-3-[{1-(3,4-dihydroxycinnamoyl)-2-dimethylaminocarbonylpyrrolidin}-4-ylthio]-4-methyl-6-(1-hydroxyethyl)-1-azabicyclo[3.2.0]hept-2-en-7-one-2-carboxylate (258 mg) was dissolved in tetrahydrofuran (13.0 ml) and 0.1M phosphate buffer (pH, 7.0; 13.0 ml), followed by addition of 10 % palladium-carbon (250 mg). Catalytic hydrogenation was performed at room temperature under atmospheric pressure for 1 hour. After removal of the catalyst by filtration, the filtrate was washed with methylene chloride three times, and the organic solvent in the aqueous phase was removed by distillation under reduced pressure. The reaction mixture was filtered through a membrane filter, and the filtrate was purified by column chromatography (CHP-20P polymer) with a 2 % aqueous tetrahydrofuran solution as an eluent and freeze-dried to give (4R,5S,6S,8R,2'S,4'S)-3-[{1-(3,4-dihydroxycinnamoyl)-2-dimethylaminocarbonylpyrrolidin}-4-ylthio]-4-methyl-6-(1-hydroxyethyl)-1-azabicyclo[3.2.0]hept-2-en-7-one-2-carboxylic acid as a white amorphous substance.

$UV_{max}$ nm ($H_2O$): 218 (sh), 245 (sh), 297.

$IR_{max}$ $cm^{-1}$ (KBr): 3420, 1739, 1634, 1585, 1390, 1289.

NMR δ ppm ($D_2O$): 1.22 (3H, d, J = 6.9 Hz), 1.29 (3H, d, J = 6.6 Hz), 1.83 (1H, m), 2.85 (1H, m), 2.97 (3H, s), 3.19 (3H, s), 6.70 (1H, d, J = 15.5 Hz), 6.94 (1H, d, J = 8.3 Hz), 7.14 (1H, dd, J = 8.3 & 2.0 Hz), 7.22 (1H, d, J = 2.0 Hz), 7.44 (1H, d, J = 15.5 Hz).

Example 3

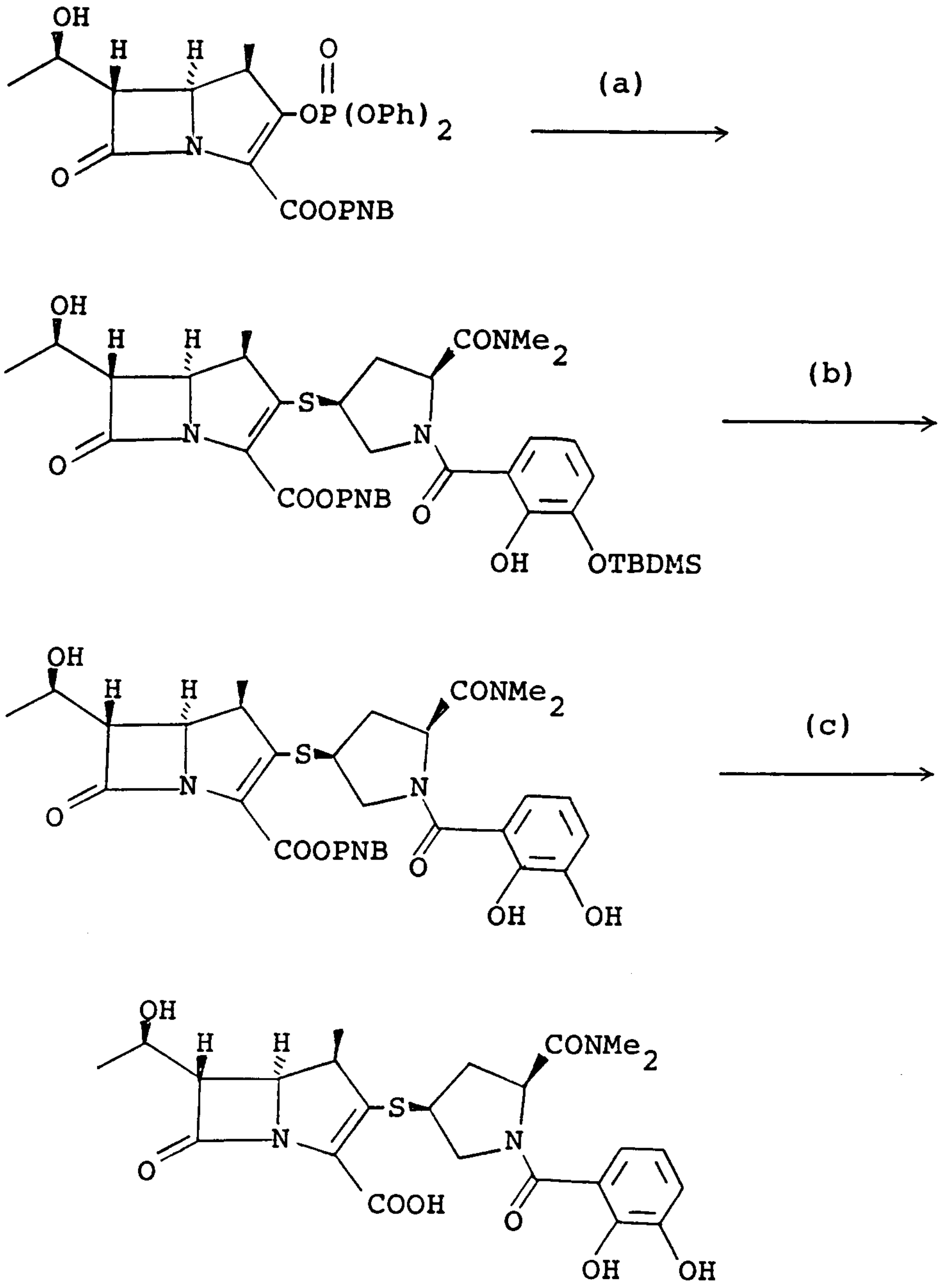


(a) To a solution of (4R,5R,6S,8R)-p-nitrobenzyl-3-diphenylphosphoryloxy-4-methyl-6-(1-hydroxyethyl)-1-azabicyclo[3.2.0]hept-2-en-7-one-2-carboxylate (772 mg), (2S,4S)-1-[(2-hydroxy-3-t-butyldimethylsilyloxy)benzoyl]-2-dimethylaminocarbonyl-4-mercaptopyrrolidine (547 mg) in dry acetonitrile (6 ml), diisopropylethylamine (168 mg) was added under nitrogen stream while ice-cooling, and the resultant mixture was stirred at the same temperature for 50 minutes. The reaction mixture was diluted with ethyl acetate, washed with a 2.5 % aqueous potassium dihydrogen-phosphate solution and an aqueous sodium chloride solution in order and dried over magnesium sulfate and sodium carbonate. After removal of the solvent, the residue was chromatographed on silica gel to give (4R,5S,6S,8R,2'S,4'S))-p-nitrobenzyl-3-[{(1-(2-hydroxy-3-(t-butyldimethylsilyloxy)-benzoyl)-2-dimethylaminocarbonylpyrrolidin}-4-ylthio]-4-methyl-6-(1-hydroxyethyl)-1-azabicyclo[3.2.0]hept-2-en-7-one-2-carboxylate (794 mg).

$IR_{max}$ $cm^{-1}$ (neat): 3375 (br), 1758, 1697, 1627, 1512, 1338, 1250.

NMR $\delta$ ppm ($CDCl_3$): 0.20 (6H, s), 1.00 (9H, s), 1.25 (3H, d, J = 6.3 Hz), 1.34 (3H, d, J = 6.3 Hz), 2.71 (1H, m), 3.01 (3H, s), 3.19 (3H, s), 3.5 - 4.35 (5H, m), 5.0 - 5.6 (3H, m), 6.75 (1H, t, J = 7.9 Hz), 6.94 (1H, d, J = 7.9 Hz), 7.00 (1H, d, J = 5.9 Hz), 7.65 (2H, d, J = 8.9 Hz), 8.23 (2H, d, J = 8.9 Hz).

(b) To a solution of (4R,5S,6S,8R,2'S,4'S)-p-nitrobenzyl-3-[{1-(2-hydroxy-3-(t-butyldimethylsilyloxy)-benzoyl)-2-dimethylaminocarbonylpyrrolidin}-4-ylthio]-4-methyl-6-(1-hydroxyethyl)-1-azabicyclo[3.2.0]hept-2-en-7-one-2-carboxylate (741 mg) in tetrahydrofuran (10 ml), acetic acid (173 mg) was added under ice-cooling, followed by dropwise addition of a 1M tetrahydofuran solution of tetrabutylammonium fluoride (0.96 ml). The resultant mixture was stirred at room temperature for 1 hour, diluted with ethyl acetate and neutralized with an aqueous sodium hydrogen carbonate solution (242 mg). The organic layer was washed with an aqueous sodium chloride solution and dried over magnesium sulfate. Removal of the solvent gave (4R,5S,6S,8R,2'S,4'S)-p-nitrobenzyl-3-[{1-(2,3-dihydroxybenzoyl)-2-dimethylaminocarbonylpyrrolidin}-4-ylthio]-4-methyl-6-(1-hydroxyethyl)-1-azabicyclo[3.2.0]hept-2-en-7-one-2-carboxylate.

$IR_{max}$ $cm^{-1}$ (neat): 3370 (br), 1762, 1700 (br), 1634, 1518, 1344.

NMR $\delta$ ppm ($CDCl_3$): 1.23 (3H, d, J = 7.3 Hz), 1.34 (3H, d, J = 7.3 Hz), 2.76 (1H, m), 3.00 (3H, s), 3.17 (3H, s), 3.5 - 4.3 (5H, m), 5.1 - 5.6 (3H, m), 6.80 (1H, t, J = 7.9 Hz), 6.92 (1H, d, J = 6.3 Hz), 7.01 (1H, d, J = 7.9 Hz), 7.65 (2H, d, J = 8.9 Hz), 8.23 (2H, d, J = 8.9 Hz).

(c) (4R,5S,6S,8R,2'S,4'S)-p-Nitrobenzyl-3-[{1-(2,3-dihydroxybenzoyl)-2-dimethylaminocarbonylpyrrolidin}-4-ylthio]-4-methyl-6-(1-hydroxyethyl)-1-azabicyclo[3.2.0]-hept-2-en-7-one-2-carboxylate (300 mg) was dissolved in tetrahydrofuran (15 ml) and 0.1M phosphate buffer (pH, 7.0; 15 ml), followed by addition of 10 % palladium-carbon (300 mg). Catalytic hydrogenation was performed at room temperature under atmospheric pressure for 2 hours, followed by removal of the catalyst by filtration. The filtrate was washed with methylene chloride three times, and the organic solvent in the aqueous phase was removed by filtration under reduced pressure. The reaction mixture was filtered through a membrane filter, and the filtrate was purified by column chromatography (CHP-20P polymer) with a 2 to 4 % aqueous tetrahydrofuran solution as an eluent and freeze-dried to give (4R,5S,6S,8R,2'S,4'S)-3-[{1-(2,3-dihydroxybenzoyl)-2-dimethylaminocarbonylpyrrolidin}-4-ylthio]-4-methyl-6-(1-hydroxyethyl)-1-azabicyclo[3.2.0]-hept-2-en-7-one-2-carboxylic acid as a white amorphous substance.

$UV_{max}$ nm ($H_2O$): 297.

$IR_{max}$ $cm^{-1}$ (KBr): 3400 (br), 1752, 1634, 1398, 1265.

NMR $\delta$ ppm ($D_2O$): 1.18 (3H, d, J = 7.3 Hz), 1.31 (3H, d, J = 6.3 Hz), 1.88 (1H, m), 2.86 (1H, m), 3.01 (3H, s), 3.22 (3H, s), 6.66 (1H, dd, J = 7.6 & 1.3 Hz), 6.86 (1H, t, J = 7.6 Hz), 7.00 (1H, dd, J = 7.6 & 1.3 Hz).

Example 4

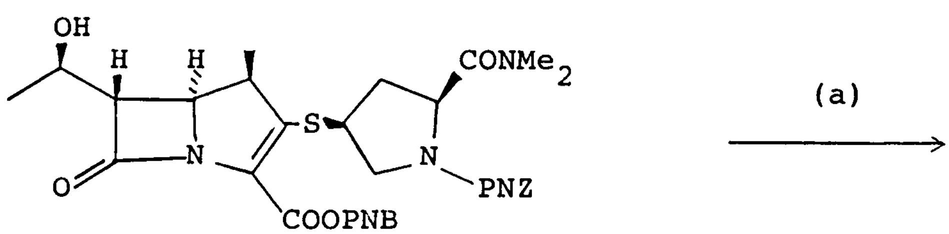

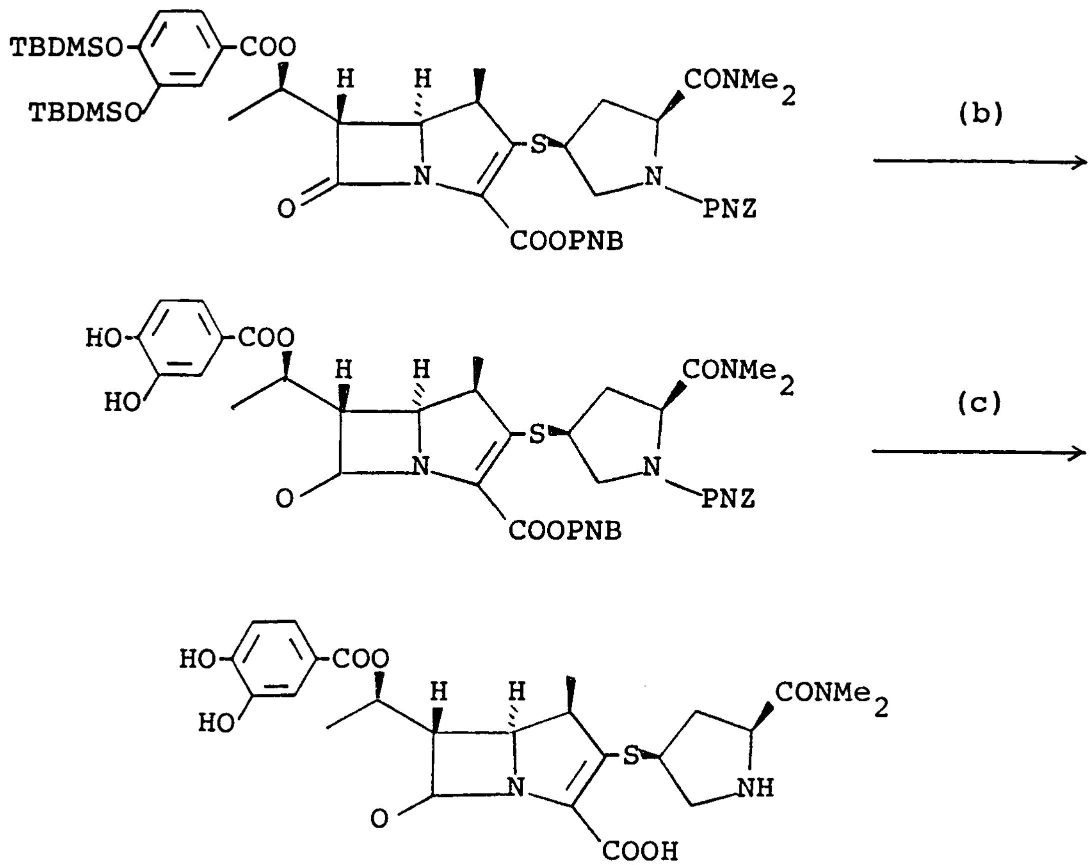


(a) To a solution of (4R,5S,6S,8R,2'S,4'S)-p-nitrobenzyl-3-(1-p-nitrobenzyloxycarbonyl-2-dimethylaminocarbonylpyrrolidin-4-ylthio)-4-methyl-6-(1-hydroxyethyl)-1-azabicyclo[3.2.0]hept-2-en-7-one-2-carboxylate (8.35 g) and 4-dimethylaminopyridine (392 mg) in dry methylene chloride (40 ml), a solution of diisopropylethylamine (2.48 g) in dry methylene chloride (7 ml) was added. To the resultant mixture, a solution of 3,4-di(t-butyldimethylsilyloxy)benzoyl chloride (10.5 mmol) in dry methylene chloride (32 ml) was dropwise added in nitrogen stream while ice-cooling, followed by allowing to react under ice-cooling for 1 hour and at room temperature overnight. The reaction mixture was washed with an aqueous sodium chloride solution, dried over magnesium sulfate and concentrated. The residue was chromatographed on silica gel column to give (4R,5S,6S,8R,2'S,4'S)-p-nitrobenzyl-3-(1-p-nitrobenzyloxycarbonyl-2-dimethylaminocarbonylpyrrolidin-4-ylthio)-4-methyl-6-[1-{3,4-di(t-butyldimethylsilyloxy)benzoyl}oxyethyl]-1-azabicyclo[3.2.0]hept-2-en-7-one-2-carboxylate (3.34 g).

$IR_{max}$ $cm^{-1}$ (neat): 1777, 1708, 1653, 1599, 1515, 1415, 1404, 1346, 1298, 1252, 1207, 1174, 1135, 1111.

NMR $\delta$ ppm ($CDCl_3$): 0.20 (3H, s), 0.21 (3H, s), 0.22 (6H, s), 0.98 (18H, s), 1.27 (3H, m), 1.52 (3H, m), 1.94 (1H, m), 2.72 (1H, m), 2.93 - 3.10 (6H, m), 3.3 - 3.8 (3H, m), 4.0 - 4.2 (1H, m), 4.32 (1H, m), 4.72 (1H, m), 5.03 - 5.51 (5H, m), 6.84 (1H, d, J = 7.9 Hz), 7.4 - 7.7 (6H, m), 8.18 - 8.23 (4H, m).

(b) To a solution of (4R,5S,6S,8R,2'S,4'S)-p-nitrobenzyl-3-(1-p-nitrobenzyloxycarbonyl-2-dimethylaminocarbonylpyrrolidin-4-ylthio]-4-methyl-6-[1-{(3,4-di(t-butyldimethylsilyloxy)-benzoyl}oxyethyl]-1-azabicyclo[3.2.0]hept-2-en-7-one-2-carboxylate (1.0 g) in tetrahydrofuran (12 ml), acetic acid (339 mg) was added at room temperature, followed by dropwise addition of a 1M tetrahydofuran solution of tetrabutylammonium fluoride (1.9 ml). The resultant mixture was stirred at room temperature for 1 hour, diluted with ethyl acetate and neutralized with an aqueous solution of sodium hydrogen carbonate (475 mg). The organic layer was washed with water, dried over magnesium sulfate and concentrated. The residue was chromatographed on silica gel column to give (4R,5S,6S,8R,2'S,4'S)-p-nitrobenzyl-3-(1-p-nitrobenzyloxycarbonyl-2-dimethylaminocarbonylpyrrolidin-4-ylthio)-4-methyl-6-[1-(3,4-dihydroxybenzoyl)oxyethyl]-1-azabicyclo[3.2.0]hept-2-en-7-one-2-carboxylate (316 mg).

$IR_{max}$ $cm^{-1}$ (neat): 3300 (br), 1764, 1700, 1640, 1633, 1601, 1514, 1434, 1400, 1342, 1287, 1206,

1177, 1107.

NMR δ ppm ($CDCl_3$): 1.28 (3H, m), 1.49 (3H, m), 2.72 (1H, m), 2.94 - 3.17 (6H, m), 3.4 - 3.6 (2H, m), 4.12 (1H, m), 4.33 (1H, m), 4.79 (1H, m), 4.89 - 5.50 (5H, m), 6.80 - 6.88 (1H, m), 7.10 (1H, d, J = 8.2 Hz), 7.37 - 7.65 (5H, m), 8.1 - 8.2 (4H, m).

(c) (4R,5S,6S,8R,2'S,4'S)-p-Nitrobenzyl-3-(1-p-nitrobenzyloxycarbonyl-2-dimethylaminocarbonylpyrrolidin)-4-ylthio)-4-methyl-6-[1-(3,4-dihydroxybenzoyl)oxyethyl]-1-azabicyclo[3.2.0]hept-2-en-7-one-2-carboxylate (33 mg) was dissolved in tetrahydrofuran (1.5 ml) and 0.1M phosphate buffer (pH, 7.0; 1.5 ml), followed by addition of 10 % palladium-carbon (41 mg). Catalytic hydrogenation was performed at room temperature under atmospheric pressure for 1 hour. After removal of the catalyst by filtration, the filtrate was washed with methylene chloride three times and the organic solvent in the aqueous phase was removed by distillation under reduced pressure. The residual solution was filtered through a membrane filter, and the filtrate was purified by column chromatography (CHP-20P polymer) with a 2 to 16 % aqueous tetrahydrofuran solution as an eluent and freeze-dried to give (4R,5S,6S,8R,2'S,4'S)-3-(2-dimethylaminocarbonylpyrrolidin-4-ylthio)-4-methyl-6-[1-(3,4-di hydroxybenzoyl)-oxyethyl]-1-azabicyclo-[3.2.0]hept-2-en-7-one-2-carboxylic acid as a white amorphous substance.

$UV_{max}$ nm ($H_2O$): 262, 294.

$IR_{max}$ $cm^{-1}$ (KBr): 3440 (br), 1758, 1602, 1394, 1282, 1223.

NMR δ ppm ($D_2O$): 1.25 (3H, m), 1.47 (3H, d, J = 4.3 Hz), 2.99 (3H, s), 3.08 (3H, s), 5.50 (1H, m), 6.91 (1H, m), 7.55 (2H, m).

Example 5

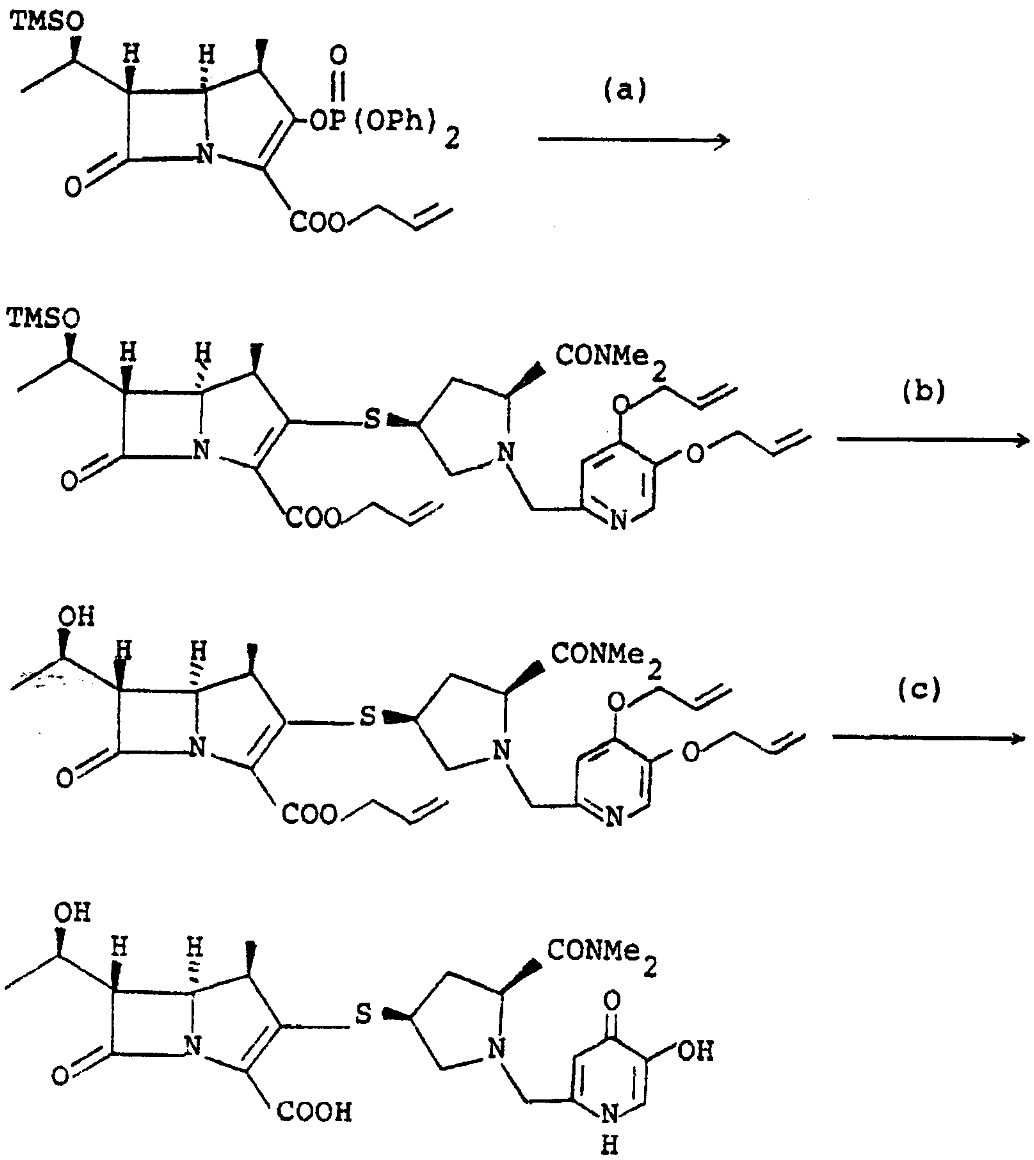


(a) To a solution of (4R,5R,5S,8R)-allyl-3-diphenylphosphoryloxy-4-methyl-6-(1-trimethylsilyloxyethyl)-1-azabicyclo[3.2.0]hept-2-en-7-one-2-carboxylate (154 mg) and diisopropylethylamine (47 mg) in dry acetonitrile (3 ml), a solution of (2S,4S)-1-(4,5-diallyloxy-2-pyridyl)methyl-2-dimethylaminocarbonyl-4-mercaptopyrrolidine (102 mg) in dry acetonitrile (3 ml) was added, and the resultant mixture was allowed to react overnight. The reaction mixture was diluted with ethyl acetate, washed with water and an aqueous sodium chloride solution in order, dried over magnesium sulfate and sodium carbonate and concentrated. The residue was chromatographed on silica gel column to give (4R,5S,6S,8R,2'S,4'S)-allyl-3-[{1-(4,5-diallyloxy-2-pyridyl)methyl-2-dimethylaminocarbonylpyrrolidin}-4-ylthio]-4-methyl-6-(1-trimethylsilyloxyethyl)-1-azabicyclo[3.2.0]-hept-2-en-7-one-2-carboxylate (88 mg).

$IR_{max}$ $cm^{-1}$ (neat): 1768, 1700, 1644, 1507, 1320, 1250, 1207, 1140.

NMR δ ppm ($CDCl_3$): 0.13 (9H, s), 1.21 (3H, d, J = 7.3 Hz), 1.26 (3H, d, J = 6.3 Hz), 1.93 (1H, m), 2.64 (1H, m), 2.8 - 3.4 (4H, m), 2.92 (3H, s), 3.06 (3H, s), 3.50 - 4.05 (4H, m), 4.05 (1H, dd, J = 2.6 & 9.2 Hz), 4.19 (1H, m), 4.50 - 4.85 (6H, m), 5.20 - 5.55 (6H, m), 5.85 - 6.20 (3H, m), 7.10 (1H, s), 8.04 (1H, s).

(b) To a solution of (4R,5S,6S,8R,2'S,4'S)-allyl-3-[{1-(4,5-diallyloxy-2-pyridyl)methyl-2-dimethylaminocarbonylpyrrolidin}-4-ylthio]-4-methyl-6-(1-trimethylsilyloxyethyl)-1-azabicyclo[3.2.0]hept-2-en-7-one-2-carboxylate (88 mg) in tetrahydrofuran (1.6 ml), acetic acid (31 mg) was added under ice-cooling, followed by dropwise addition of a 1M tetrahydrofuran solution of tetrabutylammonium fluoride (0.13 ml). The resultant mixture was stirred for 1 hour, diluted with ethyl acetate and neutralized with an aqueous solution of sodium hydrogen carbonate (55 mg). The organic layer was washed with water and

an aqueous sodium chloride solution and dried over magnesium sulfate and concentrated to give (4R,5S,6S,8R,2'S,4'S)-allyl-3-[{1-(4,5-diallyloxy-2-pyridyl)methyl-2-dimethylaminocarbonylpyrrolidin}-4-ylthio]-4-methyl-6-(1-hydroxyethyl)-1-azabicyclo[3.2.0]hept-2-en-7-one-2-carboxylate (73 mg).

$IR_{max}$ $cm^{-1}$ (neat): 3350 (br), 1760, 1690, 1633, 1504, 1315.

NMR $\delta$ ppm ($CDCl_3$): 1.20 (3H, d, J = 7.3 Hz), 1.35 (3H, d, J = 6.3 Hz), 1.89 (1H, m), 2.93 (3H, s), 3.06 (3H, s), 3.21 (1H, dd, J = 2.6 & 7.3 Hz), 3.55 - 3.90 (2H, m), 3.90 - 4.30 (2H, m), 4.50 - 4.90 (6H, m), 5.15 - 5.55 (6H, m), 5.85 - 6.20 (3H, m), 7.14 (1H, s), 8.04 (1H, s).

(c) To a solution of (4R,5S,6S,8R,2'S,4'S)-allyl-3-[{1-(4,5-diallyloxy-2-pyridyl)methyl-2-dimethylaminocarbonylpyrrolidin}-4-ylthio]-4-methyl-6-(1-hydroxyethyl)-1-azabicyclo[3.2.0]hept-2-en-7-one-2-carboxylate (85 mg) in monochlorobenzene (2 ml), aniline (0.124 ml) and tetrakistriphenylphosphine palladium (47 mg) were added, and the resultant mixture was stirred for 45 minutes. The reaction mixture was separated with addition of 0.2M phosphate buffer (pH, 7.0), and the aqueous layer was washed with methylene chloride. After removal of the solvent in the aqueous layer by distillation under reduced pressure, the residue was purified by column chromatography (CHP-20P polymer) with a 2 to 8 % aqueous tetrahydrofuran solution as an eluent and freeze-dried to give (4R,5S,6S,8R,2'S,4'S)-3-[{1-(5-hydroxy-4-pyridon-2-yl)methyl-2-dimethylaminocarbonylpyrrolidin}-4-ylthio]-4-methyl-6-(1-hydroxyethyl)-1-azabicyclo[3.2.0]-hept-2-en-7-one-2-carboxylic acid as a white amorphous substance.

$UV_{max}$ nm ($H_2O$): 277, 302 (sh)

$IR_{max}$ $cm^{-1}$ (KBr): 3400 (br), 1756, 1635, 1558, 1506, 1394, 1260.

NMR $\delta$ ppm ($D_2O$): 1.20 (3H, d, J = 7.3 Hz), 1.29 (3H, d, J = 6.3 Hz), 1.77 (1H, m), 2.85 (3H, s), 3.07 (3H, s), 2.85 - 3.50 (6H, m), 3.92 (2H, s), 4.16 (2H, m), 4.23 (1H, m), 6.62 (1H, s), 7.71 (1H, s).

Example 6

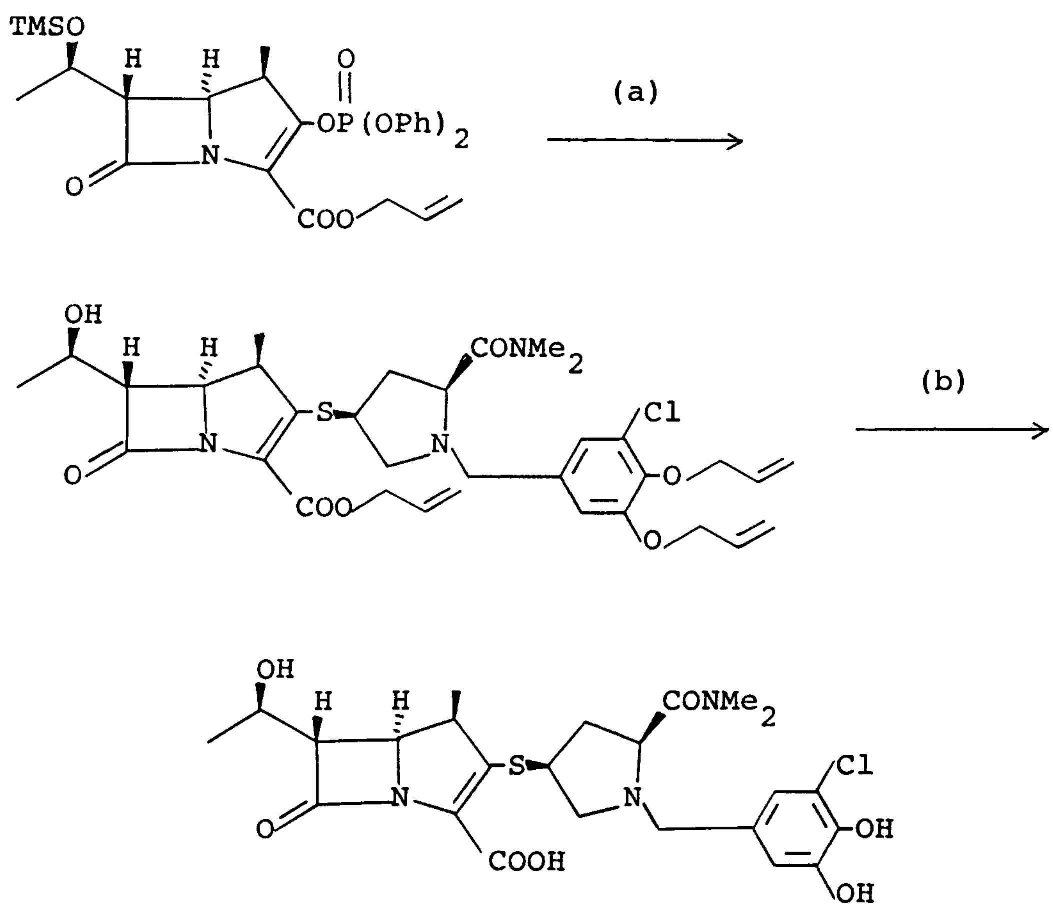


(a) To a solution of (4R,5R,6S,8R)-allyl-3-diphenylphosphoryloxy-4-methyl-6-(1-trimethylsilyloxyethyl)-1-azabicyclo[3.2.0]hept-2-en-7-one-2-carboxylate (114 mg) and diisopropylethylamine (35 mg) in dry acetonitrile (1 ml), a solution of (2S,4S)-1-(4,5-diallyloxy-3-chlorophenyl)methyl-2-dimethylaminocarbonyl-4-mercaptopyrrolidine (93 mg) in dry acetonitrile (1 ml) was added in nitrogen stream while ice-cooling, and the resultant mixture was allowed to react for 1 hour. 1,8-Diazabicyclo[5.4.0]undec-7-ene (DBU) (0.039 ml) was added to the reaction mixture, and the reaction was continued for 1 hour. The reaction mixture was diluted with ethyl acetate, washed with water and dried over magnesium sulfate and sodium carbonate. After removal of the solvent, the residue was combined with ethyl acetate (10 ml), and 1N hydrochloric acid (0.5 ml) was added thereto under ice-cooling and vigorously stirred for 5 minutes. The reaction mixture was diluted with ethyl acetate, washed with an aqueous sodium hydrogen carbonate solution and an aqueous sodium chloride solution in order, dried and concentrated. The residue was chromatographed on silica gel column to give (4R,5S,6S,8R,2'S,4'S)-allyl-3-[{1-(4,5-diallyloxy-3-chlorophenyl)methyl-2-dimethylaminocarbonylpyrrolidin}-4-ylthio]-4-methyl-6-(1-hydroxyethyl)-1-azabicyclo[3.2.0]hept-2-en-7-one-2-carboxylate.

$IR_{max}$ $cm^{-1}$ (neat): 3350, 1758, 1688, 1627, 1475, 1412, 1312, 1265, 1198, 1125.

NMR $\delta$ ppm ($CDCl_3$): 1.22 (3H, d, J = 7.3 Hz), 1.34 (3H, d, J = 6.3 Hz), 1.97 (1H, m), 2.66 (1H, m), 2.81 (1H, dd, J = 9.9 & 7.3 Hz), 2.90 (3H, s), 3.02 (3H, s), 3.13 (1H, dd, J = 9.6 & 4.6 Hz), 3.20 (1H, dd, J = 7.3 & 2.4 Hz), 3.36 (1H, m), 3.41 (1H, d, J = 13.2 Hz), 3.59 (1H, m), 3.70 (1H, m), 3.83 (1H, d, J = 13.2 Hz), 4.17 (2H, m), 4.56 (4H, m), 4.67 (1H, m), 4.80 (1H, m), 5.20 - 5.40 (6H, m), 5.90 - 6.24 (3H, m), 6.88 (2H, m).

(b) To a solution of (4R,5S,6S,8R,2'S,4'S)-allyl-3-[{1-(4,5-diallyloxy-3-chlorophenyl)methyl-2-dimethylaminocarbonylpyrrolidin}-4-ylthio]-4-methyl-6-(1-hydroxyethyl)-1-azabicyclo[3.2.0]hept-2-en-7-one-2-carboxylate (90 mg) in monochlorobenzene (2 ml), aniline (0.181 ml) and tetrakistriphenylphosphine palladium (47 mg) were added in nitrogen stream under ice-cooling, and stirring was continued for 50 minutes. The reaction mixture was combined with 0.1M phosphate buffer (pH, 7.0), and the aqueous layer was separated and washed with methylene chloride two times. After removal of the solvent in the aqueous layer by distillation under reduced pressure, the residue was purified by column chromatography (CHP-20P polymer) with a 2 to 4 % aqueous tetrahydrofuran solution as an eluent and freeze-dried to give (4R,5S,6S,8R,2'S,4'S)-3-[{1-(4,5-dihydroxy-3-chlorophenyl)methyl-2-dimethylaminocarbonylpyrrolidin}-4-ylthio]-4-methyl-6-(1-hydroxyethyl)-1-azabicyclo[3.2.0]hept-2-en-7-one-2-carboxylic acid as a white amorphous substance.

$UV_{max}$ nm ($H_2O$): 297.

$IR_{max}$ $cm^{-1}$ (KBr): 3380 (br), 1754, 1593, 1392, 1291.

NMR $\delta$ ppm ($D_2O$): 1.19 (3H, d, J = 7.3 Hz), 1.31 (3H, d, J = 6.6 Hz), 1.71 (1H, m), 2.76 (3H, s), 2.85 (1H, m), 2.95 (3H, s), 3.37 (4H, m), 3.75 (1H, d, J = 12.5 Hz), 3.90 (1H, m), 4.00 (1H, d, J = 12.5 Hz), 4.22 (3H, m), 6.79 (1H, s), 6.91 (1H, s).

## Examples 7 to 60

In the same manner as in Examples 1 to 6, the compounds as shown in Tables 2, 3, 4, 5-(1) and 5-(2) were obtained.

Table 2

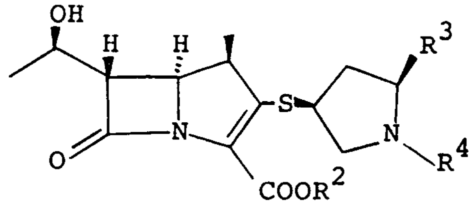

| Example No. | $R^2$ | $R^4$ | $R^3$ |
|---|---|---|---|
| 7-1 | PNB | 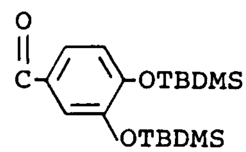 | $CONMe_2$ |
| 7-2 | PNB | 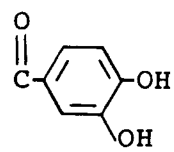 | $CONMe_2$ |
| 7-3 | H | 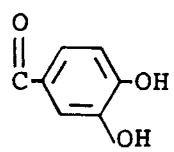 | $CONMe_2$ |
| 8-1 | PNB | 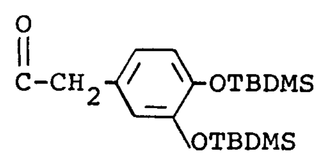 | $CONMe_2$ |
| 8-2 | PNB | 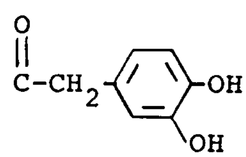 | $CONMe_2$ |
| 8-3 | H | 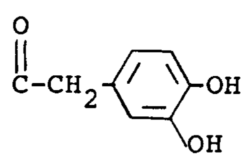 | $CONMe_2$ |
| 9-1 | PNB | 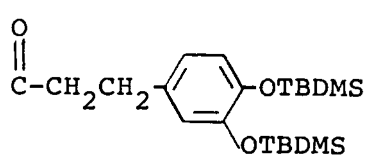 | $CONMe_2$ |

| | | | |
|---|---|---|---|
| 9-2 | PNB | 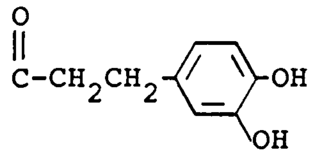 | $CONMe_2$ |
| 9-3 | H | 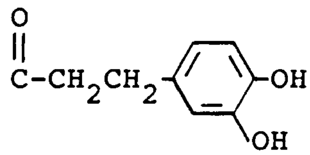 | $CONMe_2$ |
| 10-1 | PNB | 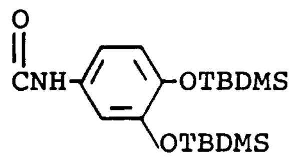 | $CONMe_2$ |
| 10-2 | PNB | 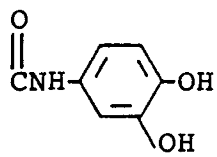 | $CONMe_2$ |
| 10-3 | H | 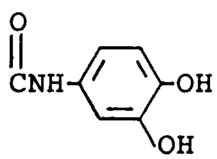 | $CONMe_2$ |
| 11-1 | PNB | AOC | $CONH_2$ |
| 11-2 | PNB | 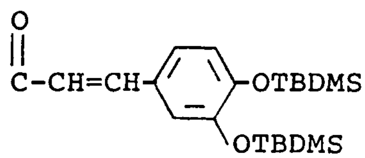 | $CONH_2$ |
| 11-3 | PNB | 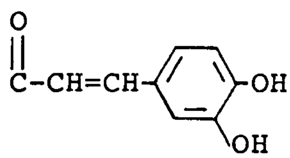 | $CONH_2$ |
| 11-4 | H | 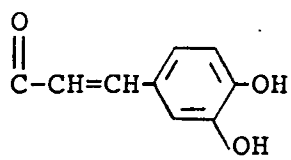 | $CONH_2$ |
| 12-1 | PNB | AOC | $CH_2OH$ |

| | | | |
|---|---|---|---|
| 12-2 | PNB | 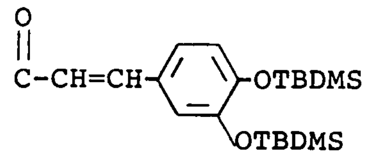 | $CH_2OH$ |
| 12-3 | PNB | 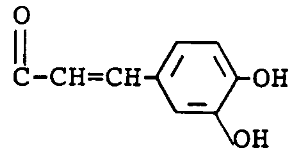 | $CH_2OH$ |
| 12-4 | H | 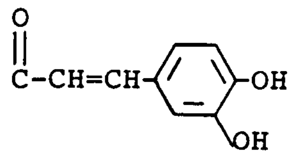 | $CH_2OH$ |
| 13-1 | PNB | AOC | 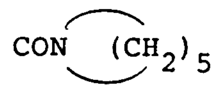 |
| 13-2 | PNB | 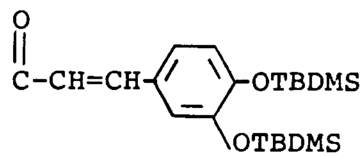 | 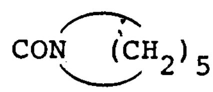 |
| 13-3 | PNB | 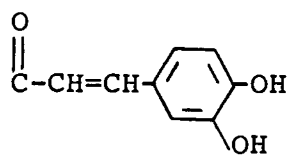 | 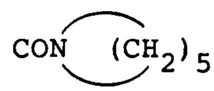 |
| 13-4 | H | 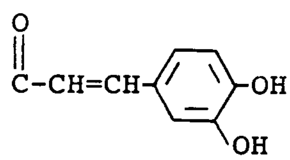 | 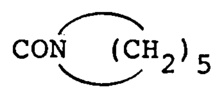 |
| 14-1 | PNB | 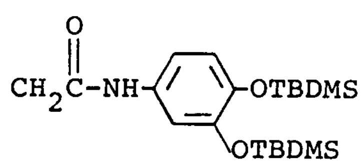 | $CONMe_2$ |
| 14-2 | PNB | 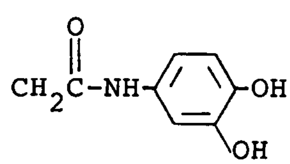 | $CONMe_2$ |

| | | | |
|---|---|---|---|
| 14-3 | H | 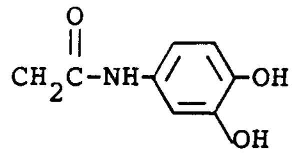 | CONMe$_2$ |
| 15-1 | $CH_2CH=CH_2$ | 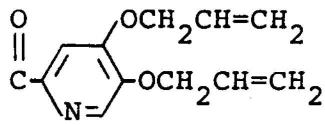 | CONMe$_2$ |
| 15-2 | H | 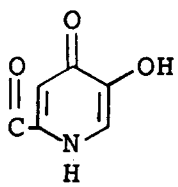 | CONMe$_2$ |
| 16-1 | PNB | 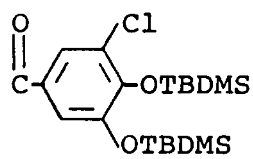 | CONMe$_2$ |
| 16-2 | PNB | 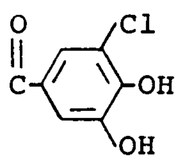 | CONMe$_2$ |
| 16-3 | H | 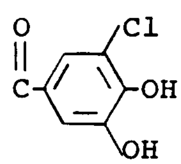 | CONMe$_2$ |
| 17-1 | $CH_2CH=CH_2$ | 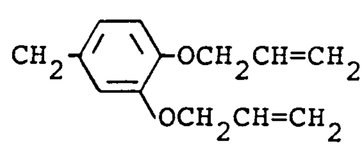 | CONMe$_2$ |
| 17-2 | H | 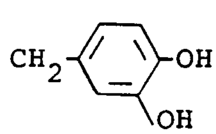 | CONMe$_2$ |

| | | | |
|---|---|---|---|
| 18-1 | $CH_2CH=CH_2$ | 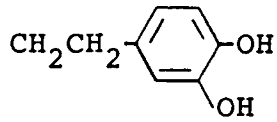 | $CONMe_2$ |
| 18-2 | H | 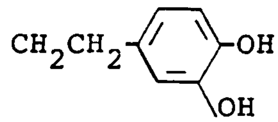 | $CONMe_2$ |
| 19-1 | $CH_2CH=CH_2$ | 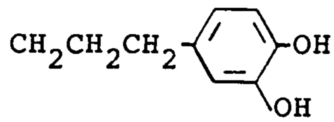 | $CONMe_2$ |
| 19-2 | H | 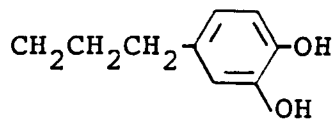 | $CONMe_2$ |
| 20-1 | $CH_2CH=CH_2$ | 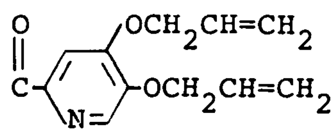 | $CH_2NH-AOC$ |
| 20-2 | H | 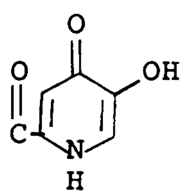 | $CH_2NH_2$ |
| 21-1 | $CH_2CH=CH_2$ | 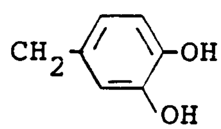 | $CONH_2$ |
| 21-2 | H | 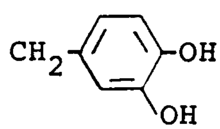 | $CONH_2$ |
| 22-1 | $CH_2CH=CH_2$ | 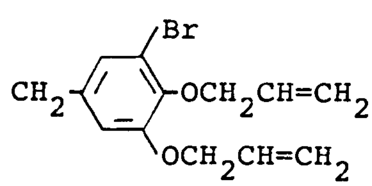 | $CONMe_2$ |

| | | | |
|---|---|---|---|
| 22-2 | H | 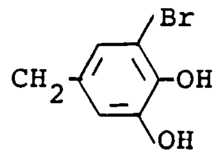 | $CONMe_2$ |
| 23-1 | $CH_2CH=CH_2$ | 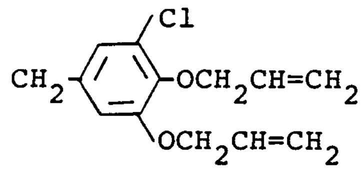 | $CONH_2$ |
| 23-2 | H | 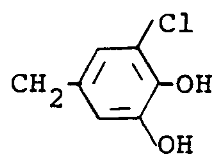 | $CONH_2$ |
| 24-1 | $CH_2CH=CH_2$ | 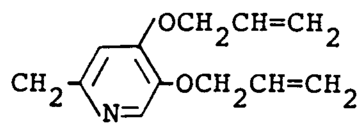 | $CONH_2$ |
| 24-2 | H | 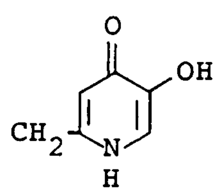 | $CONH_2$ |
| 25-1 | H | 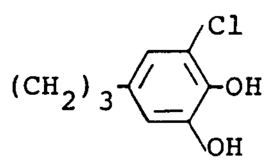 | $CONMe_2$ |
| 26-1 | $CH_2CH=CH_2$ | 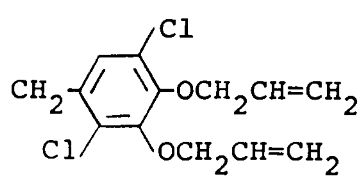 | $CONMe_2$ |

| | | | |
|---|---|---|---|
| 26-2 | H | 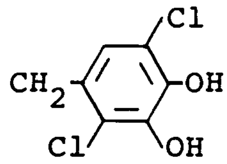 | $CONMe_2$ |
| 27-1 | $CH_2CH=CH_2$ | 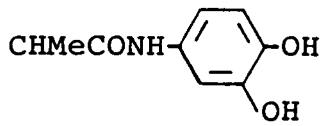 | $CONMe_2$ |
| 27-2 | H | 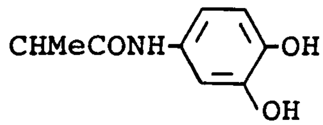 | $CONMe_2$ |
| 28-1 | $CH_2CH=CH_2$ | 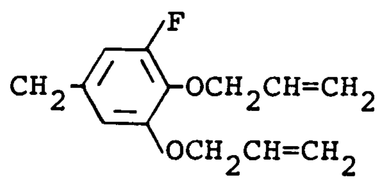 | $CONMe_2$ |
| 28-2 | H | 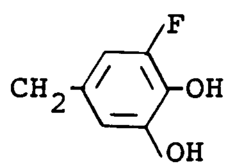 | $CONMe_2$ |
| 29-1 | $CH_2CH=CH_2$ | 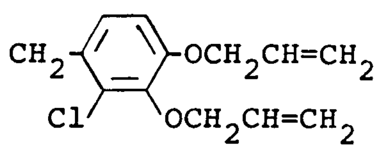 | $CONMe_2$ |
| 29-2 | H | 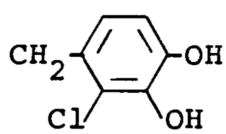 | $CONMe_2$ |

Physical property

Example 7-1

$IR_{max}$ $cm^{-1}$ (neat): 3400 (br), 1700, 1700, 1635, 1610 (sh), 1518, 1342, 1296.

NMR $\delta$ ppm ($CDCl_3$): 0.19 (3H, s) 0.20 (3H, s), 0.21 (6H, s), 0.97 (9H, s), 0.98 (9H, s), 1.25 (3H, d, J = 6.9 Hz), 1.35 (3H, d, J = 6.3 Hz), 2.6 - 2.8 (1H, m), 2.88 - 3.40 (8H, m), 3.45 - 4.10 (3H, m), 4.23 (1H, m), 5.1 - 5.6 (3H, m), 6.83 (1H, d, J = 8.9 Hz), 7.13 (1H, s), 7.65 (3H, d, J = 8.9 Hz), 8.23 (2H, d, J = 8.9 Hz).

Example 7-2

$IR_{max}$ $cm^{-1}$ (KBr): 3360 (br), 1760, 1700, 1630, 1595, 1510, 1427, 1338, 1270, 1190

| | |
|---|---|
| | (sh), 1185, 1127. |
| NMR δ ppm ($CDCl_3$-$CD_3OD$ (20:1)): | 1.24 (3H, d, J = 6.9 Hz), 3.00 (3H, s), 3.22 (3H, s), 3.45 - 4.00 (3H, m), 5.0 - 5.6 (3H, m), 6.85 (1H, d, J = 8.9 Hz), 7.02 (1H, s), 7.65 (3H, d, J = 8.9 Hz), 8.23 (2H, d, J = 8.9 Hz). |

Example 7-3

| | |
|---|---|
| $UV_{max}$ nm ($H_2O$): | 256, 288. |
| $IR_{max}$ $cm^{-1}$ (KBr): | 3420 (br), 1745, 1630, 1590, 1400, 1286, 1262. |
| NMR δ ppm ($D_2O$): | 1.19 (3H, d, J = 7.3 Hz), 1.29 (3H, d, J = 6.0 Hz), 1.86 (1H, m), 2.90 (1H, m), 3.01 (3H, s), 3.24 (3H, s), 3.42 (2H, m), 3.73 (2H, m), 4.12 (1H, m), 4.28 (2H, m), 5.18 (1H, m), 6.80 - 7.22 (3H, m). |

Example 8-1

| | |
|---|---|
| $IR_{max}$ $cm^{-1}$ (neat): | 3425 (br), 1775, 1707, 1647, 1514, 1347. |
| NMR δ ppm ($CDCl_3$): | 0.18 (12H, s), 0.97 (18H, s), 1.18 (3H, d, J = 7.3 Hz), 1.37 (3H, d, J = 6.3 Hz), 1.95 (1H, m), 2.55 (1H, m), 3.3 - 3.7 (4H, m), 3.91 (1H, m), 4.05 - 4.35 (3H, m), 4.57 (1H, m), 4.87 (1H, t, J = 8.1 Hz), 5.15 - 5.60 (2H, m), 6.75 (3H, m), 7.65 (2H, d, J = 8.9 Hz), 8.23 (2H, d, J = 8.9 Hz). |

Example 8-2

| | |
|---|---|
| $IR_{max}$ $cm^{-1}$ (KBr): | 3356, 1775, 1709, 11642, 1521, 1445, 1347, 1210, 1140, 1046. |
| NMR δ ppm ($CDCl_3$): | 1.49 (3H, d, J = 5.9 Hz), 2.53 (1H, m), 2.98 (3H, s), 3.18 (3H, s), 3.4 - 3.9 (6H, m), 3.94 (1H, dd, J = 2.0 & 8.6 Hz), 4.30 (1H, m), 4.98 (1H, t, J = 8.4 Hz), 5.15 - 5.55 (2H, m), 6.58 (1H, d, J = 7.6 Hz), 6.74 (1H, d, J = 2.0 Hz), 6.84 (1H, d, J = 7.9 Hz), 7.61 (2H, d, J = 8.9 Hz), 8.21 (2H, d, J = 8.9 Hz). |

Example 8-3

| | |
|---|---|
| $UV_{max}$ nm ($H_2O$): | 288, 302. |
| $IR_{max}$ $cm^{-1}$ (KBr): | 3370 (br), 1763, 1638, 1517, 1447, 1285, 1210, 1147. |
| NMR δ ppm ($D_2O$): | 1.10 (3H, d, J = 7.3 Hz), 1.34 (3H, d, J = 6.6 Hz), 1.78 (1H, m), 2.83 (1H, m), 2.98 (3H, s), 3.18 (3H, s), 6.57 (1H, d, J = 8.3 Hz), 6.69 (1H, s), 6.83 (1H, d, J = 8.3 Hz). |

Example 9-1

| | |
|---|---|
| $IR_{max}$ $cm^{-1}$ (neat): | 3380 (br), 1765, 1640, 1507, 1340. |
| NMR δ ppm ($CDCl_3$): | 0.18 (12H, s), 0.98 (18H, s), 1.28 (3H, d, J = 6.9 Hz), 1.37 (3H, d, J = 5.9 Hz), 1.98 (1H, m), 2.40 - 2.75 (2H, m), 2.82 (1H, m), 2.99 (3H, s), 3.16 (3H, s), 3.20 - 3.45 (2H, m), 3.58 (1H, m), 4.26 (1H, dd, J = 2.6 & 9.2 Hz), 4.85 (1H, t, J = 8.1 Hz), 5.2 - 5.6 (2H, m), 6.55 - 6.85 (2H, m), 7.28 (1H, m), 7.65 (2H, d, J = 8.9 Hz), 8.23 (2H, d, J = 8.9 Hz). |

Example 9-2

| | |
|---|---|
| $IR_{max}$ $cm^{-1}$ (KBr): | 3364, 1770, 1709, 1636, 1522, 1447, 1376, 1348, 1284, 1210, 1140, 1112, 1047. |
| NMR δ ppm ($CDCl_3$): | 1.25 (3H, d, J = 7.3 Hz), 1.49 (3H, d, J = 6.3 Hz), 1.84 (1H, m), 2.35 - 2.95 (6H, m), 2.98 (3H, s), 3.19 (3H, s), 3.05 - 3.35 (2H, m), 4.08 (1H, m), 4.31 (1H, m), 4.83 (1H, m), 5.15 - 5.55 (2H, m), 6.5 - 6.9 (3H, m), 7.61 (2H, d, J = 8.9 Hz), 8.21 (2H, d, J = 8.9 Hz). |

Example 9-3

| | |
|---|---|
| $UV_{max}$ nm ($H_2O$): | 300 (sh), 288. |
| $IR_{max}$ $cm^{-1}$ (KBr): | 3400 (br), 1758, 1637, 1524, 1449, 1287, 1150. |

NMR δ ppm ($D_2O$): 1.18 (3H, d, J = 7.3 Hz), 1.38 (3H, d, J = 6.3 Hz), 1.66 (1H, m), 2.56 (1H, m), 2.97 (3H, s), 3.18 (3H, s), 3.46 (3H, m), 3.98 (1H, t, J = 7.9 Hz), 3.85 (4H, m), 4.12 - 4.30 (3H, m), 4.30 (2H, t, J = 6.6 Hz), 6.70 (1H, dd, J = 2.3 & 8.2 Hz), 6.78 (1H, d, J = 2.3 Hz), 6.98 (1H, d, J = 8.2 Hz).

Example 10-1

$IR_{max}$ $cm^{-1}$ (neat): 3300 (br), 1763, 1700, 1638, 1597, 1505, 1416, 1342, 1270, 1245, 1208, 1138.
NMR δ ppm ($CDCl_3$): 0.16 (12H, s), 0.94 (9H, s), 0.96 (9H, s), 1.23 (3H, d, J = 7.3 Hz), 1.33 (3H, d, J = 6.3 Hz), 2.70 (1H, m), 2.90 (3H, s), 3.06 (3H, s), 3.20 (1H, dd, J = 2.3 & 7.3 Hz), 3.3 - 3.6 (2H, m), 3.73 (1H, m), 4.02 (2H, m), 4.18 (1H, m), 5.01 (1H, t, J = 8.1 Hz), 5.15 - 5.60 (2H, m), 6.70 (1H, d, J = 8.6 Hz), 6.84 (1H, dd, J = 2.6 & 8.6 Hz), 6.99 (1H, m), 7.65 (2H, d, J = 8.9 Hz), 8.23 (2H, d, J= 8.9 Hz).

Example 10-2

$IR_{max}$ $cm^{-1}$ (KBr): 3392 (br), 2361, 1769, 1642, 1521, 1446, 1347, 1212, 1140.
NMR δ ppm ($CDCl_3$-$CD_3OD$ (20:1)): 1.19 (3H, d, J = 6.9 Hz), 1.34 (3H, d, J = 6.3 Hz), 2.62 (1H, m), 2.91 (3H, s), 3.06 (3H, s), 3.20 (1H, dd, J = 2.5 & 7.5 Hz), 3.55 (1H, m), 3.92 (1H, m), 4.11 (3H, m), 4.81 (1H, m), 5.2 - 5.6 (2H, m), 6.54 (1H, d, J = 6.6 Hz), 6.69 (1H, d, J = 8.3 1H), 6.90 (1H, t, J = 2.0 Hz), 7.64 (2H, d, J = 8.9 Hz), 8.23 (2H, d, J = 8.9 Hz).

Example 10-3

$UV_{max}$ nm ($H_2O$): 238, 293.
$IR_{max}$ $cm^{-1}$ (KBr): 3300 (br), 1754, 1638, 1514, 1390, 1252.
NMR δ ppm ($D_2O$): 1.24 (3H, d, J = 6.9 Hz), 1.32 (3H, d, J = 6.3 Hz), 1.84 (1H, m), 2.88 (1H, m), 2.97 (3H, s), 3.17 (3H, s), 3.47 (3H, m), 3.88 (1H, m), 4.11 (1H, m), 4.27 (3H, m), 6.70 (1H, dd, J = 2.3 & 8.6 Hz), 6.87 (1H, d, J = 8.6 Hz), 6.88 (1H, d, J = 2.3 Hz).

Example 11-1

$IR_{max}$ $cm^{-1}$ (neat): 3400 (br), 1764, 1696, 1680, 1518, 1410,
NMR δ ppm ($CDCl_3$): 1.28 (3H, d, J = 7.6 Hz), 1.37 (3H, d, J = 6.3 Hz), 3.25 - 3.55 (3H, m), 4.0 - 4.2 (1H, m), 4.28 (2H, dd, J = 2.6 & 9.2 Hz), 4.30 (1H, t, J = 7.3 Hz), 5.2 - 5.7 (5H, m), 5.90 (1H, m), 7.66 (2H, d, J = 8.9 Hz), 8.24 (2H, d, J = 8.9 Hz).

Example 11-2

$IR_{max}$ $cm^{-1}$ (neat): 3370 (br), 1762, 1678, 1640, 1587, 1510, 1420, 1340, 1300, 1247, 1207.
NMR δ ppm ($CDCl_3$): 0.20 (6H, s), 0.22 (6H, s), 0.98 (9H, s), 0.99 (9H, s), 1.25 (3H, d, J = 7.3 Hz), 1.36 (3H, d, J = 6.3 Hz), 2.58 (1H, m), 3.2 - 3.5 (2H, m), 3.64 (1H, m), 3.79 (1H, m), 6.83 (1H, d, J = 7.9 Hz), 6.96 (2H, m), 7.63 (3H, m), 8.23 (2H, d, J = 8.9 Hz).

Example 11-3

$IR_{max}$ $cm^{-1}$ (KBr): 3330 (br), 1768, 1680, 1646, 1604, 1521, 1421, 1346, 1286, 1211, 1138.
NMR δ ppm ($CDCl_3$-$CD_3OD$ (9:1)): 1.25 (3H, d, J = 7.3 Hz), 1.34 (3H, d, J = 6.3 Hz), 2.28 (1H, m), 2.64 (1H, m), 3.71 (1H, m), 4.05 - 4.45 (3H, m), 4.62 (1H, m), 5.2 - 5.6 (2H, m), 6.49 (1H, d, J = 16 Hz), 6.82 (1H, d, J = 7.9 Hz), 6.85 - 7.15 (2H, m), 7.66 (2H, d, J = 8.8 Hz), 8.23 (2H, d, J = 8.8 Hz).

Example 11-4

UV$_{max}$ nm ($H_2O$): 218, 242 (sh), 300.
IR$_{max}$ cm$^{-1}$ (KBr): 3400 (br), 1680, 1598, 1414, 1289.
NMR δ ppm ($D_2O$): 1.24 (3H, d, J = 7.3 Hz), 1.31 (3H, d, J = 5.9 Hz), 1.96 (1H, m), 2.89 (1H, m), 3.49 (2H, m), 3.75 (1H, m), 3.98 (2H, m), 4.30 (3H, m), 6.72 (1H, d, J = 15.5 Hz), 6.96 (1H, d, J = 8.3 Hz), 7.14 (1H, d, J = 8.3 Hz), 7.24 (1H, s), 7.50 (1H, d, J = 15.5 Hz).

Example 12-1

IR$_{max}$ cm$^{-1}$ (neat): 3300 (br), 1768 (sh), 1760 1747 (sh),
NMR δ ppm ($CDCl_3$): 1.29 (3H, d, J = 7.3 Hz), 1.38 (3H, d, J = 6.3 Hz), 2.49 (1H, m), 3.15 - 3.85 (6H, m), 3.90 - 4.45 (5H, m), 4.61 (2H, d, J = 5.3 Hz), 5.2 - 5.6 (4H, m), 5.94 (1H, m), 7.66 (2H, d, J = 8.8 Hz), 8.23 (2H, d, J = 8.8 Hz).

Example 12-2

IR$_{max}$ cm$^{-1}$ (neat): 3380 (br), 1766, 1700, 1642, 1590, 1512, 1344, 1302.
NMR δ ppm ($CDCl_3$): 0.20 (6H, s), 0.22 (6H, s), 0.98 (9H, s), 0.99 (9H, s), 1.27 (3H, d, J = 6.3 Hz), 1.37 (3H, d, J = 6.3 Hz), 3.2 - 3.9 (6H, m), 4.0 - 4.5 (5H, m), 5.2 - 5.6 (2H, m), 6.42 (1H, d, J = 15 Hz), 6.84 (1H, d, J = 8.3 Hz), 6.97 (1H, s), 7.09 (1H, d, J = 7.9 Hz), 7.66 (2H, d, J = 8.9 Hz), 8.23 (2H, d, J = 8.9 Hz).

Example 12-3

IR$_{max}$ cm$^{-1}$ (neat): 3375 (br), 1760, 1750 (sh), 1638, 1518, 1342, 1283.
NMR δ ppm ($CDCl_3$-$CD_3OD$ (20:1)): 1.27 (3H, d, J = 6.3 Hz), 3.2 - 3.9 (6H, m), 4.1 - 4.5 (5H, m), 5.2 - 5.6 (2H, m), 6.45 (1H, d, J = 15 Hz), 6.83 (1H, d, J = 7.9 Hz), 6.9 - 7.15 (2H, m), 7.56 (1H, d, J = 15 Hz), 7.66 (2H, d, J = 8.9 Hz), 8.23 (2H, d, J = 8.9 Hz).

Example 12-4

UV$_{max}$ nm ($H_2O$): 276, 305.
IR$_{max}$ cm$^{-1}$ (KBr): 3510 (br), 1685, 1654, 1560, 1508, 1458, 1290.
NMR δ ppm ($D_2O$): 1.22 (3H, d, J = 7.3 Hz), 1.29 (3H, d, J = 6.3 Hz), 1.95 (1H, m), 2.58 (1H, m), 6.67 (1H, d, J = 15.5 Hz), 6.93 (1H, d, J = 8.3 Hz), 7.11 (1H, d, J = 8.3 Hz), 7.19 (1H, br. s), 7.42 (1H, d, J = 15.5 Hz).

Example 13-1

IR$_{max}$ cm$^{-1}$ (neat): 3430, 1768, 1700, 1520, 1443, 1412, 1347, 1210, 1138.
NMR δ ppm ($CDCl_3$): 1.23 - 1.38 (6H, m), 1.4 - 1.8 (7H, m), 1.88 (1H, m), 2.68 (1H, m), 3.2 - 3.7 (7H, m), 4.0 - 4.3 (3H, m), 4.57 (2H, m), 4.75 (1H, m), 5.15 - 5.52 (4H, m), 5.89 (1H, m), 7.66 (2H, d, J = 8.9 Hz), 8.22 (2H, m).

Example 13-2

IR$_{max}$ cm$^{-1}$ (neat): 3400 (br), 1768, 1707, 1643, 1590, 1512, 1440, 1402, 1345, 1301, 1290 (sh), 1251, 1207, 1129.
NMR δ ppm ($CDCl_3$): 0.19 (6H, s), 0.21 (6H, s), 0.98 (18H, s), 1.30 (3H, d, J = 7.3 Hz), 1.37 (3H, d, J = 6.3 Hz), 1.99 (1H, m), 2.64 (1H, m), 3.29 (1H, dd, J = 2.6 & 6.6 Hz), 3.3 - 3.8 (6H, m), 4.15 (1H, m), 4.21 - 4.32 (2H, m), 5.04 (1H, t, J = 8.3 Hz), 5.26 (1H, d, J = 13.9 Hz), 5.50 (1H, d, J = 13.9 Hz), 6.47 (1H, d, J = 15.3 Hz), 6.81 (1H, d, J = 8.3 Hz), 6.95 (1H, d, J = 2.1 Hz), 7.05 (1H, dd, J = 2.1 & 8.3 Hz), 7.56 (1H, d, J = 15.3 Hz), 7.66 (2H, d, J = 8.9 Hz), 8.24 (2H, d, J = 8.9 Hz).

Example 13-3

IR$_{max}$ cm$^{-1}$ (neat): 3300 (br), 1766, 1702, 1641, 1600, 1519, 1441, 1343, 1283, 1205, 1135.
NMR δ ppm ($CDCl_3$): 1.23 (3H, d, J = 6.9 Hz), 1.34 (3H, d, J = 5.9 Hz), 2.52 (1H, m), 3.23 (1H, m), 3.3 - 3.7 (6H, m), 3.75 (1H, m), 4.18 (2H, m), 4.77 (1H, m), 5.16 (1H, d, J = 13.7 Hz), 5.44 (1H, d, J = 13.7 Hz), 6.12 (1H, m), 6.55 - 6.70 (2H, m), 6.84 (1H, m), 7.18 (1H, d, J = 14.9 Hz), 7.60 (2H, d, J = 8.7 Hz), 8.17 (2H, d, J = 8.7 Hz).

Example 13-4

UV$_{max}$ nm ($H_2O$): 245, 300.
IR$_{max}$ cm$^{-1}$ (KBr): 3400 (br), 1755, 1596, 1444, 1394, 1287.
NMR δ ppm ($D_2O$): 1.23 (3H, d, J = 6.6 Hz), 1.30 (3H, d, J = 6.3 Hz), 1.50 - 1.90 (7H, m), 2.85 (1H, m), 3.45 (2H, m), 3.60 (4H, m), 3.85 (1H, m), 3.97 (2H, m), 4.37 (3H, m), 6.74 (1H, d, J = 15.5 Hz), 6.95 (1H, d, J = 8.3 Hz), 7.15 (1H, dd, J = 1.3 & 8.3 Hz), 7.24 (1H, d, J = 1.3 Hz), 7.45 (1H, d, J = 15.5 Hz).

Example 14-1

IR$_{max}$ cm$^{-1}$ (neat): 3400 (br), 3260, 1767, 1700, 1640, 1602, 1511, 1343, 1318, 1276.
NMR δ ppm ($CDCl_3$): 0.15 (3H, s), 0.16 (3H, s), 0.23 (6H, s), 0.97 (9H, s), 0.99 (9H, s), 1.25 (3H, d, J = 7.3 Hz), 1.36 (3H, d, J = 6.3 Hz), 1.87 (1H, m), 2.76 (1H, m), 2.98 (3H, s), 3.02 (3H, s), 3.10 (1H, m), 3.20 (1H, d, J = 16.5 Hz), 3.25 (2H, m), 3.41 (1H, d, J = 16.5 Hz), 3.76 (2H, m), 4.23 (2H, m), 5.10 (1H, d, J = 13.9 Hz), 5.43 (1H, d, J = 13.9 Hz), 6.72 (1H, d, J = 8.6 Hz), 6.90 (1H, dd, J = 2.3 & 8.6 Hz), 7.55 (1H, d, J = 2.3 Hz), 7.64 (1H, d, J = 8.9 Hz), 8.21 (1H, d, J = 8.9 Hz).

Example 14-2

IR$_{max}$ cm$^{-1}$ (KBr): 3240 (br), 1771, 1665, 1520, 1346, 1210,
NMR δ ppm ($CDCl_3$-$CD_3OD$ (5:1)): 1.25 (3H, d, J = 6.9 Hz), 1.33 (3H, d, J = 6.3 Hz), 1.83 (1H, m), 2.87 (1H, m), 3.00 (3H, s), 3.06 (3H, s), 4.22 (2H, m), 5.14 (1H, d, J = 13.5 Hz), 5.40 (1H, d, J = 13.5 Hz), 6.74 (1H, d, J = 8.2 Hz), 6.97 (1H, dd, J = 2.3 & 8.2 Hz), 7.11 (1H, d, J = 2.3 Hz), 7.64 (2H, d, J = 8.9 Hz), 8.21 (2H, d, J = 8.9 Hz).

Example 14-3

UV$_{max}$ nm ($H_2O$): 254, 300.
IR$_{max}$ cm$^{-1}$ (KBr): 3270 (br), 1754, 1630, 1530, 1392, 1260.
NMR δ ppm ($D_2O$): 1.19 (3H, d, J = 7.3 Hz), 1.27 (3H, d, J = 6.3 Hz), 1.71 (1H, m), 2.95 (1H, m), 2.96 (3H, s), 3.09 (3H, s), 3.12 (1H, d, J = 16.2 Hz), 3.18 (1H, m), 3.39 (2H, m), 3.42 (1H, d, J = 16.2 Hz), 3.92 (2H, m), 4.09 (1H, dd, J = 1.3 & 10.9 Hz), 4.20 (1H, quint, J = 6.8 Hz), 6.91 (2H, s), 6.95 (1H, s).

Example 15-1

IR$_{max}$ cm$^{-1}$ (neat): 3425 (br), 1770, 1714, 1648, 1580, 1445, 1430, 1374, 1327.
NMR δ ppm ($CDCl_3$): 1.28 (3H, d, J = 7.3 Hz), 1.36 (3H, d, J = 6.3 Hz), 2.5 - 3.3 (9H, m), 3.3 - 4.00 (3H, m), 4.23 (1H, m), 4.6 - 5.2 (7H, m), 5.2 - 5.6 (6H, m), 5.85 - 6.25 (3H, m), 7.5 - 8.2 (2H, m).

Example 15-2

UV$_{max}$ nm ($H_2O$): 290.
IR$_{max}$ cm$^{-1}$ (KBr): 3410, 1754, 1594, 1398, 1290, 1150.
NMR δ ppm ($D_2O$): 1.25 (6H, m), 1.88 (1H, m), 2.66 (3H x 0.5, s), 2.90 (1H, m), 2.96 (3H x 0.5, s),

3.00 (3H x 0.5, s), 3.22 (3H x 0.5, s), 3.30 - 3.90 (4H, m), 4.10 - 4.35 (3H, m), 6.73 (0.5H, s), 6.89 (0.5H, s), 7.72 (0.5H, s), 7.73 (0.5H, m).

Example 16-1

| | |
|---|---|
| $IR_{max}$ $cm^{-1}$ (neat): | 3400 (br), 1770, 1703, 1636, 1518, 1419, 1405, 1344, 1318, 1252. |
| NMR δ ppm ($CDCl_3$): | 0.19 (6H, s), 0.21 (6H, s), 0.95 (9H, s), 1.03 (9H, s), 1.27 (3H, d, J = 7.3 Hz), 1.35 (3H, d, J = 5.9 Hz), 2.04 (1H, m), 2.70 (1H, m), 2.99 (3H, s), 3.20 (3H, s), 3.26 (1H, m), 3.56 (1H, m), 3.76 (1H, m), 3.90 (1H, m), 4.15 - 4.26 (2H, m), 5.12 (1H, m), 5.24 (1H, d, J = 13.8 Hz), 5.50 (1H, d, J = 13.8 Hz), 7.03 (1H, s), 7.65 (2H, d, J = 8.9 Hz), 8.23 (2H, d, J = 8.3 Hz). |

Example 16-2

| | |
|---|---|
| $IR_{max}$ $cm^{-1}$ (KBr): | 3347 (br), 1768, 1636, 1522, 1424, 1348, 1210, 1139. |
| NMR δ ppm ($CDCl_3$-$CD_3OD$ (9:1)): | 1.23 - 1.30 (6H, m), 1.94 (1H, m), 2.73 (1H, m), 3.00 (3H, s), 3.22 (3H, s), 3.91 (1H, m), 4.1 - 4.25 (2H, m), 5.07 (1H, m), 5.23 - 5.50 (2H, m), 6.94 (1H, m), 7.12 (1H, m), 7.66 (2H, d, J = 8.6 Hz), 8.23 (2H, d, J = 8.6 Hz). |

Example 16-3

| | |
|---|---|
| $UV_{max}$ nm ($H_2O$): | 260, 291. |
| $IR_{max}$ $cm^{-1}$ (KBr): | 3400 (br), 1751, 1598, 1398. |
| NMR δ ppm ($D_2O$): | 1.18 (3H x 0.7, d, J = 6.9 Hz), 1.23 (3H x 0.3, d, J = 7.3 Hz), 1.26 (3H x 0.7, d, J = 6.3 Hz), 1.31 (3H x 0.3, d, J = 6.3 Hz), 1.87 (1H, m), 2.65 (3H x 0.3, s), 2.85 (3H x 0.3, s), 2.99 (3H x 0.7, s), 3.22 (3H x 0.7, s), 2.86 (1H, m), 3.42 (3H, m), 3.76 (1H, m), 4.10 - 4.32 (3H, m), 6.73 (0.3H, s), 6.91 (0.3H, s), 6.99 (0,7H, s), 7.21 (0.7H, s). |

Example 17-1

| | |
|---|---|
| $IR_{max}$ $cm^{-1}$ (neat): | 3370 (br), 1763, 1695, 1635, 1505, 1320. |
| NMR δ ppm ($CDCl_3$): | 1.22 (3H, d, J = 7.3 Hz), 1.35 (3H, d, J = 6.3 Hz), 1.8 - 2.0 (1H, m), 2.55 - 2.75 (1H, m), 2.80 (1H, m), 2.90 (3H, s), 3.06 (3H, s), 3.1 - 3.4 (3H, m), 3.4 - 4.0 (4H, m), 4.15 (1H, dd, J = 2.5 & 9.1 Hz), 4.22 (1H, m), 4.59 (4H, d, J = 5.6 Hz), 4.6 - 4.9 (2H, m), 5.2 - 5.5 (6H, m), 5.9 - 6.2 (3H, m),6.7 - 7.0 (3H, m). |

Example 17-2

| | |
|---|---|
| $UV_{max}$ nm ($H_2O$): | 245 (sh), 290, 302 (sh). |
| $IR_{max}$ $cm^{-1}$ (KBr): | 3370 (br), 1756, 1601, 1387, 1287. |
| NMR δ ppm ($D_2O$): | 1.19 (3H, d, J = 6.6 Hz), 1.30 (3H, d, J = 6.3 Hz), 1.69 (1H, m), 2.77 (3H, s), 2.80 (1H, m), 2.93 (3H, s), 3.38 (3H, m), 3.55 - 4.40 (7H, m), 6.90 (3H, m). |

Example 18-1

| | |
|---|---|
| $IR_{max}$ $cm^{-1}$ (neat): | 3320 (br), 1761, 1698, 1634, 1510, 1445, 1370, 1323, 1276, 1206, 1180, 1138. |
| NMR δ ppm ($CDCl_3$): | 1.17 (3H, d, J = 7.3 Hz), 1.39 (3H, d, J = 6.3 Hz), 2.90 (3H,s), 3.00 (3H, s), 3.4 - 3.6 (2H, m), 4.14 (1H, dd, J = 2.3 & 8.9 Hz), 4.27 (1H, m), 4.75 (2H, m), 5.25 (1H, m), 5.45 (1H, m), 5.96 (1H, m), 6.53 (1H, dd, J = 2.0 & 8.0 Hz), 6.78 (1H, d, J = 8.0 Hz), 6.81 (1H, d, J = 2.0 Hz). |

Example 18-2

| | |
|---|---|
| $UV_{max}$ nm ($H_2O$): | 290. |
| $IR_{max}$ $cm^{-1}$ (KBr): | 3306 (br), 1758, 1654, 1594, 1388, 1286. |
| NMR δ ppm ($D_2O$): | 1.19 (3H, d, J = 6.9 Hz), 1.31 (3H, d, J = 6.6 Hz), 1.86 (1H, m), 2.86 (3H, s), |

2.93 (3H, s), 3.00 (3H, m), 3.15 - 3.66 (5H, m), 4.03 (1H, m), 4.18 (1H, br. d, J = 9.6 Hz), 4.27 (1H, m), 6.72 (1H, dd, J = 7.9 & 2.0 Hz), 6.82 (1H, d, J = 2.0 Hz), 6.89 (1H, d, J = 7.9 Hz).

Example 19-1

$IR_{max}$ $cm^{-1}$ (neat): 3280 (br), 1762, 1699, 1633, 1510, 1441, 1363, 1320, 1279, 1204, 1134.
NMR δ ppm ($CDCl_3$): 1.21 (3H, d, J = 7.3 Hz), 1.38 (3H, d, J = 6.3 Hz), 2.95 (3H, s), 3.13 (3H, s), 3.32 (1H, m), 3.48 (1H, m), 3.60 (1H, m), 4.18 - 4.30 (2H, m), 4.72 (2H, m), 5.22 (1H, m), 5.42 (1H, m), 5.94 (1H, m), 6.48 (1H, dd, J = 7.9 & 2.0 Hz), 6.77 (1H, d, J = 2.0 Hz), 6.77 (1H, d, J = 7.9 Hz).

Example 19-2

$UV_{max}$ nm ($H_2O$): 287.
$IR_{max}$ $cm^{-1}$ (KBr): 3388 (br), 1756, 1654, 1598, 1388, 1286.
NMR δ ppm ($D_2O$): 1.20 (3H, d, J = 7.3 Hz), 1.30 (3H, d, J = 6.3 Hz), 1.90 (3H, m), 2.57 (2H, m), 2.97 (3H, s), 3.04 (3H, s), 3.08 (1H, m), 3.33 (1H, dd, J = 8.9 & 7.3 Hz), 3.46 (1H, dd, J = 5.9 & 3.3 Hz), 3.60 (1H, m), 4.03 (1H, m), 4.27 (3H, m), 4.39 (1H, m), 6.71 (1H, dd, J = 8.2 & 2.3 Hz), 6.79 (1H, d, J = 2.3 Hz), 6.87 (1H, d, J = 8.2 Hz).

Example 20-1

$IR_{max}$ $cm^{-1}$ (neat): 3350 (br), 1770, 1707, 1620, 1440, 1320,
NMR δ ppm ($CDCl_3$): 1.25 (3H, d, J = 7.3 Hz), 1.34 (3H, d, J = 5.9 Hz), 1.82 (1H, m), 2.54 (1H, m), 3.1 - 3.85 (6H, m), 4.0 - 4.95 (12H, m), 5.1 - 5.6 (8H, m), 5.7 - 6.2 (4H, m), 7.50 (1H, s), 8.11 (1H, s).

Example 20-2

$UV_{max}$ nm ($H_2O$): 292.
$IR_{max}$ $cm^{-1}$ (KBr): 1350, 1752, 1560, 1508, 1438, 1395,
NMR δ ppm ($D_2O$): 1.19 (3H, m), 1.26 (3H, d, J = 5.6 Hz), 1.87 (1H, m), 2.75 (1H, m), 3.15 - 3.56 (5H, m), 3.65 (1H, m), 3.78 (1H, m), 4.12 (2H, m), 4.26 (1H, m), 6.85 (1H, s), 7.75 (1H, s).

Example 21-1

$IR_{max}$ $cm^{-1}$ (neat): 3440 (br), 3330 (br), 1765, 1670, 1442, 1370, 1280.
NMR δ ppm ($CDCl_3$): 1.18 (3H, d, J = 7.6 Hz), 1.32 (3H, d, J = 6.3 Hz), 1.85 (1H, m), 2.65 - 4.3 (11H, m), 4.6 - 4.9 (2H, m), 5.2 - 5.55 (2H, m), 5.9 - 6.1 (1H, m), 6.55 - 6.9 (3H, m).

Example 21-2

$UV_{max}$ nm ($H_2O$): 288, 302 (sh).
$IR_{max}$ $cm^{-1}$ (KBr): 3421, 1752, 1670, 1592, 1393, 1290.
NMR δ ppm ($D_2O$): 1.15 (3H, d, J = 7.3 Hz), 1.29 (3H, d, J = 6.6 Hz), 1.74 (1H, m), 2.83 (2H, m), 3.02 (1H, br. d, J = 11.2 Hz), 3.29 (2H, m), 3.40 (1H, dd, J = 6.3 & 2.6 Hz), 3.57 (1H, d, J = 12.9 Hz), 3.71 (1H, d, J = 12.9 Hz), 3.74 (1H, m), 4.10 (1H, dd, J = 8.9 & 2.6 Hz), 4.23 (1H, m), 6.80 (1H, dd, J = 8.3 & 2.0 Hz), 6.87 (1H, d, J = 8.3 Hz), 6.89 (1H, d, J = 2.0 Hz).

Example 22-1

$IR_{max}$ $cm^{-1}$ (neat): 3391, 1771,1705, 1645, 1683, 1419, 1324, 1276, 1208, 1135, 1029.
NMR δ ppm ($CDCl_3$): 1.23 (3H, d, J = 7.3 Hz), 1.35 (3H, d, J = 6.3 Hz), 1.93 (1H, m), 2.66 (1H, m),

2.84 (1H, m), 2.91 (3H, s), 3.02 (3H, s), 3.14 (1H, dd, J = 9.9 & 4.9 Hz), 3.21 (1H, dd, J = 7.3 & 2.3 Hz), 3.35 (1H, m), 3.44 (1H, d, J = 13.2 Hz), 3.61 (1H, m), 3.70 (1H, m), 3.83 (1H, d, J = 13.2 Hz), 4.20 (2H, m), 4.55 (4H, m), 4.68 (1H, dd, J = 13.5 & 5.6 Hz), 6.69 (1H, dd, J = 13.5 & 5.3 Hz), 5.37 (6H, m), 6.07 (3H, m), 6.88 (1H, d, J = 2.0 Hz), 7.04 (1H, d, J = 2.0 Hz).

Example 22-2

$UV_{max}$ nm ($H_2O$): 290, 300 (sh).
$IR_{max}$ $cm^{-1}$ (KBr): 3380, 1752, 1594, 1394, 1290.
NMR δ ppm ($D_2O$): 1.19 (3H, d, J = 7.3 Hz), 1.31 (3H, d, J = 6.3 Hz), 1.70 (1H, m), 2.76 (3H, s), 2.85 (1H, m), 2.96 (3H, s), 3.40 (3H, m), 3.70 - 4.30 (7H, m), 6.80 (1H, s), 7.03 (1H, s).

Example 23-1

$IR_{max}$ $cm^{-1}$ (neat): 3445, 1771, 1683, 1488, 1419, 1372, 1279, 1209, 1142, 1035.
NMR δ ppm ($CDCl_3$): 1.23 (3H, d, J = 7.3 Hz), 1.34 (3H, d, J = 6.3 Hz), 1.97 (1H, m), 2.77 (2H, m), 3.04 (1H, br. d, J = 11.7 Hz), 3.24 (3H, m), 3.40 (1H, d, J = 13.9 Hz), 3.70 (1H, m), 3.90 (1H, d, J = 13.9 Hz), 4.20 (2H, m), 4.55 (4H, m), 4.67 (1H, dd, J = 13.6 & 5.6 Hz), 4.82 (1H, dd, J = 13.6 & 5.6 Hz), 5.35 (6H, m), 6.04 (3H, m), 6.71 (1H, d, J = 2.0 Hz), 6.89 (1H, d, J = 2.0 Hz).

Example 23-2

$UV_{max}$ nm ($H_2O$): 290, 300.
$IR_{max}$ $cm^{-1}$ (KBr): 3328, 1757, 1673, 1583, 1436, 1388,
NMR δ ppm ($D_2O$): 1.17 (3H, d, J = 7.6 Hz), 1.30 (3H, d, J = 6.3 Hz), 1.98 (1H, m), 2.99 (1H, m), 3.28 (2H, m), 3.45 (1H, dd, J = 6.3 & 2.6 Hz), 3.55 (2H, m), 3.95 (1H, m), 4.20 (4H, m), 6.93 (1H, d, J = 1.7 Hz), 7.05 (1H, d, J = 1.7 Hz).

Example 24-1

$IR_{max}$ $cm^{-1}$ (neat): 3430 (br), 1765, 1700 (sh), 1675 1582, 1510, 1367, 1320, 1202, 1134.
NMR δ ppm ($CDCl_3$): 1.22 (3H, d, J = 7.3 Hz), 1.35 (3H, d, J = 6.3 Hz), 1.97 (1H, m), 2.80 (1H, m), 2.9 - 3.15 (2H, m), 3.22 (1H, dd, J = 2.6 & 6.9 Hz), 3.25 - 3.45 (2H, m), 3.55 - 4.05 (3H, m), 4.16 (1H, dd, J = 2.6 & 9.2 Hz), 4.23 (1H, m), 4.55 - 4.9 (6H, m), 5.2 - 5.6 (7H, m), 5.85 - 6.2 (3H, m), 6.78 (1H, s), 7.56 (1H, br. s), 8.08 (1H, s).

Example 24-2

$UV_{max}$ nm ($H_2O$): 278, 303 (sh).
$IR_{max}$ $cm^{-1}$ (KBr): 3421, 1751, 1676, 1594, 1559, 1394, 1217, 1180.
NMR δ ppm ($D_2O$): 1.18 (3H, d, J = 7.3 Hz), 1.28 (3H, d, J = 6.3 Hz), 1.83 (1H, m), 3.00 (3H, m), 3.35 (2H, m), 3.42 (1H, m), 3.63 (1H, d, J = 14.2 Hz), 3.88 (2H, m), 4.09 (1H, d, J = 9.2 Hz), 4.22 (1H, m), 6.54 (1H, s), 7.64 (1H, s).

Example 25-1

$UV_{max}$ nm ($H_2O$): 290, 300.
$IR_{max}$ $cm^{-1}$ (KBr): 3419, 1752, 1654, 1388, 1290.
NMR δ ppm ($D_2O$): 1.20 (3H, d, J = 6.9 Hz), 1.30 (3H, d, J = 6.3 Hz), 1.90 (3H, m), 2.54 (2H, m), 2.98 (3H, s), 3.04 (3H, s), 3.16 (1H, m), 3.34 (1H, m), 3.47 (1H, dd, J = 6.3 & 2.6 Hz), 3.53 (1H, m), 3.66 (1H, br. d, J = 12.5 Hz), 3.97 (1H, m), 4.07 (1H, m), 4.26 (2H, m), 6.72 (1H, d, J = 2.0 Hz), 6.82 (1H, d, J = 2.0 Hz).

Example 26-1

$IR_{max}$ $cm^{-1}$ (neat): 3410 (br), 1768, 1642, 1408, 1280, 1206,
NMR δ ppm ($CDCl_3$): 1.28 (3H, d, J = 6.9 Hz), 1.36 (3H, d, J = 6.3 Hz), 1.95 (1H, m), 2.69 (1H, m), 2.8 - 3.5 (4H, m), 2.93 (3H s), 3.07 (3H, s), 3.6 - 4.0 (3H, m), 4.0 - 4.4 (2H, m), 4.5 - 4.9 (6H, m), 5.2 - 5.6 (6H, m), 5.9 - 6.3 (3H, m), 7.31 (1H, s).

Example 26-2

$UV_{max}$ nm ($H_2O$): 270 (sh), 296.
$IR_{max}$ $cm^{-1}$ (KBr): 3435, 1753, 1636, 1396, 1281.
NMR δ ppm ($D_2O$): 1.19 (3H, d, J = 7.3 Hz), 1.32 (3H, d, J = 6.3 Hz), 1.72 (1H, m), 2.81 (3H, s), 2.82 (1H, m), 2.97 (3H, s), 3.40 (4H, m), 3.94 (2H, m), 4.26 (4H, m), 6.82 (1H, s).

Example 27-1

$IR_{max}$ $cm^{-1}$ (KBr): 3391 (br), 1771, 1646, 1541, 1325, 1211, 1137.
NMR δ ppm ($CDCl_3$-$CD_3OD$ (9:1)): 1.15 - 1.34 (10H, m), 1.84 (1H, m), 2.72 (1H, m), 2.99, 3.06 and 3.09 (6H in total, each s), 2.8 - 4.2 (8H, m), 4.54 - 4.82 (2H, m), 5.20 - 5.46 (2H, m), 5.93 (1H, m), 6.77 (1H, m), 6.99 - 7.15 (2H, m).

Example 27-2

$UV_{max}$ nm ($H_2O$): 253, 296.
$IR_{max}$ $cm^{-1}$ (KBr): 3418, 1751, 1636, 1523, 1394, 1261.
NMR δ ppm ($D_2O$): 1.24 (9H, m), 1.71 (1H, m), 2.93 (2H, m), 2.95 (3H x 0.5, s), 2.98 (3H x 0.5, s), 3.11 (3H x 0.5, s), 3.14 (3H x 0.5, s), 3.24 (1H, m), 3.36 (2H, m), 3.58 (1H, q, J = 7.3 Hz), 3.84 (1H, m), 4.04 (2H, m), 4.20 (1H, m), 6.91 (2H, s), 6.92 (0.5H, s), 7.04 (0.5H, s).

Example 28-1

$IR_{max}$ $cm^{-1}$ (neat): 3401, 1770, 1704, 1651, 1504, 1440, 1208.
NMR δ ppm ($CDCl_3$): 1.22 (3H, d, J = 7.3 Hz), 1.35 (3H, d, J = 6.3 Hz), 1.91 (1H, m), 2.66 (1H, m), 2.81 (1H, m), 2.91 (3H, s), 2.92 (1H, m), 3.04 (3H, s), 3.15 (1H, m), 3.20 (1H, m), 3.33 (1H, m), 3.41 (1H, d, J = 13.5 Hz), 3.52 (1H, m), 3.72 (1H, m), 3.85 (1H, d, J = 13.5 Hz), 4.20 (1H, m), 4.59 (4H, m), 4.69 (1H, dd, J = 16.0 & 5.5 Hz), 4.82 (1H, dd, J = 16.0 & 6.5 Hz), 5.35 (6H, m), 6.04 (3H, m), 6.66 (1H, d, J = 14.2 Hz), 6.70 (1H, br. s).

Example 28-2

$UV_{max}$ nm ($H_2O$): 296.
$IR_{max}$ $cm^{-1}$ (KBr): 3401, 1754, 1597, 1391.
NMR δ ppm ($D_2O$): 1.19 (3H, d, J = 7.3 Hz), 1.31 (3H, d, J = 6.3 Hz), 1.70 (1H, m), 2.78 (3H, s), 2.86 (1H, m), 2.96 (3H, s), 3.40 (4H, m), 3.77 (1H, d, J = 12.2 Hz), 3.91 (1H, m), 3.98 (1H, d, J = 12.2 Hz), 4.13 (1H, m), 4.24 (3H, m), 6.72 (1H, s), 6.74 (1H, d, J = 12.8 Hz).

Example 29-1

$IR_{max}$ $cm^{-1}$ (neat): 3390, 1762, 1638, 1483, 1277, 1187, 1133.
NMR δ ppm ($CDCl_3$): 1.25 (3H, d, J = 7.3 Hz), 1.35 (3H, d, J = 6.3 Hz), 1.95 (1H, m), 2.65 (1H, m), 2.83 (1H, m), 2.88 (3H, s), 3.11 (3H, s), 3.20 (2H, m), 3.31 (1H, m), 3.66 (1H, m), 3.74 (1H, d, J = 13.2 Hz), 3.85 (1H, d, J = 12.2 Hz), 3.87 (1H, m), 4.56 (4H, m), 4.69 (1H, dd, J = 16.0 & 5.5 Hz), 4.80 (1H, dd, J = 16.0 & 6.5 Hz), 5.37 (4H, m), 6.07 (2H, m), 6.79 (1H, d, J = 8.6 Hz), 7.10 (1H, d, J = 8.6 Hz).

Example 29-2

| | |
|---|---|
| $UV_{max}$ nm ($H_2O$): | 298, 305 (sh). |
| $IR_{max}$ $cm^{-1}$ (KBr): | 3356, 2968, 1754, 1604, 1390, 1288. |
| NMR δ ppm ($D_2O$): | 1.19 (3H, d, J = 7.3 Hz), 1.31 (3H, d, J = 6.5 Hz), 1.76 (1H, m), 2.81 (3H, s), 2.82 (2H, m), 2.97 (3H, s), 3.40 (3H, m), 3.8 - 4.3 (6H, m), 6.81 (2H, s). |

Table 3

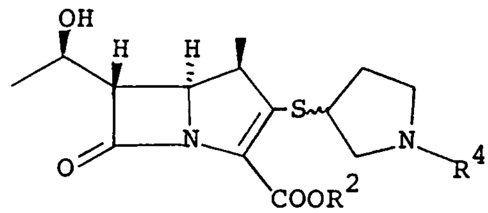

| Example No. | $R^2$ | $R^4$ |
|---|---|---|
| 30-1 | PNB | AOC |
| 30-2 | PNB | 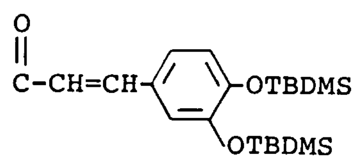 |
| 30-3 | PNB | 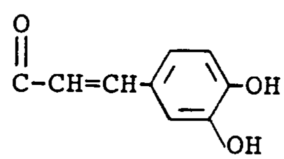 |
| 30-4 | H | 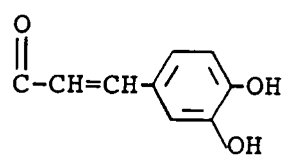 |
| 31-1 | PNB | 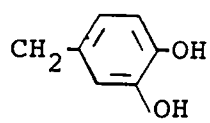 |
| 31-2 | H | 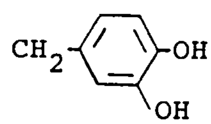 |
| 32-1 | PNB | 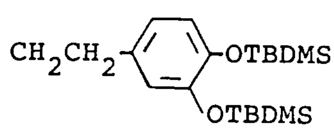 |
| 32-2 | PNB | 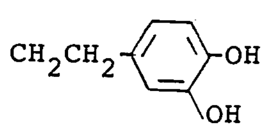 |

| | | |
|---|---|---|
| 32-3 | H | 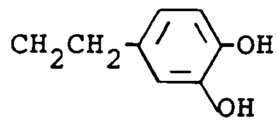 |
| 33-1 | PNB | 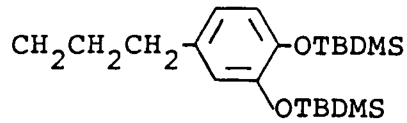 |
| 33-2 | PNB | 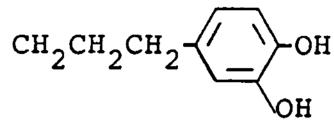 |
| 33-3 | H | 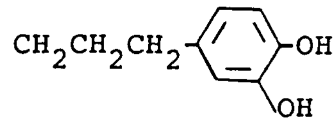 |
| 34-1 | $CH_2CH{=}CH_2$ | 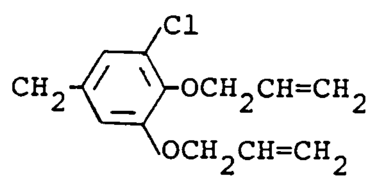 |
| 34-2 | H | 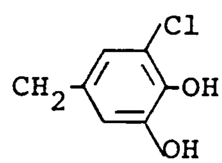 |
| 35-1 | $CH_2CH{=}CH_2$ | 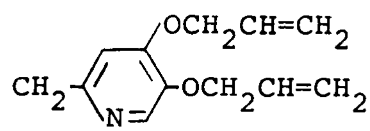 |
| 35-2 | H | 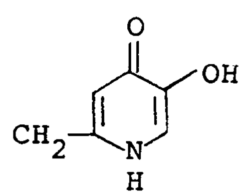 |
| 36-1 | $CH_2CH{=}CH_2$ | 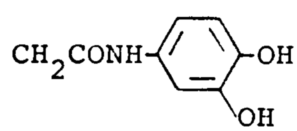 |

| | | |
|---|---|---|
| 36-2 | H | 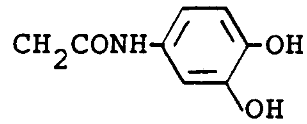 |
| 37-1 | $CH_2CH=CH_2$ | 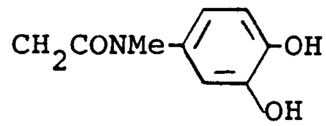 |
| 37-2 | H | 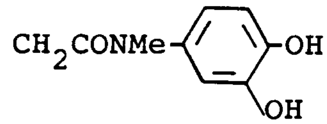 |

Physical property

Example 30-1

$IR_{max}$ $cm^{-1}$ (neat): 3450 (br), 1765, 1696, 1682 (sh), 1518, 1410, 1342, 1205, 1133.
NMR δ ppm ($CDCl_3$): 1.27 (3H, d, J = 7.3 Hz), 1.38 (3H, d, J = 6.3 Hz), 1.98 (1H, m), 2.30 (1H, m), 3.25 - 3.75 (5H, m), 3.80 (2H, m), 4.27 (2H, m), 4.60 (2H, m), 5.15 - 5.65 (4H, m), 5.93 (1H, m), 7.66 (2H, d, J = 8.8 Hz), 8.23 (2H, d, J = 8.8 Hz).

Example 30-2

$IR_{max}$ $cm^{-1}$ (neat): 3400 (br), 1764, 1700, 1640, 1595, 1510, 1434, 1342, 1302, 1250, 1208, 1130.
NMR δ ppm ($CDCl_3$): 0.21 (6H, s), 0.22 (6H, s), 0.99 (9H, s), 1.00 (9H, s), 1.25 (3H, d, J = 6.3 Hz), 1.38 (3H, d, J = 6.3 Hz), 3.25 - 3.50 (2H, m), 3.5 - 4.0 (4H, m), 4.01 (1H, m), 4.28 (2H, m), 5.2 - 5.6 (2H, m), 6.48 (1H, d, J = 15 Hz), 6.82 (1H, d, J = 8.3 Hz), 6.9 - 7.15 (2H, m), 7.58 (1H, d, J = 15 Hz), 7.66 (2H, d, J = 8.9 Hz), 8.22 (2H, d, J = 8.9 Hz).

Example 30-3

$IR_{max}$ $cm^{-1}$ (neat): 3400 (br), 1760, 1700, 1640, 1600 (br), 1518, 1440, 1342.
NMR δ ppm ($CDCl_3$-$CD_3OD$ (9:1)): 1.28 (3H, d, J = 7.3 Hz), 1.35 (3H, d, J = 6.3 Hz), 3.0 - 3.3 (2H, m), 3.5 - 4.0 (2H, m), 4.25 (1H, m), 5.2 - 5.6 (2H, m), 6.49 (1H, d, J = 16 Hz), 6.82 (1H, d, J = 8.3 Hz), 6.9 - 7.1 (2H, m), 7.54 (1H, d, J = 16 Hz), 7.65 (1H, d, J = 8.9 Hz), 7.66 (1H, d, J = 8.6 Hz), 8.22 (2H, d, J = 8.9 Hz).

Example 30-4

$UV_{max}$ nm ($H_2O$): 218, 243 (sh), 306, 320 (sh).
$IR_{max}$ $cm^{-1}$ (KBr): 3348 (br), 1752, 1643, 1592, 1438, 1394, 1290.
NMR δ ppm ($D_2O$): 1.15 - 1.38 (6H, m), 6.59 (0.4H, d, J = 15.3 Hz), 6.68 (0.6H, d, J = 15.3 Hz), 6.93 (1H, d, J = 8.3 Hz), 7.11 (1H, br. d, J = 8.3 Hz), 7.19 (1H, br. s), 7.38 (0.4H, d, J = 15.3 Hz), 7.40 (0.6H, d, J = 15.3 Hz).

Example 31-1

$IR_{max}$ $cm^{-1}$ (neat): 3350 (br), 1755, 1700, 1518, 1375, 1346, 1280, 1207, 1138.
NMR δ ppm ($CDCl_3$-$CD_3OD$ (9:1): 1.24 (3H, d, J = 7.3 Hz), 1.34 (3H, d, J = 5.9 Hz), 4.0 - 4.3 (2H, m), 5.2 - 5.6 (2H, m), 6.55 - 6.95 (3H, m), 7.66 (2H, d, J = 8.6 Hz),

8.23 (2H, d, J = 8.6 Hz).

Example 31-2

$UV_{max}$ nm ($H_2O$): 286, 304 (sh).
$IR_{max}$ $cm^{-1}$ (KBr): 3380 (br), 1752, 1594, 1390, 1290.
NMR δ ppm ($D_2O$): 1.18 (3H, d, J = 7.3 Hz), 1.29 (3H, d, J = 6.6 Hz), 2.02 (2H, m), 2.54 (1H, m), 3.05 - 3.76 (5H, m), 3.98 (1H, m), 4.18 (3H, m), 6.93 (3H, m)

Example 32-1

$IR_{max}$ $cm^{-1}$ (neat): 3400 (br), 1760, 1700, 1507, 1342, 1287, 1248, 1126.
NMR δ ppm ($CDCl_3$): 0.18 (6H, s), 0.18 (6H, s), 0.98 (18H, m), 1.23 - 1.40 (6H, m), 2.34 (1H, m), 2.49 - 3.0 (6H, m), 3.2 - 3.4 (2H, m), 3.74 (1H, m), 4.25 (2H, m), 5.24 (1H, d, J = 13.7 Hz), 5.51 (1H d, J = 13.7 Hz), 6.59 - 6.75 (3H, m), 7.66 (2H, d, J = 8.9 Hz), 8.22 (2H, m).

Example 32-2

$IR_{max}$ $cm^{-1}$ (KBr): 3385 (br), 1772, 1608, 1522, 1347, 1209, 1138.
NMR δ ppm ($CDCl_3$-$CD_3OD$ (10:1)): 1.36 (3H, m), 1.62 (1H, m), 2.37 (1H, m), 3.76 (1H, m), 4.19 (2H, m), 5.37 (2H, m), 6.55 (1H, m), 6.74 (2H, m), 7.66 (2H, d, J = 8.7 Hz), 8.22 (2H, d, J = 8.7 Hz).

Example 32-3

$UV_{max}$ nm ($H_2O$): 289, 300 (sh).
$IR_{max}$ $cm^{-1}$ (KBr): 3350 (br), 1755, 1592, 1390, 1287.
NMR δ ppm ($D_2O$): 1.19 (3H, d, J = 6.6 Hz), 1.30 (3H, d, J = 6.3 Hz), 2.00 (2H, m), 2.52 (1H, m), 2.96 (2H, m), 3.20 - 3.70 (6H, m), 4.00 (1H, m), 4.28 (3H, m).

Example 33-1

$IR_{max}$ $cm^{-1}$ (neat): 3450 (br), 1768, 1702, 1513, 1346, 1294, 1253, 1211, 1132.
NMR δ ppm ($CDCl_3$): 0.18 (12H, s), 0.98 (9H, s), 0.98 (9H, s), 1.27 (3H, d, J = 7.3 Hz), 1.37 (3H, m), 1.74 (3H ,m), 2.2 - 2.6 (6H, m), 2.77 (1H, m), 3.06 - 3.41 (3H, m), 3.71 (1H, m), 4.24 (2H, m), 5.23 (2H, d, J = 13.9 Hz), 5.48 (2H, m), 6.58 - 6.63 (2H, m), 6.71 (1H, d, J = 7.9 Hz), 7.66 (2H, d, J = 8.4 Hz), 8.22 (2H, d, J = 8.4 Hz).

Example 33-2

$IR_{max}$ $cm^{-1}$ (neat): 3300 (br), 1752, 1701, 1602, 1520, 1346, 1280, 1208, 1139.
NMR δ ppm ($CDCl_3$-$CD_3OD$ (10:1)): 1.21 - 1.36 (6H, m), 1.7 - 2.0 (3H, m), 2.3 - 3.0 (7H, m), 4.20 (2H, m), 5.23 - 5.51 (2H, m), 6.67 - 6.95 (3H, m), 7.66 (2H, d, J = 7.9 Hz), 8.22 (2H, m).

Example 33-3

$UV_{max}$ nm ($H_2O$): 287.
$IR_{max}$ $cm^{-1}$ (KBr): 3390 (br), 1756, 1592, 1390, 1286.
NMR δ ppm ($D_2O$): 1.20 (3H, d, J = 6. 0 Hz), 1.29 (3H, d, J = 6.6 Hz), 2.02 (4H, m), 2.59 (3H, m), 3.06 - 3.70 (7H, m), 4.01 (1H, m), 4.24 (2H, m), 6.60 - 6.96 (3H, m).

Example 34-1

$IR_{max}$ $cm^{-1}$ (neat): 3380, 1765, 1701, 1571, 1544, 1485, 1419, 1368, 1320, 1274, 1205, 1137.
NMR δ ppm ($CDCl_3$): 1.25 (3H, d, J = 7.3 Hz), 1.35 (3H x 0.5, d, J = 6.3 Hz), 1.36 (3H x 0.5, d, J = 6.3 Hz), 1.82 (1H, m), 2.25 - 2.86 (5H, m), 2.95 - 3.40 (3H, m), 3.52 (2H, br. s),

3.71 (1H, m), 4.23 (1H, m), 4.58 (4H, m), 4.65 (1H, dd, J = 13.5 & 5.6 Hz), 4.82 (1H, dd, J = 13.5 & 4.6 Hz), 5.35 (6H, m), 6.07 (3H, m), 6.80 (1H, br. s), 6.92 (1H, br. s).

Example 34-2

| | |
|---|---|
| $UV_{max}$ nm ($H_2O$): | 289, 300 (sh). |
| $IR_{max}$ $cm^{-1}$ (KBr): | 3396, 1752, 1594, 1392, 1293. |
| NMR δ ppm ($D_2O$)): | 1.18 (3H, d, J = 7.3 Hz), 1.29 (3H, d, J = 6.3 Hz), 2.03 (1H, m), 2.56 (1H, m), 3.10 - 4.30 (9H, m), 6.95 (2H, m). |

Example 35-1

| | |
|---|---|
| $IR_{max}$ $cm^{-1}$ (neat): | 3350 (br), 1764, 1698, 1582, 1505, 1365, 1318, 1272, 1238, 1200, 1134. |
| NMR δ ppm ($CDCl_3$): | 1.25 (3H x 0.5, d, J = 6.9 Hz), 1.25 (3H x 0.5, d, J = 7.3 Hz), 1.35 (3H x 0.5, d, J = 6.3 Hz), 1.37 (3H x 0.5, d, J = 6.3 Hz), 2.25 - 2.85 (4H, m), 2.9 - 3.4 (3H, m), 3.55 - 3.85 (3H, m), 4.05 - 4.35 (2H, m), 4.5 - 4.9 (6H, m), 5.15 - 5.55 (6H, m), 5.85 - 6.2 (3H, m), 6.93 (1H x 0.5, s) 6.94 (1H x 0.5, s), 8.07 (1H x 0.5, s), 8.08 (1H x 0.5, s). |

Example 35-2

| | |
|---|---|
| $UV_{max}$ nm ($H_2O$): | 280, 303 (sh). |
| $IR_{max}$ $cm^{-1}$ (KBr): | 3254, 1751, 1595, 1558, 1391, 1262. |
| NMR δ ppm ($D_2O$)): | 1.19 (3H, d, J = 7.3 Hz), 1.29 (3H, d, J = 6.3 Hz), 1.83 (1H, m), 2.30 - 3.50 (7H, m), 3.75 (3H, m), 4.26 (2H, m), 6.57 (0.4H, s), 6.59 (0.6H, s), 7.61 (1H, br. s). |

Example 36-1

| | |
|---|---|
| $IR_{max}$ $cm^{-1}$ (KBr): | 3293 (br), 1768, 1662, 1541, 1280, 1210, 1138. |
| NMR δ ppm ($CDCl_3$): | 1.26 - 1.39 (6H, m), 2.40 (1H, m), 2.6 - 3.5 (7H, m), 3.77 (1H, m), 4.14 - 4.25 (2H, m), 4.6 - 4.9 (2H, m), 5.23 - 5.51 (2H, m), 5.97 (1H, m), 6.72 - 6.96 (2H, m), 7.62 and 7.73 (1H in total, each d, J = 2.0 & 2.3 Hz). |

Example 36-2

| | |
|---|---|
| $UV_{max}$ nm ($H_2O$): | 252, 293. |
| $IR_{max}$ $cm^{-1}$ (KBr): | 3438, 1751, 1577, 1395, 1255. |
| NMR δ ppm ($D_2O$)): | 1.21 (3H, m), 1.30 (3H, m), 1.95 (2H, m), 2.51 (1H, m), 2.96 - 3.66 (5H, m), 3.80 (2H, m), 3.95 (1H, m), 4.26 (2H, m), 6.89 (2H, m), 7.03 (1H, m). |

Example 37-1

| | |
|---|---|
| $IR_{max}$ $cm^{-1}$ (neat): | 3380 (br), 1750, 1639, 1500, 1360, 1290, 1260, 1200, 1124. |
| NMR δ ppm ($CDCl_3$): | 1.25 (3H, m), 1.37 (3H, d, J = 6.3 Hz), 3.20 and 3.22 (3H in total, each s), 3.0 - 3.4 (4H, m), 3.6 - 3.8 (2H, m), 4.23 (2H, m), 4.74 (2H, m), 5.23 - 5.50 (2H, m), 5.99 (1H, m), 6.55 - 6.90 (3H, m). |

Example 37-2

| | |
|---|---|
| $UV_{max}$ nm ($H_2O$): | 286, 305 (sh). |
| $IR_{max}$ $cm^{-1}$ (KBr): | 3430, 1754, 1660, 1604, 1391, 1260. |
| NMR δ ppm ($D_2O$): | 1.20 (3H, d, J = 7.3 Hz), 1.30 (3H, d, J = 6.3 Hz), 3.22 (3H, s), 6.86 (3H, m). |

Table 4

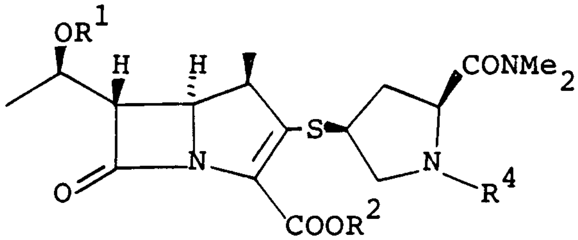

| Example No. | $R^2$ | $R^4$ | $R^1$ |
|---|---|---|---|
| 38-1 | $CH_2CH=CH_2$ | AOC | 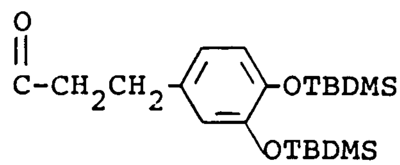 |
| 38-2 | $CH_2CH=CH_2$ | AOC | 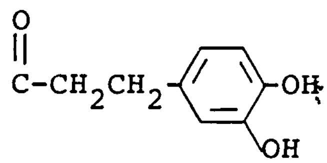 |
| 38-3 | H | H | 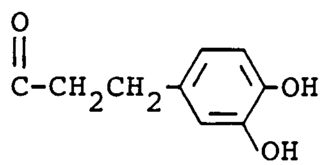 |
| 39-1 | $CH_2CH=CH_2$ | AOC | 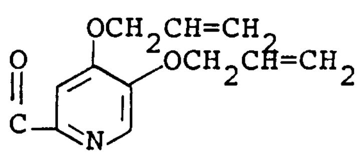 |
| 39-2 | H | H | 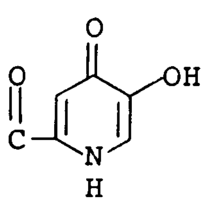 |

| | | | |
|---|---|---|---|
| 40-1 | $CH_2CH=CH_2$ | AOC | 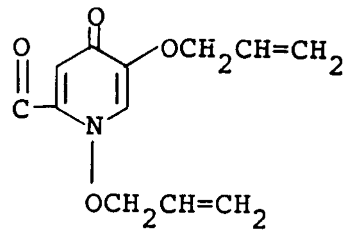 |
| 40-2 | H | H | 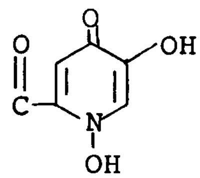 |
| 41-1 | $CH_2CH=CH_2$ | AOC | 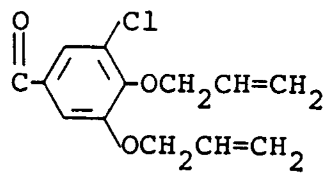 |
| 41-2 | H | H | 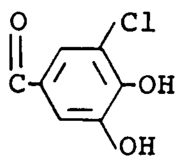 |
| 42-1 | $CH_2CH=CH_2$ | AOC | 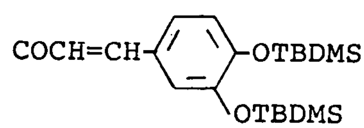 |
| 42-2 | $CH_2CH=CH_2$ | AOC | 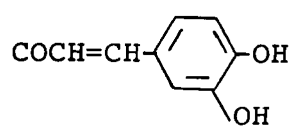 |
| 42-3 | H | H | 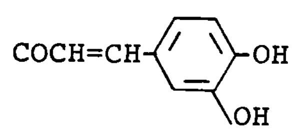 |
| 43-1 | $CH_2CH=CH_2$ | AOC | 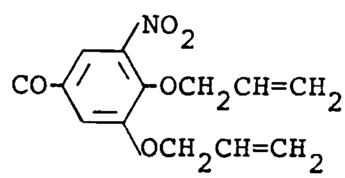 |

| | | | |
|---|---|---|---|
| 43-2 | H | H | 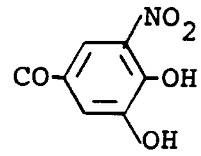 |
| 44-1 | $CH_2CH=CH_2$ | AOC | 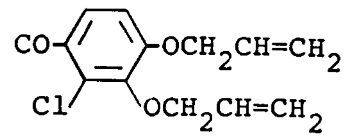 |
| 44-2 | H | H | 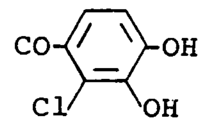 |
| 45-1 | $CH_2CH=CH_2$ | AOC | 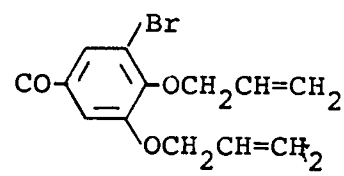 |
| 45-2 | H | H | 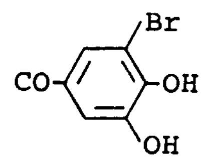 |
| 46-1 | $CH_2CH=CH_2$ | AOC | 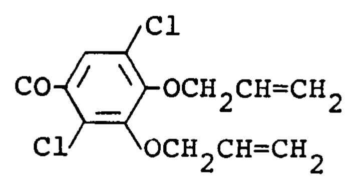 |
| 46-2 | H | H | 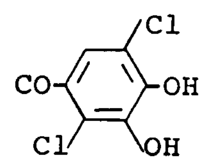 |
| 47-1 | $CH_2CH=CH_2$ | AOC | 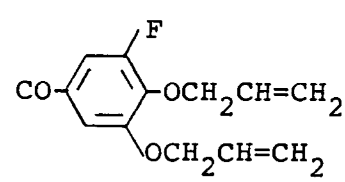 |

| | | | |
|---|---|---|---|
| 47-2 | H | H | 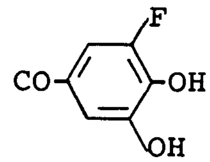 |

Physical property

Example 38-1

$IR_{max}$ $cm^{-1}$ (neat): 1780, 1707, 1654, 1510, 1407, 1287, 1255, 1211, 1140.

NMR δ ppm ($CDCl_3$): 0.18 (6H, s), 0.18 (6H, s), 0.97 (9H, s), 0.98 (9H, s), 1.21 - 1.26 (3H, m), 1.34 - 1.38 (3H, m), 1.96 (1H, m), 2.54 - 2.71 (3H, m), 2.82 (2H, m), 2.96 - 3.11 (6H, m), 3.25 - 4.2 (5H, m), 4.5 - 4.9 (5H, m), 5.16 - 5.47 (5H, m), 5.75 - 6.05 (2H, m), 6.60 - 6.75 (3H, m).

Example 38-2

$IR_{max}$ $cm^{-1}$ (neat): 3350 (br), 1776, 1698, 1639, 1510, 1440, 1411, 1338, 1320, 1280, 1207, 1179, 1141.

NMR δ ppm ($CDCl_3$): 1.08 (3H, m), 1.35 (3H, m), 1.90 (1H, m), 2.55 - 2.75 (3H, m), 2.64 (2H, m), 3.00 - 3.14 (6H, m), 3.2 - 4.2 (5H, m), 3.20 - 4.20 (5H, m), 4.52 - 4.83 (5H, m), 5.14 - 5.46 (5H, m), 5.78 - 6.03 (2H, m), 6.56 (1H, m), 6.72 - 6.78 (2H, m).

Example 38-3

$UV_{max}$ nm ($H_2O$): 290, 304 (sh).

$IR_{max}$ $cm^{-1}$ (KBr): 3400 (br), 1762, 1654, 1597, 1386, 1256.

NMR δ ppm ($D_2O$): 1.08 (3H, d, J = 7.3 Hz), 1.27 (3H, d, J = 6.3 Hz), 1.93 (1H, m), 2.77 (2H, m), 2.84 (2H, m), 3.01 (3H, s), 3.09 (3H, s), 3.10 (3H, m), 3.40 (1H, dd, J = 11.9 & 5.0 Hz), 3.48 (1H, dd, J = 6.3 & 2.6 Hz), 3.71 (1H, dd, J = 11.9 & 6.3 Hz), 3.85 (1H, dd, J = 9.5 & 2.6 Hz), 5.19 (1H, quint, J = 6.0 Hz), 6.72 (1H, dd, J = 7.9 & 2.0 Hz), 6.83 (1H, d, J = 2.0 Hz), 6.86 (1H, d, J = 7.9 Hz).

Example 39-1

$IR_{max}$ $cm^{-1}$ (neat): 1778, 1711, 1652, 1580, 1512, 1408, 1322, 1279, 1209.

NMR δ ppm ($CDCl_3$): 1.27 (3H, m), 1.59 (3H, d, J = 6.3 Hz), 1.94 (1H, m), 2.64 (1H, m), 2.9 - 3.2 (6H, m), 3.2 - 3.7 (4H, m), 3.95 - 4.2 (1H, m), 4.2 - 4.4 (1H, m), 4.4 - 4.95 (10H, m), 5.1 - 5.7 (9H, m), 5.7 - 6.2 (4H, m), 7.69 (1H, d, J = 7.6 Hz), 8.25 (1H, s).

Example 39-2

$UV_{max}$ nm ($H_2O$): 297.

$IR_{max}$ $cm^{-1}$ (KBr): 3000 (br), 1764, 1654, 1600, 1386, 1257.

NMR δ ppm ($D_2O$): 1.25 (3H, d, J = 6.9 Hz), 1.50 (3H, d, J = 6.3 Hz), 1.96 (1H, m), 3.00 (3H, s), 3.07 (3H, s), 3.13 (1H, m), 3.43 (2H, m), 3.74 (1H, dd, J = 11.2 & 6.3 Hz), 4.05 (1H, m), 4.40 (1H, br. d, J = 6.9 Hz), 5.58 (1H, quint., J = 6.0 Hz), 7.29 (1H, s), 7.76 (1H, s).

Example 40-1

$IR_{max}$ $cm^{-1}$ (neat): 1778, 1718 (sh), 1708, 1660, 1610, 1588, 1415, 1282, 1218, 1144.

NMR δ ppm ($CDCl_3$): 1.29 (3H, d, J = 6.9 Hz), 1.52 (3H, d, J = 6.3 Hz), 1.85 - 2.10 (1H, m), 2.5 - 2.8 (1H, m), 2.9 - 3.2 (6H, m), 3.3 - 3.8 (4H, m), 4.0 - 4.3 (2H, m), 4.5 - 5.0 (10H, m), 5.1 - 5.65 (9H, m), 5.75 - 6.15 (4H, m), 6.74 (1H, s), 7.29 (1H, m).

Example 40-2

UV$_{max}$ nm ($H_2O$): 297.
IR$_{max}$ $cm^{-1}$ (KBr): 3420 (br), 1763, 1657, 1383, 1264, 1156.
NMR δ ppm ($D_2O$): 1.25 (3H, d, J = 7.3 Hz), 1.49 (3H, d, J = 6.3 Hz), 2.12 (1H, m), 3.00 (3H, s), 3.07 (3H, s), 3.15 (1H, m), 3.77 (4H, m), 3.98 (1H, m), 4.06 (1H, m), 4.40 (1H, m), 5.56 (1H, m), 7.19 (1H, s), 7.73 (1H, s).

Example 41-1

IR$_{max}$ $cm^{-1}$ (neat): 1775, 1701, 1651, 1406, 1276.
NMR δ ppm ($CDCl_3$): 1.26 (3H, d, J = 6.3 Hz), 1.52 (3H, d, J = 6.6 Hz), 1.96 (1H, m), 2.67 (1H, m), 2.96 (3H x 0.3, s), 2.98 (3H x 0.7, s), 3.06 (3H x 0.3, s), 3.10 (3H x 0.7, s), 3.30 - 3.70 (4H, m), 4.05 (1H, m), 4.27 (1H, m), 4.50 - 4.86 (7H, m), 5.15 - 5.55 (7H, m), 5.76 - 6.18 (3H, m), 7.49 (1H, d, J = 2.0 Hz, 7.66 (1H, d, J = 2.0 Hz).

Example 41-2

UV$_{max}$ nm ($H_2O$): 242 (sh), 308.
IR$_{max}$ $cm^{-1}$ (KBr): 3400 (br), 1754, 1600, 1492, 1396, 1260.
NMR δ ppm ($D_2O$): 1.25 (3H, d, J = 6.6 Hz), 1.46 (3H, d, J = 6.0 Hz), 1.64 (1H, m), 2.82 (1H, m), 2.97 (3H, s), 3.07 (3H, s), 3.13 (1H, m), 3.41 (1H, m), 3.79 (3H, m), 3.97 (1H, m), 4.16 (1H, m), 4.36 (1H, br. d, J = 8.6 Hz), 5.44 (1H, quint., J = 6.0 Hz), 7.34 (1H, d, J = 2.0 Hz), 7.66 (1H, d, J = 2.0 Hz).

Example 42-1

IR$_{max}$ $cm^{-1}$ (neat): 1779, 1709, 1659, 1512, 1412, 1287, 1260, 1154, 1130;
NMR δ ppm ($CDCl_3$) : 0.21 (6H, s), 0.22 (6H, s), 0.98 (9H, s), 1.00 (9H, s), 1.27 (3H, m), 1.48 (3H, m), 1.94 (1H, m), 2.64 (1H, m), 2.96 and 2.98 (3H in total, each s), 3.05 and 3.10 (3H in total, each s), 3.3 - 4.3 (5H, m), 4.51 - 4.86 (5H, m), 5.15 - 5.47 (5H, m), 5.78 - 6.04 (2H, m), 6.20 (1H, d, J = 16.0 Hz), 6.82 (1H, m), 7.00 - 7.07 (2H, m), 7.58 (1H, d, J = 16.0 Hz).

Example 42-2

IR$_{max}$ $cm^{-1}$ (neat): 3320 (br), 1769, 1699, 1633, 1600, 1513, 1440, 1407, 1271, 1148.
NMR δ ppm ($CDCl_3$): 1.26 (3H, m), 1,45 (3H, m), 1.94 (1H, m), 2.69 (1H, m), 2.98 and 2.99 (3H in total, each s), 3.08 and 3.10 (3H in total, each s), 3.3 - 4.3 (5H, m), 4.50 - 4.84 (5H, m), 5.15 - 5.50 (5H, m), 5.75 - 6.02 (2H, m), 6.21 (1H, d, J = 15.8 Hz), 6.81 - 7.08 (3H, m), 7.56 (1H, m).

Example 42-3

UV$_{max}$ nm ($H_2O$): 302, 320 (sh),
IR$_{max}$ $cm^{-1}$ (KBr): 3436, 1760, 1694, 1607, 1403, 1259, 1175.
NMR δ ppm ($D_2O$): 1.25 (3H, d, J = 7.0 Hz), 1.42 (3H, d, J = 6.0 Hz), 1.62 (1H, m), 2.98 (3H, s), 3.08 (3H, s), 5.42 (1H, m), 6.35 (1H, d, J = 16.5 Hz), 7.86 (1H, d, J = 6.6 Hz), 7.08 (1H, d, J = 6.6 Hz), 7.16 (1H, br. s), 7.64 (1H, d, J = 16.5 Hz).

Example 43-1

IR$_{max}$ $cm^{-1}$ (neat): 1762, 1720, 1694, 1653, 1533, 1407, 1280, 1190, 1139.
NMR δ ppm ($CDCl_3$): 1.24 (3H, d, J = 7.3 Hz), 1.53 (3H, d, J = 6.3 Hz), 1.96 (1H, m), 2.67 (1H, m), 2.96 (3H x 0.4, s), 2.98 (3H x 0.6, s), 3.06 (3H x 0.4, s), 3.11 (0.6H, s), 3.50 (4H, m), 4.32 (2H, m), 4.73 (9H, m), 5,36 (9H, m), 6.03 (4H, m), 7.75 (0.6H, d, J = 2.0 Hz), 7.78 (0.4H, d, J = 2.0 Hz), 7.94 (1H, d, J = 2.0 Hz).

Example 43-2

$UV_{max}$ nm ($H_2O$): 292.
$IR_{max}$ $cm^{-1}$ (KBr): 3435, 1753, 1702, 1616, 1560, 1388, 1280,
NMR δ ppm ($D_2O$)): 1.25 (3H, m), 1.47 (3H, d, J = 6.3 Hz), 1.67 (1H, m), 2.82 (1H, m), 2.96 (1H, m), 2.98 (3H, s), 3.06 (3H, s), 3.10 - 3.52 (3H, m), 3.82 (3H, m), 4.30 (1H, m), 5.47 (1H, m), 7.38 (1H, br. s), 8.26 (1H, s).

Example 44-1

$IR_{max}$ $cm^{-1}$ (neat): 1775, 1705, 1653, 1405, 1277.
NMR δ ppm ($CDCl_3$): 1.28 (3H, d, J = 7.3 Hz), 1.54 (3H, d, J = 6.3 Hz), 1.97 (1H, m), 2.66 (1H, m), 2.97 (3H x 0.3, s), 2.98 (3H x 0.7, s), 3.06 (3H x 0.3, s), 3.11 (3H x 0.7, s), 3.47 (4H, m), 4.00 - 4.40 (2H, m), 4,70 (9H, m), 5.37 (9H, m), 6.04 (4H, m), 6.84 (1H, d, J = 9.9 Hz), 7.62 (0.7H, d, J = 9.9 Hz), 7.65 (0.3H, d, J = 9.9 Hz).

Example 44-2

$UV_{max}$ nm ($H_2O$): 268, 291.
$IR_{max}$ $cm^{-1}$ (KBr): 3432, 1765, 1655, 1603, 1388, 1294.
NMR δ ppm ($D_2O$): 1.21 (3H, br. s), 1.46 (3H, d, J = 6.0 Hz), 1.92 (1H, m), 3.00 (3H, s), 3.02 (1H, m), 3.06 (3H, s), 3.39 (2H, m), 3.66 (1H, m), 3.77 (1H, m), 3.98 (1H, m), 4.37 (1H, m), 5.49 (1H, m), 6.82 (1H, m), 7.40 (1H, m).

Example 45-1

NMR δ ppm ($CDCl_3$): 1.31 (3H, d, J = 7.3 Hz), 1.53 (3H, d, J = 6.3 Hz), 1.94 (1H, m), 2.66 (1H, m), 2.97 (3H x 0.3, s), 2.99 (3H x 0.7, s), 3.06 (3H x 0.3, s), 3.11 (3H x 0.7, s), 3.50 (4H, m), 4.00 - 4.35 (2H, m), 4.65 (9H, m), 5.38 (9H, m), 6.02 (4H, m), 7.53 (1H, br. s), 7.81 (1H, s).

Example 45-2

$UV_{max}$ nm ($H_2O$): 270, 297.
$IR_{max}$ $cm^{-1}$ (KBr): 3436, 1764, 1705, 1655, 1604, 1388, 1298, 1224.
NMR δ ppm ($D_2O$): 1.14 (3H, d, J = 7.3 Hz), 1.23 (3H, d, J = 6.3 Hz), 1.76 (1H, m), 2.97 (3H, s), 2.98 (2H, m), 3.05 (3H, s), 3.15 - 4.00 (6H, m), 4.50 (1H, m), 5.43 (1H, m), 7.36 (1H, s), 7.8 (1H, s).

Example 46-1

$IR_{max}$ $cm^{-1}$ (neat): 1780, 1715, 1652, 1404, 1285, 1176, 1140.
NMR δ ppm ($CDCl_3$): 1.27 (3H x 0.5, d, J = 6.9 Hz), 1.29 (3H x 0.5, d, J = 7.3 Hz), 1.55 (3H x 0.5, d, J = 6.3 Hz), 1.57 (3H x 0.5, d, J = 6.6 Hz), 1.95 (1H, m), 2.66 (1H, m), 2.98 (3H, s), 3.11 (3H, s), 3.25 - 4.4 (8H, m), 4.5 - 5.0 (8H, m), 5.1 - 5.7 (8H, m), 5.7 - 6.3 (4H, m), 7.65 (1H, m).

Example 46-2

$UV_{max}$ nm ($H_2O$): 304.
$IR_{max}$ $cm^{-1}$ (KBr): 3430, 1758, 1704, 1612, 1493, 1393, 1300, 1178.
NMR δ ppm ($D_2O$): 1.23 (3H, d, J = 7.3 Hz), 1.46 (3H, d, J = 6.3 Hz), 1.83 (1H, m), 2.90 (1H, m), 2.98 (3H, s), 3.07 (3H, s), 3.26 (1H, m), 3.38 (2H, m), 3.75 (1H, m), 3.86 (1H, m), 3.96 (1H, m), 4.36 (1H, m), 5.43 (1H, m), 7.61 (1H, s).

Example 47-1

NMR δ ppm ($CDCl_3$): 1.28 (3H, d, J = 7.3 Hz), 1.52 (3H, d, J = 6.6 Hz), 1.94 (1H, m), 2.64 (1H, m),

2.97 (3H x 0.4, s), 2.98 (3H x 0.6, s), 3.06 (3H x 0.4, s), 3.10 (3H x 0.6, s), 3.51 (4H, m), 4.05 (1H, m), 4.28 (1H, m), 4.66 (9H, m), 5.33 (9H, m), 6.00 (4H, m), 7.40 (2H, m).

Example 47-2

| | |
|---|---|
| $UV_{max}$ nm ($H_2O$): | 266, 294. |
| $IR_{max}$ $cm^{-1}$ (KBr): | 3357, 1764, 1702, 1655, 1366, 1224. |
| NMR δ ppm ($D_2O$): | 1.25 (3H, d, J = 6.9 Hz), 1.46 (3H, d, J = 6.3 Hz), 2.01 (1H, m), 3.01 (3H, s), 3.03 (2H, m), 3.08 (3H, s), 3.42 (2H, m), 3.76 (1H, m), 3.82 (1H, m), 4.06 (1H, m), 4.43 (1H, m), 5.47 (1H, m), 7.38 (1H, s), 7.41 (1H, d, J = 12.5 Hz). |

Table 5-(1)

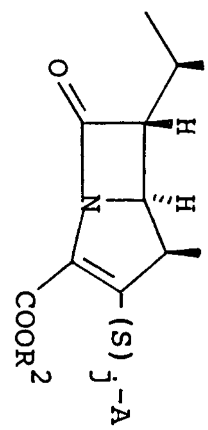

| Example No. | $R^1$ | $R^2$ | j | A |
|---|---|---|---|---|
| 48-1 | 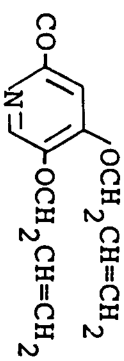 | $CH_2CH=CH_2$ | 1 | 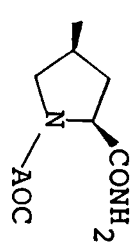 |
| 48-2 | 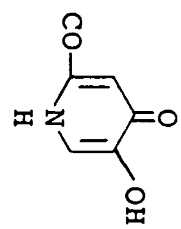 | H | 1 | 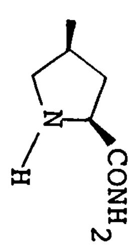 |
| 49-1 | 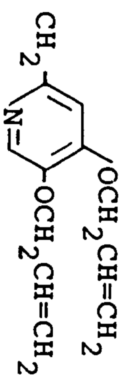 | $CH_2CH=CH_2$ | 1 | 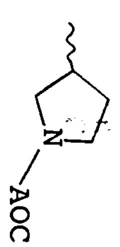 |

| | | | | |
|---|---|---|---|---|
| 49-2 | 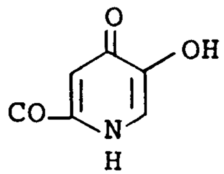 | H | 1 | 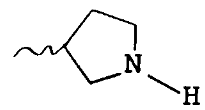 |
| 50-1 | 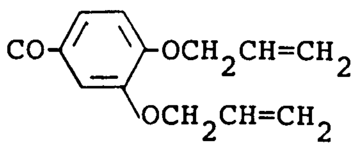 | $CH_2CH=CH_2$ | 1 | $-(CH_2)_2NHCH=N-AOC$ |
| 50-2 | 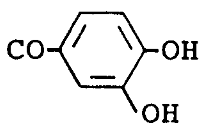 | H | 1 | $-(CH_2)_2NHCH=NH$ |
| 51-1 | TMS | $CH_2CH=CH_2$ | 1 | 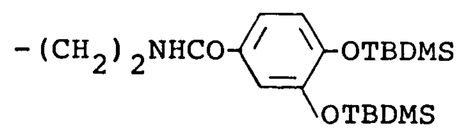 |
| 51-2 | H | $CH_2CH=CH_2$ | 1 | 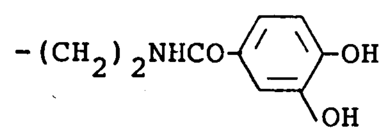 |

| | | | | |
|---|---|---|---|---|
| 51-3 | H | H | 1 | 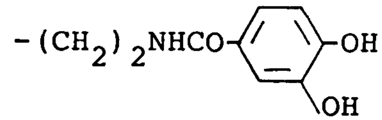 |
| 52-1 | 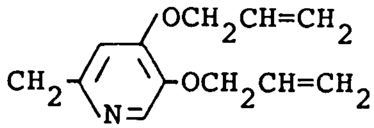 | $CH_2CH=CH_2$ | 1 | Et |
| 52-2 | 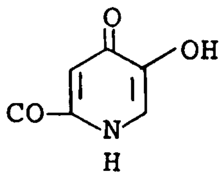 | H | 1 | Et |
| 53-1 | 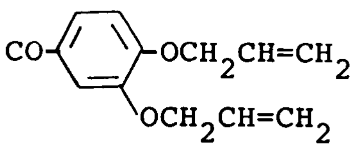 | $CH_2CH=CH_2$ | 1 | Et |
| 53-2 | 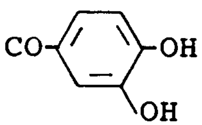 | H | 1 | Et |
| 54-1 | 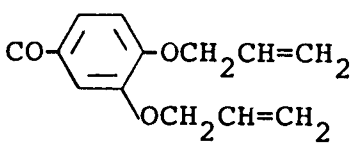 | $CH_2CH=CH_2$ | 1 | $-(CH_2)_2OCONHMe$ |

| | | | | |
|---|---|---|---|---|
| 54-2 | 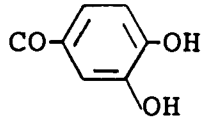 | H | 1 | $-(CH_2)_2OCONHMe$ |
| 55-1 | 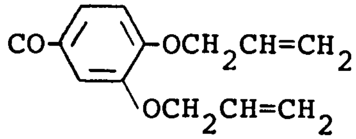 | $CH_2CH=CH_2$ | 1 | 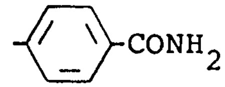 |
| 55-2 | 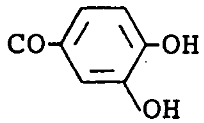 | H | 1 | 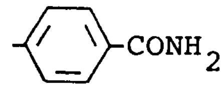 |
| 56-1 | 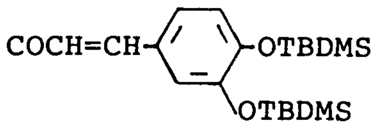 | PNB | 1 | 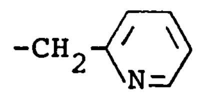 |
| 56-2 | 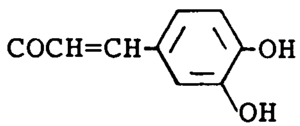 | PNB | 1 | 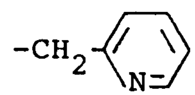 |
| 56-3 | 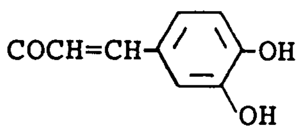 | H | 1 | 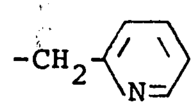 |

Table 5-(2)

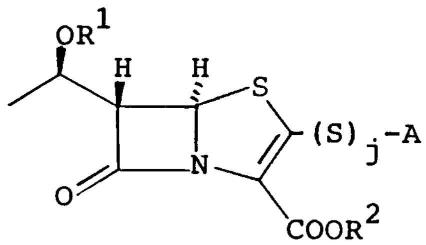

| Example No. | $R^1$ | $R^2$ | j | A |
|---|---|---|---|---|
| 57-1 | 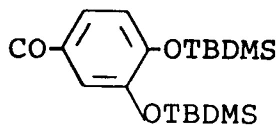 | PNB | 1 | Et |
| 57-2 | 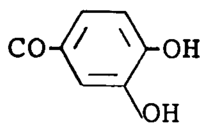 | PNB | 1 | Et |
| 57-3 | 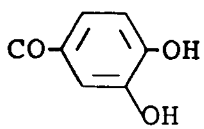 | H | 1 | Et |
| 58-1 | 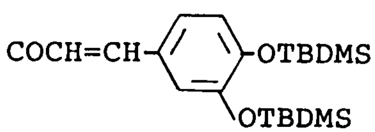 | PNB | 1 | 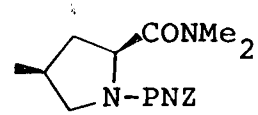 |

| | | | | |
|---|---|---|---|---|
| 58-2 | COCH=CH- 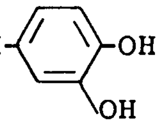 | PNB | 1 | 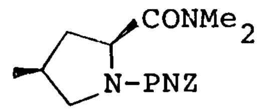 |
| 58-3 | COCH=CH- 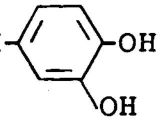 | H | 1 | 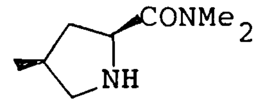 |
| 59-1 | COCH=CH- 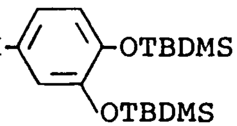 | PNB | 0 | 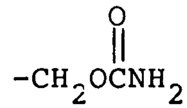 |
| 59-2 | COCH=CH- 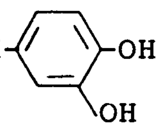 | PNB | 0 | 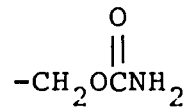 |
| 59-3 | COCH=CH- 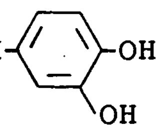 | H | 0 | 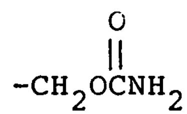 |
| 60-1 | COCH=CH- 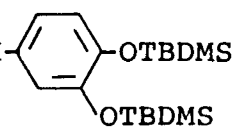 | PNB | 0 | 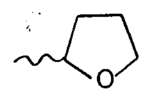 |

| | | | | |
|---|---|---|---|---|
| 60-2 | 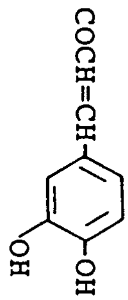 | PNB | 0 | 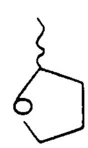 |
| 60-3 | 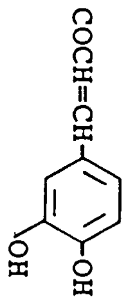 | H | 0 | 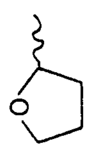 |

Physical property

Example 48-1

IR$_{max}$ cm$^{-1}$ (neat): 1775, 1700, 1405, 1320, 1278, 1204.

NMR $\delta$ ppm ($CDCl_3$): 1.27 (3H, d, J = 7.6 Hz), 1.59 (3H, d, J = 6.3 Hz), 3.2 - 4.5 (8H, m), 4.5 - 5.0 (8H, m), 5.2 - 5.7 (8H, m), 5.8 - 6.2 (4H, m), 7.68 (1H, s), 8.26 (1H, s).

Example 48-2

UV$_{max}$ nm ($H_2O$): 296.

$IR_{max}$ $cm^{-1}$ (KBr): 3348, 1755, 1682, 1599, 1387, 1265.
NMR δ ppm ($D_2O$): 1.24 (3H, d, J = 7.3 Hz), 1.50 (3H, d, J = 6.3 Hz), 1.95 (1H, m), 2.86 (2H, m), 3.14 - 4.52 (7H, m), 5.57 (1H, m), 7.27 (1H, s), 7.73 (1H, s).

Example 49-1

$IR_{max}$ $cm^{-1}$ (neat): 1775, 1700, 1575, 1408, 1320, 1275, 1205, 1134, 1110.
NMR δ ppm ($CDCl_3$): 1.28 (3H, d, J = 7.3 Hz), 1.59 (3H, d, J = 6.9 Hz), 1.95 (1H, m), 2.27 (1H, m), 3.2 - 3.9 (8H, m), 4.27 (1H, m), 4.5 - 4.9 (8H, m), 5.15 - 5.7 (8H, m), 5.8 - 6.2 (4H, m), 7.67 (1H, s), 8.25 (1H, s).

Example 49-2

$UV_{max}$ nm ($H_2O$): 298.
$IR_{max}$ $cm^{-1}$ (KBr): 3425, 1760, 1599, 1448, 1385, 1266.
NMR δ ppm ($D_2O$): 1.25 (3H, d, J = 7.3 Hz), 1.50 (3H, d, J = 6.3 Hz), 2.06 (1H, m), 2.42 (1H, m), 3.30 - 4.50 (9H, m), 5.58 (1H, m), 7.28 (1H, s), 7.72 (1H, s)

Example 50-1

$IR_{max}$ $cm^{-1}$ (neat): 1780, 1746, 1711, 1599, 1512, 1426, 1383, 1324, 1272, 1208, 1137.
NMR δ ppm ($CDCl_3$): 1.25 (3H, d, J = 7.3 Hz), 1.52 (3H, d, J = 6.3 Hz), 2.91 (2H, m), 3.56 (2H, m), 3.86 (2H, m), 4.30 (1H, br. d, J = 9.6 Hz), 4.75 (8H, m), 5.40 (9H, m), 6.04 (4H, m), 6.89 (1H, d, J = 8.3 Hz), 7.57 (1H, s), 7.63 (1H, d, J = 8.3 Hz). 9.19 (1H, s).

Example 50-2

UV $_{max}$ nm ($H_2O$): 263, 298.
NMR δ ppm ($D_2O$): 1.21 (3H, d, J = 6.9 Hz), 1.44 (3H, d, J = 6.3 Hz), 2.85 (1H, m), 3.06 (1H, m), 3.43 (3H, m), 3.73 (1H, m), 4.34 (1H, br. d, J = 9.2 Hz), 5.43 (1H, m), 6.92 (1H, d, J = 7.9 Hz), 7.52 (1H, s), 7.52 (1H, d, J = 7.9 Hz), 8.07 (1H, s).

Example 51-1

$IR_{max}$ $cm^{-1}$ (neat): 3330, 1750 (sh), 1731, 1633, 1590, 1498, 1292, 1246, 1184.
NMR δ ppm ($CDCl_3$): 0.08 - 0.22 (21H, m), 0.99 (18H, m), 1.11 (3H, d, J = 5.9 Hz), 1.24 (3H, m), 2.98 (1H, m), 3.05 - 3.39 (2H, m), 3.51 (1H, m), 3.70 - 3.94 (2H, m), 4.13 (1H, m), 4.67 (2H, m), 5.14 - 5.50 (2H, m), 5.89 (1H, m), 6.82 (1H, m).

Example 51-2

$IR_{max}$ $cm^{-1}$ (neat): 3350 (br), 1752, 1691, 1632, 1593, 1548, 1510, 1305 (sh), 1286, 1209, 1138.
NMR δ ppm ($CDCl_3$-$CD_3OD$ (10:1)): 1.20 (3H, d, J = 7.3 Hz), 1.31 (3H, d, J = 6.3 Hz), 2.87 (1H, m), 3.57 - 3.67 (3H, m), 4.75 (2H, m), 5.36 (2H, m), 5.97 (1H, m), 6.85 (1H, d, J = 8.3 Hz), 7.17 (1H, dd, J = 8.3 & 2.0 Hz), 7.25 (1H, d, J = 2.0 Hz).

Example 51-3

$UV_{max}$ nm ($H_2O$): 258, 296.
$IR_{max}$ $cm^{-1}$ (KBr): 3384, 1750, 1593, 1512, 1394, 1293.
NMR δ ppm ($D_2O$): 1.11 (3H, d, J = 7.3 Hz), 1.19 (3H, d, J = 6.3 Hz), 2.90 (1H, m), 3.18 (1H, m), 3.30 (1H, m), 3.38 (2H, m), 3.50 (1H, m), 3.76 (1H, m), 4.08 (1H, m), 6.97 (1H, d, J = 8.3 Hz), 7.28 (2H, m).

Example 52-1

$IR_{max}$ $cm^{-1}$ (neat): 1773, 1710, 1578, 1320, 1278, 1206.
NMR δ ppm ($CDCl_3$): 1.25 (3H, d, J = 7.3 Hz), 1.31 (3H, t, J = 7.4 Hz), 1.59 (3H, d, J = 6.3 Hz), 2.82 (2H, m), 3.35 (1H, m), 3.57 (1H, dd, J = 2.6 & 8.2 Hz). 4.24 (1H, dd, J = 2.6 & 9.2 Hz), 4.6 - 4.9 (6H, m), 5.2 - 5.7 (7H, m), 5.9 - 6.2 (3H, m), 7.67 (1H, s), 8.26 (1H, s).

Example 52-2

$UV_{max}$ nm ($H_2O$): 304.
$IR_{max}$ $cm^{-1}$ (KBr): 3250, 1762, 1560, 1382, 1264.
NMR δ ppm ($D_2O$): 1.24 (3H, t, J = 6.9 Hz), 1.28 (3H, d, J = 7.3 Hz), 1.51 (3H, d, J = 6.3 Hz), 2.82 (2H, m), 3.78 (1H, dd, J = 5.6 & 1.0 Hz), 3.97 (1H, m), 4.32 (1H, br. d, J = 11.2 Hz), 5.58 (1H, m), 7.30 (1H, s), 7.72 (1H, s).

Example 53-1

$IR_{max}$ $cm^{-1}$ (neat): 1768, 1707, 1508, 1262, 1204, 1133.
NMR δ ppm ($CDCl_3$): 1.26 (3H, d, J = 7.3 Hz), 1.32 (3H, t, J = 7.4 Hz), 1.53 (3H, d, J = 6.3 Hz), 2.84 (2H, m), 3.36 (1H, m), 3.49 (1H, dd, J = 2.6 & 7.9 Hz), 4.24 (1H, dd, J = 2.6 & 9.2 Hz), 4.55 - 4.95 (6H, m), 5.15 - 5.6 (7H, m), 5.9 - 6.2 (3H, m), 6.89 (1H, d, J = 8.3 Hz), 7.55 (1H, d, J = 2.0 Hz), 7.63 (1H, dd, J = 2.0 & 8.3 Hz).

Example 53-2

$UV_{max}$ nm ($H_2O$): 262, 295.
$IR_{max}$ $cm^{-1}$ (KBr): 3440, 1753, 1703, 1597, 1396, 1296,
NMR δ ppm ($D_2O$): 1.25 (6H, m), 1.46 (3H, d, J = 6.3 Hz), 2.83 (2H, m), 3.46 (1H, m), 3.74 (1H, m), 4.37 (1H, m), 5.47 (1H, m), 6.96 (1H, m), 7.55 (2H, m).

Example 54-1

$IR_{max}$ $cm^{-1}$ (neat): 1768, 1703, 1594, 1507, 1261, 1201, 1127.
NMR δ ppm ($CDCl_3$): 1.26 (3H, d, J = 7.3 Hz), 1.52 (3H, d, J = 6.3 Hz), 2.78 (3H, d, J = 4.6 Hz), 2.91 (1H, m), 3.14 (1H, m), 3.51 (2H, m), 4.21 (3H, m), 4.65 - 4.87 (7H, m), 5.22 - 5.50 (7H, m), 5.90 - 6.14 (3H, m), 6.89 (1H, d, J = 8.4 Hz), 7.55 (1H, d, J = 2.0 Hz), 7.63 (1H, dd, J = 8.4 & 2.0 Hz).

Example 54-2

$UV_{max}$ nm ($H_2O$): 212, 298.
$IR_{max}$ $cm^{-1}$ (KBr): 3382, 1756, 1705, 1597, 1393, 1283.
NMR δ ppm ($D_2O$): 1.23 (3H, d, J = 7.3 Hz), 1,47 (3H, d, J = 6.3 Hz), 2.66 (3H, s), 2.91 (1H, m), 3.20 (1H, m), 3.47 (1H, m), 3.76 (1H, dd, J = 5.1 & 2.3 Hz), 4.28 (2H, m), 5.47 (1H, m), 6.95 (1H, d, J = 8.3 Hz), 7.54 (1H, s), 7.55 (1H, d, J = 8.3 Hz).

Example 55-1

$IR_{max}$ $cm^{-1}$ (neat): 3350 (br), 1766, 1695, 1660, 1593, 1261, 1200, 1129.
NMR δ ppm ($CDCl_3$): 0.98 (3H, d, J = 7.3 Hz), 1.49 (3H, d, J = 6.3 Hz), 3.09 (1H, m), 3.47 (1H, m), 4.23 (1H, dd, J= 9.6 & 2.6 Hz), 4.60 - 4.92 (6H, m), 5.25 - 5.51 (7H, m), 6.03 (3H, m), 7.42 - 7.60 (4H, m), 7.78 - 7.84 (2H, m).

Example 55-2

$UV_{max}$ nm ($H_2O$): 213, 295 (sh).
$IR_{max}$ $cm^{-1}$ (KBr): 3356, 1754, 1672, 1600, 1402, 1283, 1226, 1119.
NMR δ ppm ($D_2O$): 1.00 (3H, d, J = 7.6 Hz), 1.41 (3H, d, J = 5.9 Hz), 3.18 (1H, m), 3.77 (2H, m), 5.45 (1H, m), 6.90 (1H, d, J = 9.1 Hz), 7.3 - 7.9 (6H, m).

Example 56-1

$IR_{max}$ $cm^{-1}$ (neat): 1772, 1707, 1594, 1507, 1294, 1252, 1205.
NMR δ ppm ($CDCl_3$): 0.21 (6H, s), 0.22 (6H, s), 0.98 (9H, s), 1.00 (9H, s), 1.28 (3H, d, J = 7.6 Hz), 1.48 (3H, d, J = 6.3 Hz), 3.47 (1H, m), 3.73 (1H, m), 4.04 - 4.28 (3H, m). 5.19 - 5.52 (3H, m), 6.20 (1H, d, J = 15.8 Hz), 6.83 (1H, m), 7.01 (2H, m), 7.21 (1H, m), 7.38 (1H, d, J = 7.6 Hz), 7.58 (1H, d, J = 15.8 Hz), 7.63 (2H, d, J = 8.9 Hz), 7.69 (1H, dd, J = 7.6 & 1.7 Hz), 8.20 (2H, d, J = 8.9 Hz), 8.51 (1H, d, J = 5.0 Hz).

Example 56-2

$IR_{max}$ $cm^{-1}$ (neat): 3400 (br), 1759, 1693, 1592, 1513, 1338, 1266, 1141.
NMR δ ppm ($CDCl_3$): 1.25 (3H, d, J = 7.3 Hz), 1.45 (3H, d, J = 6.3 Hz), 3.45 (1H, m), 3.70 (1H, m), 4.06 - 4.29 (3H, m), 5.19 - 5.47 (3H, m), 6.21 (1H, d, J = 15.8 Hz), 6.8 - 7.8 (9H, m), 8.15 (2H, d, J = 8.9 Hz), 8.51 (1H, d, J = 5.0 Hz).

Example 56-3

$UV_{max}$ nm ($H_2O$): 307.
$IR_{max}$ $cm^{-1}$ (KBr): 3345, 1754, 1704, 1596, 1392, 1261, 1162.
NMR δ ppm ($D_2O$): 1.14 (3H, d, J = 6.6 Hz), 1.39 (3H, d, J = 6.6 Hz), 3.35 (1H, m), 3.6 - 4.3 (5H, m), 5.35 (1H, m), 6.38 (1H, d, J = 14.9 Hz), 6.92 (1H, d, J = 8.3 Hz), 7.10 (1H, d, J = 8.3 Hz), 7.17 (1H, s), 7.34 (1H, dd, J = 7.9 & 4.6 Hz), 7.52 (1H, d, J = 7.9 Hz), 7.62 (1H, d, J = 14.9 Hz), 7.83 (1H, t, J = 7.9 Hz), 8.44 (1H, d, J = 4.6 Hz).

Example 57-1

$IR_{max}$ $cm^{-1}$ (neat): 1792, 1715, 1599, 1512, 1300, 1256,
NMR δ ppm ($CDCl_3$): 0.20 (12H, s), 0.97 (9H, s), 0.98 (9H, s), 1.38 (3H, t, J = 7.3 Hz), 1.53 (3H, d, J = 6.3 Hz), 3.00 (2H, m), 4.12 (1H, dd, J = 7.6 & 1.3 Hz), 5.23 (1H, d, J = 13.9 Hz), 5.44 (1H, d, J = 13.9 Hz), 5.46 (1H, m), 6.83 (1H, d, J = 8.3 Hz), 7.63 (4H, m), 8.18 (2H, d, J = 8.9 Hz).

Example 57-2

$IR_{max}$ $cm^{-1}$ (neat): 3350, 1780, 1692, 1597, 1514, 1324, 1284, 1187, 1104.
NMR δ ppm ($CDCl_3$): 1.39 (3H, t, J = 7.6 Hz), 1.52 (3H, d, J = 6.3 Hz), 3.03 (2H, m), 3.98 (1H, dd, J = 5.9 & 2.3 Hz), 5.24 (1H, d, J = 13.5 Hz), 5.38 (1H, d, J = 13.5 Hz), 5.46 (1H, m), 5.78 (1H, d, J = 1.3 Hz), 6.82 (1H, d, J = 8.3 Hz), 7.54 (4H, m), 8.12 (2H, d, J = 8.6 Hz).

Example 57-3

$UV_{max}$ nm ($H_2O$): 257, 297 (sh).
NMR δ ppm ($D_2O$): 1.32 (3H, t, J = 8.0 Hz), 1.56 (3H, d, J = 6.3 Hz), 2.97 (2H, m), 4.22 (1H, br. d, J = 5.5 Hz), 5.47 (1H, m), 5.84 (1H, br. s), 6.94 (1H, d, J = 9.1 Hz), 7.53 (1H, br. s), 7.56 (1H, d, J = 9.1 Hz).

Example 58-1

$IR_{max}$ $cm^{-1}$ (neat): 1796, 1712, 1659, 1511, 1346, 1122.
NMR δ ppm ($CDCl_3$): 0.20, 0.22 and 0.23 (12H in total, s), 0.98 (9H, s), 0.99 (9H, s), 1.95 (1H, m), 2.81 (1H, m), 2.92, 2.98, 2.99 and 3.08 (6H in total, s), 3.58 (1H, m), 3.76 (1H, m), 3.97 (1H, br. d, J = 6.9 Hz), 4.73 (1H, m), 5.0 - 5.5 (5H, m), 6.18 (1H, d, J = 15.5 Hz), 6.95 (3H, m), 7.63 (5H, m), 8.24 (4H, m).

Example 58-2

| | |
|---|---|
| $IR_{max}$ $cm^{-1}$ (KBr): | 3336, 1794, 1702, 1638, 1607, 1522, 1346, 1114. |
| NMR δ ppm ($CDCl_3$-DMSO-$d_6$ (9:1)): | 1.47 (3H, d, J = 5.9 Hz)[6], 1.99 (1H, m), 2.82 (1H, m), 2.93, 2.98 and 3.10 (6H in total, s), 3.50 (1H, m), 3.78 (1H, m), 3.97 (1H, br. d, J = 7.3 Hz), 4.27 (1H, m), 5.0 - 5.5 (5H, m), 5.78 (1H, br. s), 6.17 (1H, d, J = 15.5 Hz), 6.90 (3H, m), 7.52 (5H, m), 8.21 (4H, m). |

<u>Example 58-3</u>

| | |
|---|---|
| $UV_{max}$ nm ($H_2O$): | 292 (sh), 323. |
| $IR_{max}$ $cm^{-1}$ (KBr): | 3344, 1781, 1702, 1631, 1376, 1262, 1162. |
| NMR δ ppm ($D_2O$): | 1.42 (3H, d, J = 6.3 Hz), 2.98 (3H, s), 3.07 (3H, s), 5.40 (1H, m), 6.41 (1H, d, J = 16.5 Hz), 6.94 (1H, d, J = 7.9 Hz), 7.13 (1H, d, J = 7.9 Hz), 7.21 (1H, s), 7.64 (1H, d, J = 16.5 Hz). |

<u>Example 59-1</u>

| | |
|---|---|
| $IR_{max}$ $cm^{-1}$ (neat): | 1782, 1700, 1589, 1505, 1341, 1304, 1286, 1251, 1159, 1122. |
| NMR δ ppm ($CDCl_3$): | 0.21 (6H, s), 0.22 (6H, s), 0.98 (9H, s), 0.99 (9H, s), 1.48 (3H, d, J = 6.6 Hz), 3.97 (1H, m), 5.07 - 5.52 (5H, m), 5.68 (1H, d, J = 2.0 Hz), 6.18 (1H, d, J = 15.8 Hz), 6.8 - 7.05 (3H, m), 7.54 - 7.65 (3H, m), 8.21 (2H, d, J = 8.6 Hz). |

<u>Example 59-2</u>

| | |
|---|---|
| $IR_{max}$ $cm^{-1}$ (neat): | 3350 (br), 1773, 1698, 1594, 1511, 1341, 1307, 1155. |
| NMR δ ppm ($CDCl_3$-$CD_3OD$ (9:1)): | 1.46 (3H, d, J = 6.3 Hz), 3.98 (1H, m), 5.06 - 5.51 (5H, m), 5.70 (1H, d, J = 1.7 Hz), 6.18 (1H, d, J = 15.8 Hz), 6.80 (1H, d, J = 7.9 Hz), 6.90 (1H, dd, J = 7.9 & 2.0 Hz), 6.99 (1H, d, J = 2.0 Hz), 7.55 (1H, d, J = 15.8 Hz), 7.58 (2H, d, J = 8.9 Hz), 8.16 (2H, d, J = 8.9 Hz). |

<u>Example 59-3</u>

| | |
|---|---|
| $UV_{max}$ nm ($H_2O$): | 297, 315 (sh). |
| $IR_{max}$ $cm^{-1}$ (KBr): | 3364, 1775, 1703, 1606, 1384, 1265, 1162. |
| NMR δ ppm ($D_2O$): | 1.43 (3H, d, J = 6.3 Hz), 4.21 (1H, d, J = 5.3 Hz), 5.06 (1H, d, J = 14.5 Hz), 5.39 (1H, m), 5.41 (1H, d, J = 14.5 Hz), 5.80 (1H, br. s), 6.41 (1H, d, J = 15.8 Hz), 6.94 (1H, d, J = 8.3 Hz), 7.10 (1H, d, J = 8.3 Hz), 7.20 (1H, s), 7.64 (1H, d, J = 15.8 Hz). |

<u>Example 60-1</u>

| | |
|---|---|
| $IR_{max}$ $cm^{-1}$ (neat): | 1785, 1707, 1630, 1591, 1508, 1345, 1313, 1288, 1253, 1159, 1122. |
| NMR δ ppm ($CDCl_3$): | 0.20 - 0.24 (12H, m), 0.98 - 1.01 (18H, m), 1.46 - 1.50 (3H, m), 1.7 - 2.5 (4H, m), 3.80 - 4.01 (3H, m), 5.18 - 5.63 (5H, m), 6.15 - 6.44 (1H, m), 6.81 (1H, m), 6.98 - 7.08 (2H, m), 7.54 - 7.65 (3H, m), 8.18 - 8.24 (2H, m). |

<u>Example 60-2</u>

| | |
|---|---|
| $IR_{max}$ $cm^{-1}$ (neat): | 3390 (br), 1780, 1700, 1600, 1515, 1343, |
| NMR δ ppm ($CDCl_3$): | 1.46 (3H, d, J = 6.3 Hz), 1.7 - 2.5 (4H, m), 3.81 - 4.02 (3H, m), 5.19 - 5.65 (5H, m), 6.20 (1H, d, J = 15.8 Hz), 6.80 - 7.00 (3H, m), 7.52 - 7.58 (3H, m), 8.10 - 8.16 (2H, m). |

<u>Example 60-3</u>

| | |
|---|---|
| $UV_{max}$ nm ($H_2O$): | 308. |
| $IR_{max}$ $cm^{-1}$ (KBr): | 3364, 1767, 1702, 1600, 1380, 1263, 1161. |

NMR δ ppm ($D_2O$): 1.42 (3H, d, J = 6.3 Hz), 2.03 (4H, m), 3.92 (2H, m), 4.16 (1H, m), 5.36 (1H, m), 5.52 (1H, m), 5.69 (0.8H, br. s), 5.75 (0.2H, br. s), 6.40 (1H, d, J = 16.2 Hz), 6.95 (1H, d, J = 8.3 Hz), 7.13 (1H, d, J = 8.3 Hz), 7.20 (1H, br. s), 7.64 (1H, d, J = 16.2 Hz).

Example 61

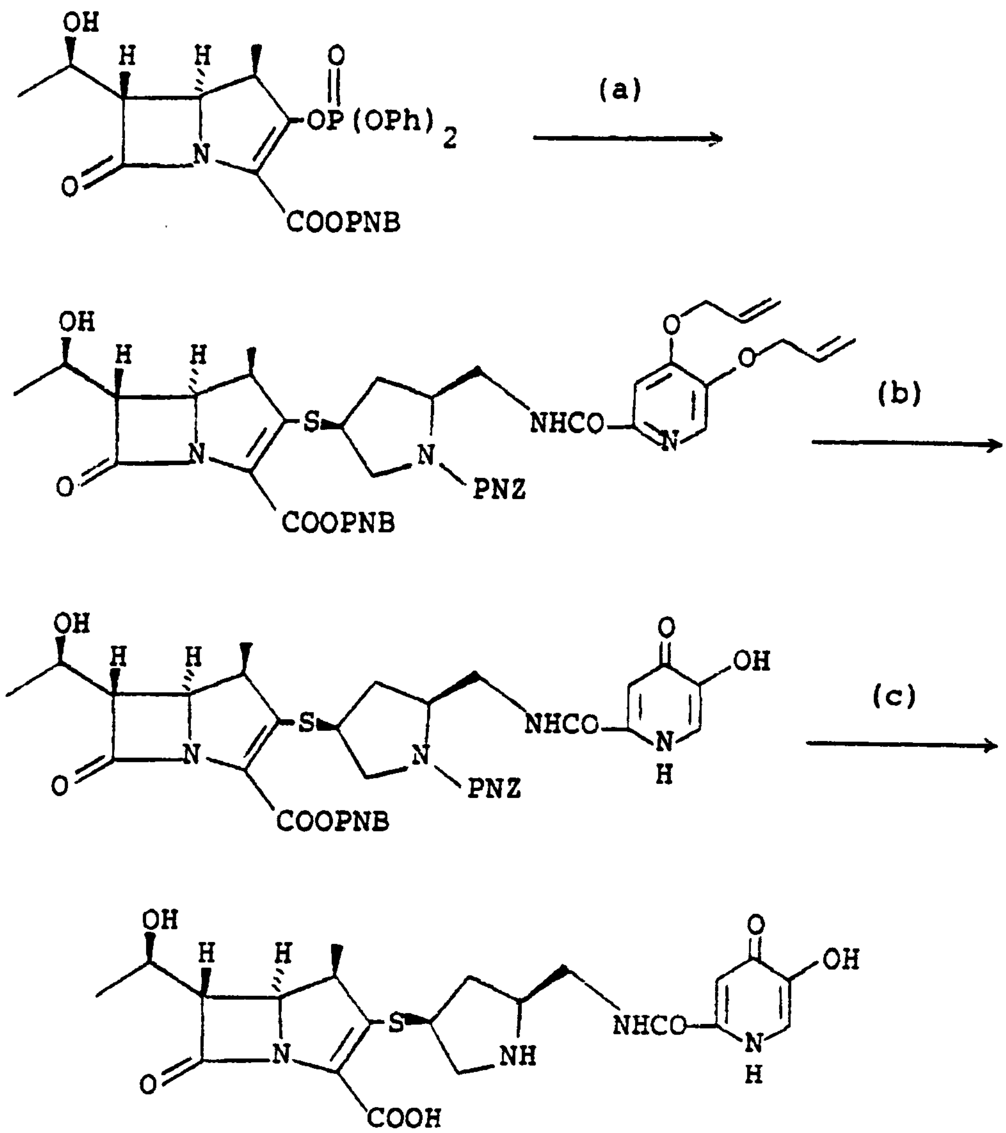


(a) To a solution of (4R,5R,5S,8R)-p-nitrobenzyl-3-diphenylphosphoryloxy-4-methyl-6-(1-hydroxyethyl)-1-azabicyclo[3.2.0]hept-2-en-7-one-2-carboxylate (356 mg) in dry acetonitrile (2.3 ml), a solution of (2S,4S)-1-p-nitrobenzyloxycarbonyl-2-(4,5-diallyloxypyridine-2-carbonyl)aminomethyl-4-mercaptopyrrolidine (270 mg) in dry acetonitrile (2.3 ml) was added in nitrogen stream while ice-cooling, followed by addition of diisopropylethylamine (77 mg). The resultant mixture was stirred at the same temperature for 2 hours. The reaction mixture was diluted with ethyl acetate, washed with water and an aqueous sodium chloride solution in order, and dried over magnesium sulfate and sodium carbonate and concentrated. The residue was chromatographed on silica gel column to give (4R,5S,6S,8R,2'S,4'S)-p-nitrobenzyl-3-[1-p-nitrobenzyloxycarbonyl-2-(4,5-diallyloxypyridine-2-carbonylaminomethyl)pyrrolidin-4-ylthio]-4-methyl-6-(1-hydroxyethyl)-1-azabicyclo[3.2.0]hept-2-en-7-one-2-carboxylate (302 mg).

$IR_{max}$ $cm^{-1}$ (neat): 3370 (br), 1762, 1698, 1680 (sh), 1670 (sh), 1516, 1502 (sh), 1341, 1316, 1272, 1202.

NMR δ ppm ($CDCl_3$): 1.27 (3H, d, J = 6.9 Hz), 1.37 (3H, d, J = 6.3 Hz), 2.56 (1H, m), 3.2 - 4.4 (10H, m), 4.72 (4H, m), 5.1 - 5.6 (8H, m), 5.95 - 6.20 (2H, m), 7.45 - 7.80 (5H, m), 7.95 - 8.45 (5H, m).

(b) To a solution of (4R,5S,6S,8R,2'S,4'S)-p-nitrobenzyl-3-[1-p-nitrobenzyloxycarbonyl-2-(4,5-diallyloxypyridine-2-carbonylaminomethyl)pyrrolidin-4-ylthio]-4-methyl-6-(1-hydroxyethyl)-1-azabicyclo-[3.2.0]hept-2-en-7-one-2-carboxylate (249 mg) in dry methylene chloride (5.6 ml), dimedone (157 mg) was added in nitrogen stream, followed by addition of tetrakistriphenylphosphine palladium (32 mg) under ice-cooling. Then, the resultant mixture was stirred at room temperature for 40 minutes. After removal of the solvent, the residue was purified by thin layer chromatography to give (4R,5S,6S,8R,2'S,4'S)-p-nitrobenzyl-3-[1-p-nitrobenzyloxycarbonyl-2-(5-hydroxy-4-pyridone-2-carbonylaminomethyl)pyrrolidin-4-ylthio]-4-methyl-6-(1-hydroxyethyl)-1-azabicyclo[3.2.0]hept-2-en-7-one-2-carboxylate (156 mg).

$IR_{max}$ $cm^{-1}$ (neat): 3320 (br), 1762, 1695, 1518, 1342.

NMR δ ppm ($CDCl_3$-$CD_3OD$ (9:1)): 1.23 (3H, d, J = 7.3 Hz), 1.33 (3H, d, J = 5.9 Hz), 1.85 (1H, m), 2.63 (1H, m), 5.1 - 5.6 (4H, m), 7.4 - 7.8 (5H, m), 8.05 - 8.35 (5H, m).

(c) (4R,5S,6S,8R,2'S,4'S)-p-Nitrobenzyl-3-[1-p-nitrobenzyloxycarbonyl-2-(5-hydroxy-4-pyridone-2-carbonylaminomethyl)pyrrolidin-4-ylthio]-4-methyl-6-(1-hydroxyethyl)-1-azabicyclo[3.2.0]hept-2-en-7-one-2-carboxylate (114 mg) was dissolved in tetrahydrofuran (6 ml) and 0.1M phosphate buffer (pH, 7.0; 0.6 ml), followed by addition of 10 % palladium-carbon (120 mg). Catalytic hydrogenation was performed at room temperature under atmospheric pressure for 1.5 hours. After removal of the catalyst by filtration, the filtrate was washed with methylene chloride. The organic solvent in the aqueous phase was removed by distillation under reduced pressure. The residual solution was filtered through a membrane filter, and the filtrate was purified by column chromatography (CHP-20P polymer) with 2 % aqueous tetrahydrofuran solution as an eluent and freeze-dried to give (4R,5S,6S,8R,2'S,4'S)-3-[2-(5-hydroxy-4-pyridone-2-carbonylaminomethylpyrrolidin-4-ylthio]-4-methyl-1-azabicyclo[3.2.0]hept-2-en-7-one-2-carboxylic acid as a white amorphous substance.

$UV_{max}$ nm ($H_2O$): 295.

$IR_{max}$ $cm^{-1}$ (KBr): 3358 (br), 1752, 1654, 1560, 1389.

NMR δ ppm ($D_2O$): 1.22 (3H, d, J = 6.6 Hz), 1.30 (3H, d, J = 6.3 Hz), 1.78 (1H, m), 2.76 (1H, m), 3.25 - 4.35 (10H, m), 7.12 (1H, s), 7.76 (1H, s).

Examples 62 to 66

In the same manner as above, the compounds as shown in Table 6 were obtained.

Table 6

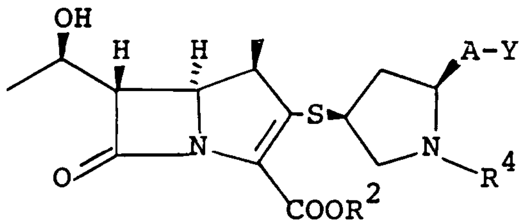

| Example No. | $R^2$ | A-Y | $R^4$ |
|---|---|---|---|
| 62-1 | PNB | 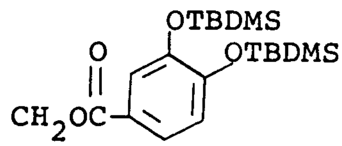 | PNZ |
| 62-2 | PNB | 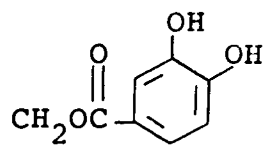 | PNZ |
| 62-3 | H | 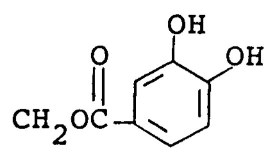 | H |

| | | | |
|---|---|---|---|
| 63-1 | PNB | 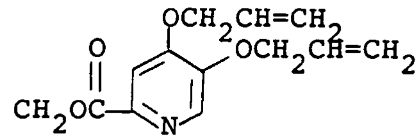 | PNZ |
| 63-2 | PNB | 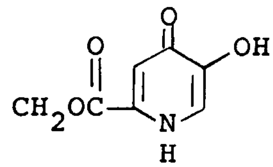 | PNZ |
| 63-3 | H | 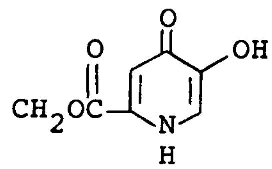 | H |
| 64-1 | $CH_2CH=CH_2$ | 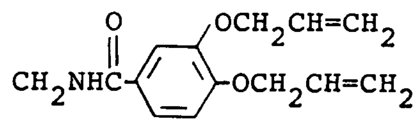 | AOC |
| 64-2 | H | 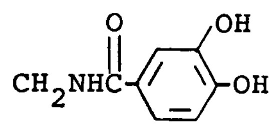 | H |
| 65-1 | $CH_2CH=CH_2$ | 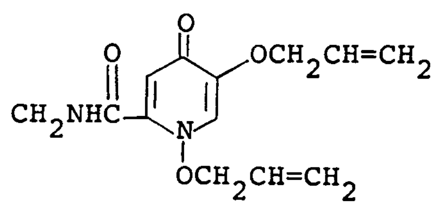 | AOC |
| 65-2 | H | 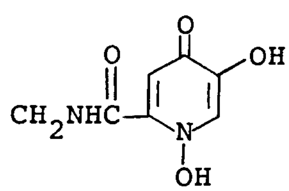 | H |
| 66-1 | $CH_2CH=CH_2$ | 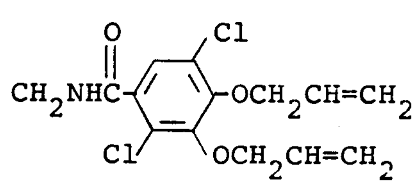 | AOC |

| | | | |
|---|---|---|---|
| 66-2 | H | 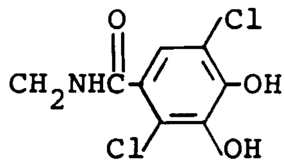 | H |

Physical property

Example 62-1

IR$_{max}$ cm$^{-1}$ (neat): 3470 (br), 1764, 1718 (sh), 1710, 1600, 1516, 1420, 1342, 1300, 1254, 1208.
NMR δ ppm ($CDCl_3$): 0.22 (6H, s), 0.23 (6H, s), 0.99 (18H, s), 1.27 (3H, d, J = 7.3 Hz), 1.37 (3H, d, J = 6.3 Hz), 2.08 (1H, m), 2.60 (1H, m), 3.28 (1H, dd, J = 2.5 & 6.8 Hz), 3.35 (1H, m), 3.65 (1H, m), 4.0 - 4.6 (6H, m), 5.1 - 5.6 (4H, m), 6.85 (1H, d, J = 8.9 Hz), 7.51 (4H, d, J = 8.9 Hz), 7.65 (2H, d, J = 8.9 Hz), 8.20 (2H, d, J = 8.9 Hz), 8.22 (2H, d, J = 8.6 Hz).

Example 62-2

IR$_{max}$ cm$^{-1}$ (neat): 3420 (br), 1760, 1715 (sh), 1700, 1602, 1518, 1440, 1344, 1290, 1208.
NMR δ ppm ($CDCl_3$-$CD_3OD$ (9:1)): 1.28 (3H, d, J = 7.3 Hz), 1.34 (3H, d, J = 6.3 Hz), 2.65 (1H, m), 3.17 (1H, m), 3.26 (1H, dd, J = 2.6 & 6.9 Hz), 4.05 - 4.55 (6H, m), 5.10 - 5.55 (4H, m), 6.83 (1H, d, J = 8.6 Hz), 7.4 - 7.6 (4H, m), 7.66 (1H, d, J = 8.9 Hz), 8.19 (2H, d, J = 8.9 Hz), 8.22 (2H, d, J = 8.9 Hz).

Example 62-3

UV$_{max}$ nm ($H_2O$): 297.
IR$_{max}$ cm$^{-1}$ (KBr): 3370 (br), 1752, 1717, 1599, 1394, 1298, 1225.
NMR δ ppm ($D_2O$): 1.24 (3H, d, J = 6.3 Hz), 1.30 (3H, d, J = 6.3 Hz), 2.73 (1H, m), 6.94 (1H, d, J = 8.6 Hz), 7.5 - 7.8 (2H, m).

Example 63-1

IR$_{max}$ cm$^{-1}$ (neat): 3400 (br), 1760, 1696, 1515, 1340, 1317 (sh), 1270, 1195.
NMR δ ppm ($CDCl_3$): 1.27 (3H, d, J = 7.3 Hz), 1.37 (3H, d, J = 6.3 Hz), 2.64 (1H, m), 3.27 (1H, dd, J = 2.5 & 6.8 Hz), 3.3 - 4.9 (13H, m), 5.1 - 5.6 (8H, m), 5.9 - 6.2 (2H, m), 7.45 - 7.75 (5H, m), 8.1 - 8.4 (5H, m).

Example 63-2

IR$_{max}$ cm$^{-1}$ (neat): 3350 (br), 1752, 1690, 1510, 1335.
NMR δ ppm ($CDCl_3$-$CD_3OD$ (9:1)): 1.29 (3H, d, J = 6.9 Hz), 1.34 (3H, d, J = 6.3 Hz), 1.96 (1H, m), 2.74 (1H, m), 5.1 - 5.6 (4H, m), 7.4 - 7.8 (5H, m), 8.05 - 8.35 (5H, m).

Example 63-3

UV$_{max}$ nm ($H_2O$): 281.
IR$_{max}$ cm$^{-1}$ (KBr): 3374 (br), 1748, 1603, 1445, 1396.
NMR δ ppm ($D_2O$): 1.19 (3H, d, J = 7.3 Hz), 1.27 (3H, d, J = 5.9 Hz), 1.9 (1H, m), 2.6 (1H, m), 3.2 - 4.5 (10H, m), 6.85 (1H, s), 7 73 (1H, s).

Example 64-1

$IR_{max}$ $cm^{-1}$ (neat): 3350 (br), 1767, 1694, 1640, 1504, 1410, 1268.
NMR δ ppm ($CDCl_3$): 1.27 (3H, d, J = 7.3 Hz), 1.36 (3H, d, J = 6.3 Hz), 2.63 (1H, m), 3.1 - 4.4 (9H, m), 4.5 - 4.9 (8H, m), 5.1 - 5.6 (8H, m), 5.8 - 6.2 (4H, m), 6.89 (1H, d, J = 8.6 Hz), 7.36 (1H, s), 7.47 (1H, s).

Example 64-2

$UV_{max}$ nm ($H_2O$): 293.
$IR_{max}$ $cm^{-1}$ (KBr): 3436 (br), 1752, 1594, 1508, 1396, 1294.
NMR δ ppm ($D_2O$): 1.19 (3H, d, J = 7.3 Hz), 1.30 (3H, d, J = 5.9 Hz), 6.94 (1H, m), 7.2 - 7.6 (2H, m).

Example 65-1

$IR_{max}$ $cm^{-1}$ (neat): 3400 (br), 1772, 1700, 1684, 1653, 1559, 1208.
NMR δ ppm ($CDCl_3$): 1.26 (3H, d, J = 6.9 Hz), 1.35 (3H, d, J = 6.3 Hz), 2.5 - 4.4 (12H, m), 4.4 - 5.0 (8H, m), 5.15 - 5.6 (8H, m), 5.8 - 6.2 (4H, m), 6.44 (1H, br. s), 8.47 (1H, br. s).

Example 65-2

$UV_{max}$ nm ($H_2O$): 300.
$IR_{max}$ $cm^{-1}$ (KBr): 3251, 1751, 1593, 1506, 1391, 1290, 1149.
NMR δ ppm ($D_2O$): 1.23 (3H, d, J = 7.3 Hz), 1.29 (3H, d, J = 6.3 Hz), 1.84 (1H, m), 2.80 (1H, m), 3.42 (3H, m), 3.80 (3H, m), 4.06 (2H, m), 4.26 (2H, m), 7.38 (1H, s), 7.61 (1H, s).

Example 66-1

$IR_{max}$ $cm^{-1}$ (neat): 3350 (br), 1750, 1690, 1642 (sh), 1534, 1400, 1306, 1200, 1180.
NMR δ ppm ($CDCl_3$): 1.28 (3H, d, J = 7.3 Hz), 1.36 (3H, d, J = 6.3 Hz), 2.62 (1H, m), 3.29 (3H, m), 3.58 (1H, m), 4.0 - 4.4 (4H, m), 4.56 - 4.88 (8H, m), 5.19 - 5.49 (8H, m), 5.82 - 6.17 (4H, m).

Example 66-2

$UV_{max}$ nm ($H_2O$): 293.
$IR_{max}$ $cm^{-1}$ (KBr): 3388, 1752, 1596, 1458, 1395, 1304.
NMR δ ppm ($D_2O$): 1.21 (3H, d, J = 7.3 Hz), 1.27 (3H, d, J = 6.3 Hz), 5.55 (1H, m), 7.32 (1H, s).

Example 67

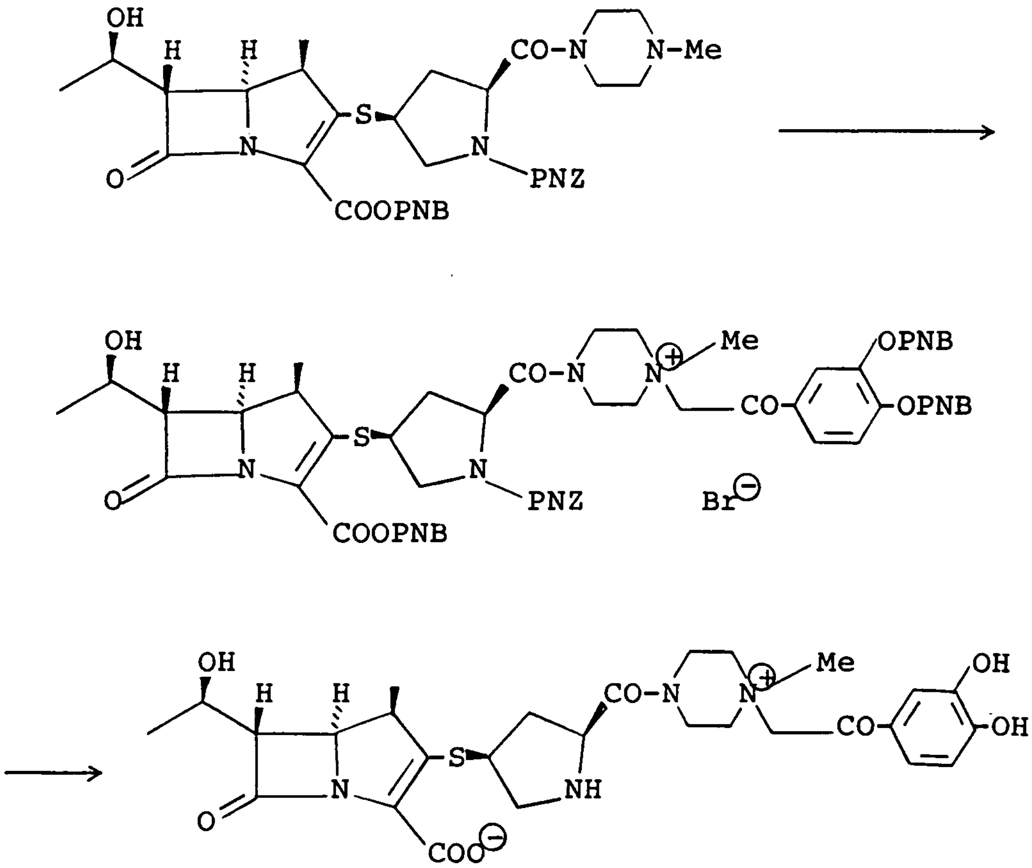

(4R,5S,6S,8R,2'S,4'S)-p-Nitrobenzyl-3-[1-p-nitrobenzyloxycarbonyl-2-(4-methylpiperazin-1-ylcarbonyl)-pyrrolidin-4-ylthio]-4-methyl-6-(1-hydroxyethyl)-1-azabicyclo[2.3.0]hept-2-en-7-one-2-carboxylate(147 mg) and 1-bromoacetyl-3,4-di(p-nitrobenzyloxy)benzene (200 mg) were dissolved in acetone (3.0 ml), and the resultant solution was allowed to react at room temperature for 10 days, followed by removal of the solvent under reduced pressure. The residue was dissolved in tetrahydrofuran (10.0 ml) and 0.1M phosphate buffer (pH, 7.0; 10.0 ml), 10 % palladium-carbon was added thereto, and catalytic hydrogenation was performed at room temperature under atmospheric pressure for 1.5 hours. The reaction mixture was treated in the same manner as in Example 1 and purified by column chromatography (CHP-20P polymer) using 8 % aqueous tetrahydrofuran solution as an eluent and freeze-dried to give (4R,5S,6S,8R,2'S,4'S)-3-[2-(4-(3,4-dihydroxyphenylcarbonylmethyl)-4-methylpiperazinium-1-ylcarbonyl)pyrrolidin-4-ylthio]-4-methyl-6-(1-hydroxyethyl)-1-azabicyclo[3.2.0]hept-2-en-7-one-2-carboxylate.

$UV_{max}$ nm ($H_2O$): 301.

$IR_{max}$ $cm^{-1}$ (KBr): 3402 (br), 1758, 1664, 1594, 1489, 1453, 1388, 1255, 1208, 1090.

NMR δ ppm ($D_2O$): 1.22 (3H, d, J = 7.3 Hz), 1.30 (3H, d, J = 6.9 Hz), 3.46 (3H, s), 6.64 (1H, d, J = 8.3 Hz), 7.24 (2H, m).

Example 68

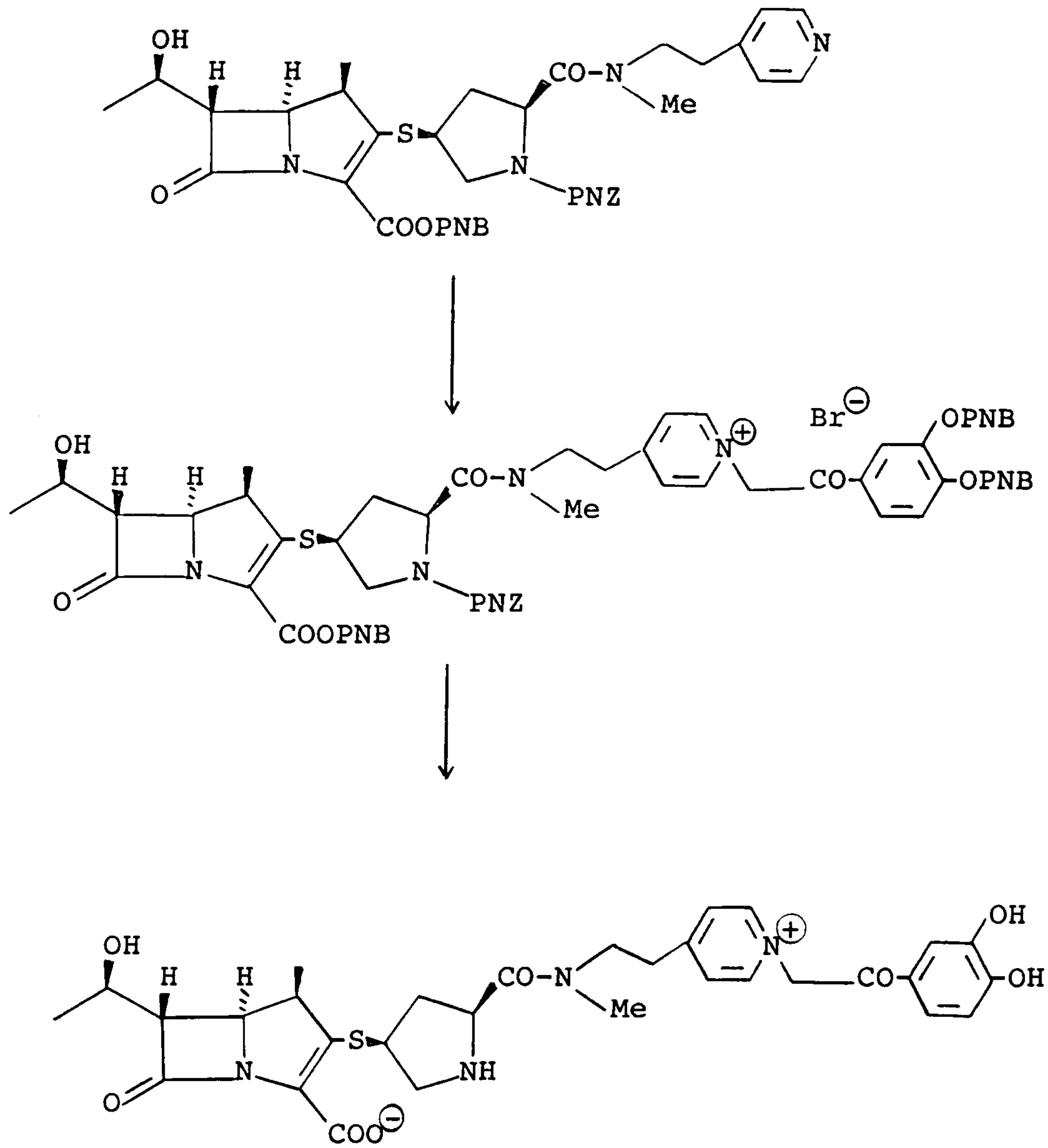

(4R,5S,6S,8R,2'S,4'S)-p-Nitrobenzyl-3-[1-p-nitrobenzyloxycarbonyl-2-((2-(4-pyridyl)ethyl)-methylaminocarbonyl)pyrrolidin-4-ylthio]-4-methyl-6-(1-hydroxyethyl)-1-azabicyclo[3.2.0]hept-2-en-7-one-2-carboxylate (248 mg) and 1-bromoacetyl-3,4-di(p-nitrobenzyloxy)benzene (228 mg) were dissolved in a mixture of dryl methylene chloride (4 ml) and dry dimethylformamide (2 ml), and the resultant solution was allowed to react at room temperature for 10 days. Catalytic hydrogenation was performed in the same manner as in Example 68, and the reaction mixture was post-treated and purified to give (4R,5S,6S,8R,2'S,4'S)-3-[2-((2-(1-(3,4-dihydroxyphenylcarbonylmethyl)pyridinium-4-yl)-ethyl)-methylaminocarbonyl)pyrrolidin-4-ylthio]-4-methyl-6-(1-hydroxyethyl)-1-azabicyclo[3.2.0]hept-2-en-7-one-2-carboxylate.

$UV_{max}$ nm ($H_2O$): 286.

$IR_{max}$ $cm^{-1}$ (KBr): 3400 (br), 2364, 1752, 1654, 1560, 1508, 1280.

NMR $\delta$ ppm ($D_2O$): 0.95 (3H, d, J = 6.9 Hz), 1.28 (3H, d, J = 6.6 Hz), 3.04 (3H, s), 6.89 (1H, d, J = 7.9 Hz), 7.2 - 7.5 (2H, m), 8.09 (2H, br. s), 8.70 (2H, br. s).

Reference Example 1

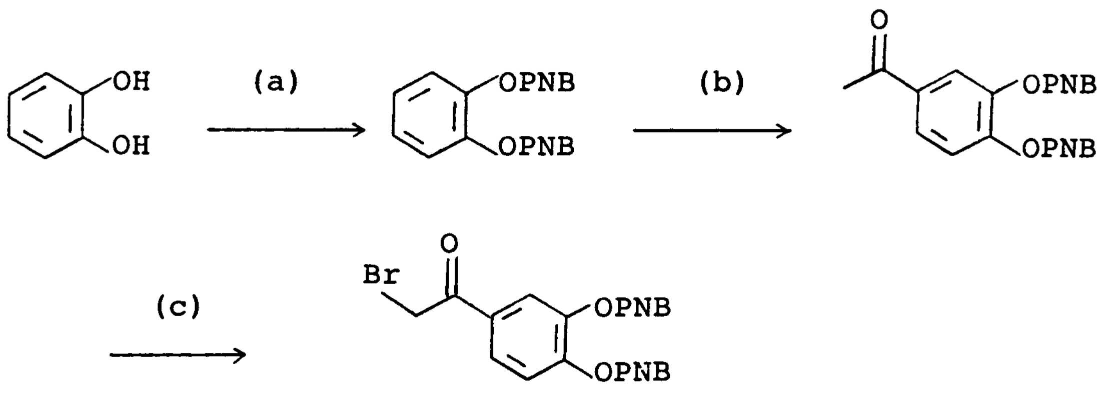


(a) To a suspension of 60 % sodium hydride (8.0 g) in dry tetrahydrofuran (50 ml), a solution of catechol (5.5 g) in dry tetrahydrofuran (25 ml) was added under nitrogen stream while ice-cooling, followed by addition of dry hexamethylphosphoramide (60 ml). The resultant mixture was stirred at 40°C for 10 minutes and again ice-cooled. A solution of p-nitrobenzyl bromide (9.5 g) in dry tetrahydrofuran (30 ml) was added thereto, and the mixture was stirred for 10 minutes. The reaction mixture was diluted with benzene, washed with a 5 % aqueous potassium phostate solution and an aqueous sodium chloride solution in order and dried over magnesium sulfate. After removal of the solvent by distillation, the residue was crystallized from ethyl acetate to give 1,2-di(p-nitrobenzyloxy)benzene (3.8 g).

$IR_{max}$ $cm^{-1}$ (KBr): 1608, 1511, 1453, 1343, 1259, 1218, 1128, 1047.

NMR δ ppm ($CDCl_3$): 5.26 (4H, s), 6.94 (4H, s), 7.62 (4H, d, J = 8.9 Hz), 8.24 (4H, d, J = 8.9 Hz).

(b) In a nitrogen gas-charged flask, anhydrous aluminum chloride (1.27 g) and dry methylene chloride (10 ml) were admitted in order, and the mixture was cooled to -10°C, followed by addition of acetyl chloride (640 μl). The resultant mixture was stirred for 10 minutes, and a solution of 1,2-di(p-nitrobenzyloxy)benzene (2.78 g) in dry methylene chloride (35 ml) was dropwise added thereto in 5 minutes. After stirring at the same temperature for 10 minutes, the reaction mixture was diluted with methylene chloride, washed with 0.1M phosphate buffer (pH, 7.0) and an aqueous sodium hydrogen carbonate solution and dried over magnesium sulfate. After removal of the solvent, the residue was crystallized from benzene to give 1-acetyl-3,4-di(p-nitrobenzyloxy)benzene (2.81 g).

$IR_{max}$ $cm^{-1}$ (KBr): 1673, 1607, 1586, 1516, 1430, 1352, 1276, 1218, 1151, 1040.

NMR δ ppm ($CDCl_3$): 2.54 (3H, s), 5.31 (2H, s), 5.34 (2H, s), 6.92 (1H, d, J = 8.2 Hz), 7.62 (6H, m), 8.27 (4H, m).

(c) A solution of 1-acetyl-3,4-di(p-nitrobenzyloxy)benzene (5.0 g) in dry tetrahydrofuran (350 ml) was stirred at 60°C, followed by addition of anhydrous aluminum choride (150 mg). To the resultant mixture, a solution of bromine (605 μl) in dry tetrahydrofuran (10 ml) was added in 5 minutes, and the mixture was stirred at the same temperatue for 10 minutes. After cooling, 0.01M phosphate buffer (pH, 7.0; 200 ml) was added to the reaction mixture, which was stirred for 1 hour under ice-cooling. The precipitated crystals were collected by filtration, washed with water, ethanol and ethyl acetate in order and dried under reduced pressure to give 1-bromoacetyl-3,4-di(p-nitrobenzyloxy)benzene (3.8 g).

$IR_{max}$ $cm^{-1}$ (KBr): 1667, 1607, 1517, 1437, 1348, 1278, 1224, 1150, 1108, 1037.

NMR δ ppm ($CDCl_3$): 4.36 (2H, s), 5.31 (2H, s), 5.35 (2H, s), 6.95 (1H, d, J = 8.9 Hz), 7.64 (6H, m), 8.27 (4H, m).

Reference Example 2

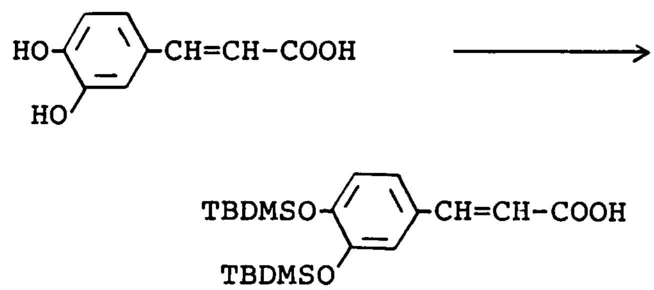

A solution of caffeic acid (3.60 g), t-butyldimethylsilyl chloride (10.85 g) and imidazole (6.12 g) in dry dimethylformamide (20 ml) was allowed to react at room temperature overnight. The reaction mixture was diluted with toluene, washed with water and an aqueous sodium chloride solution and dried over magnesium sulfate. Afte removal of the solvent, the residue was dissolved in a mixture of methanol (60 ml) and tetrahydrofuran (60 ml), and a solution of sodium hydrogen carbonate (1.35 g) in water (20 ml) was added thereto under ice-cooling, followed by stirring at room temperatur for 1 hour. The reaction mixture was made acidic with addition of dilute hydrochloric acid, extracted with ethyl acetate, washed with an aqueous sodium chloride solution and dried over magnesium sulfate. After removal of the solvent, the residue was crystallized from methanol to give 3,4-di(t-butyldimethylsilyloxy)cinnamic acid (7.37 g).

$IR_{max}$ $cm^{-1}$ (KBr): 3320 (br), 1690 (sh), 1680, 1630, 1504, 1282, 1250.

NMR δ ppm ($CDCl_3$): 0.22 (6H, s), 0.23 (6H, s), 0.99 (9H, s), 1.00 (9H, s), 6.25 (1H, d, J = 16 Hz), 6.83 (1H, d, J = 8.6 Hz), 7.03 (2H, s), 7.66 (1H, d, J = 16 Hz).

Reference Examples 3 to 5

In the same manner as in Reference Example 2, t-butyldimethylsilyl ethers as shown in Table 7 were obtained from their corresponding catechols.

Table 7

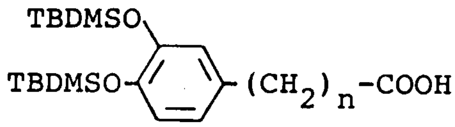

| Reference Example No. | n | Physical property | |
|---|---|---|---|
| 3 | 0 | $IR_{max}$ $cm^{-1}$ (KBr): | 3320 (br), 1684, 1599, 1515, 1438, 1298, 1255. |
| | | NMR δ ppm ($CDCl_3$): | 0.23 (6H, s), 0.24 (6H, s), 0.99 (9H, s), 1.00 (9H, s), 6.87 (1H, d, J = 8.3 Hz), 7.58 (1H, s), 7.61 (1H, d, J = 8.3 Hz). |
| 4 | 1 | $IR_{max}$ $cm^{-1}$ (neat): | 2900 (br), 1707, 1510, 1290, 1250. |
| | | NMR δ ppm ($CDCl_3$): | 0.22 (12H, s), 1.01 (18H, s), 3.54 (2H, s), 6.70 - 6.82 (3H, m). |
| 5 | 2 | $IR_{max}$ $cm^{-1}$ (KBr): | 1706, 1504, 1473, 1418, 1289, 1253, 1125. |
| | | NMR δ ppm ($CDCl_3$): | 0.18 (12H, s), 0.98 (18H, s), 2.61 (1H, m), 2.83 (2H, t, J = 7.8 Hz), 6.55 - 6.85 (3H, m). |

Reference Example 6

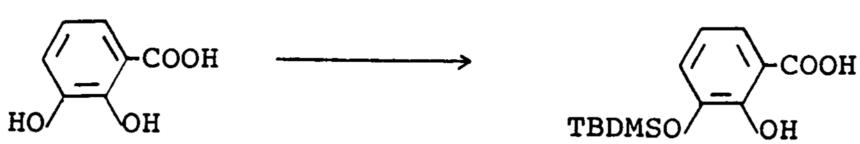

In the same manner as in Reference Example 2 but using 2,3-dihydroxybenzoic acid (4.62 g), 2-hydroxy-3-t-butyldimethylsilyloxybenzoic acid was obtained.

$IR_{max}$ $cm^{-1}$ (neat): 1645, 1459, 1442, 1390, 1300, 1267, 1255, 1228, 1176, 1158.

NMR δ ppm ($CDCl_3$): 0.22 (6H, s), 1.03 (9H, s), 6.80 (1H, m), 7.11 (1H, dd, J = 1.6 & 7.9 Hz), 7.55 (1H, dd, J = 1.6 & 8.1 Hz).

Reference Example 7

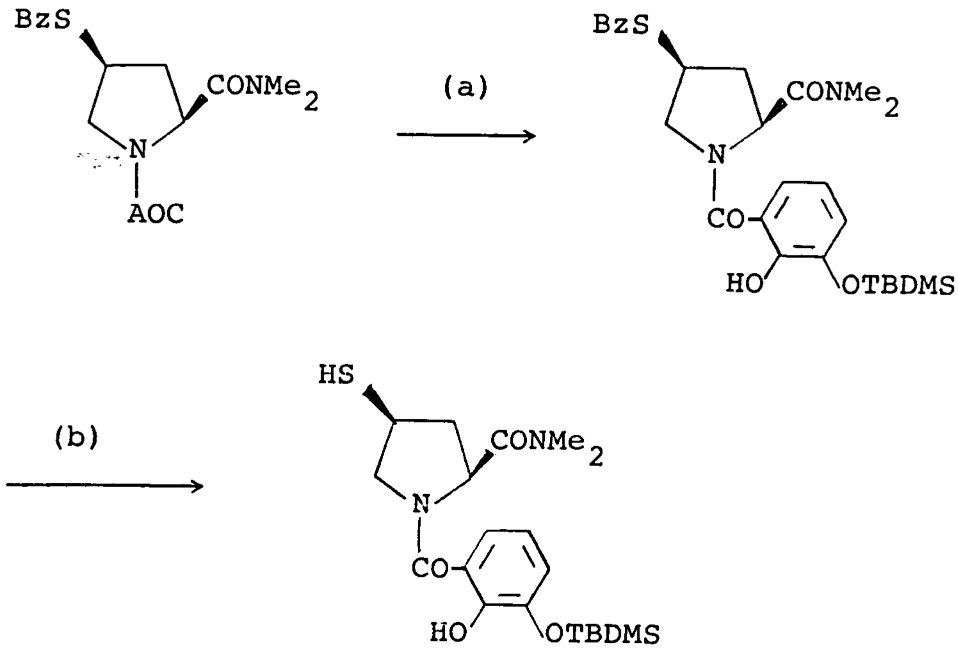


(a) To a solution of (2S,4S)-1-allyloxycarbonyl-2-dimethylaminocarbonyl-4-benzoylthiopyrrolidine (2.53 g) in dry methylene chloride (70 ml), dimedone (3.92 g) was added, and tetrakistriphenylphosphine palladium (809 mg) was added thereto in nitrogen stream under ice-cooling. The resultant mixture was stirred for 30 minutes, and 2-hydroxy-3-t-butyldimethylsilyloxybenzoic acid (1.88 g) and 1-ethyl-3-(3-dimethylaminopropyl)carbodiimide hydrochloride (1.34 g) were added thereto, followed by allowing to react at 5 °C for 1 day. The reaction mixture was diluted with ethyl acetate, washed with dilute hydrochloric acid and an aqueous sodium chloride solution in order and dried over magnesium sulfate. After removal of the solvent by distillation, the residue was chromatographed on silica gel column to give (2S,4S)-1-(2-hydroxy-3-t-butyldimethylsilyloxybenzoyl)-2-dimethylaminocarbonyl-4-benzoylthiopyrrolidine.

$IR_{max}$ $cm^{-1}$ (neat): 3150 (br), 1660, 1650 (sh), 1630 (sh), 1574, 1444, 1412, 1255, 1205.

NMR $\delta$ ppm ($CDCl_3$): 0.21 (6H, s), 1.01 (9H, s), 2.06 (1H, m), 2.83 (1H, m), 3.03 (3H, s), 3.21 (3H, s), 3.79 (1H, m), 4.13 (2H, m), 5.20 (1H, t, J = 8.3 Hz), 6.72 (1H, t, J = 7.9 Hz), 6.91 (1H, m), 7.03 (1H, d, J = 7.6 Hz), 7.4 - 7.55 (2H, m), 7.55 - 7.70 (1H, m), 7.90 (2H, dd, J = 1.5 & 8.5 Hz).

(b) (2S,4S)-1-(2-Hydroxy-3-t-butyldimethylsilyloxybenzoyl)-2-dimethylaminocarbonyl-4-benzoylthiopyrrolidine (682 mg) was dissolved in methanol (3 ml), and a 30 % methanolic solution of methylamine (399 mg) was added thereto in nitrogen stream under ice-cooling, followed by stirring for 30 minutes. A 30 % methanolic solution of methylamine (399 mg) was further added thereto, and the resultant mixture was stirred for 30 minutes. An aqueous solution of potassium phosphate (1.05 g) was added to the reaction mixture, which was extraced with methylene chloride. The organic layer was washed with a 2.5 % aqueous potassium phosphate solution and dried over magnesium sulfate. Removal of the solvent gave (2S,4S)-1-(2-hydroxy-3-t-butyldimethylsilyloxybenzoyl)-2-dimethylaminocarbonyl-4-mercaptopyrrolidine, which was used in the subsequent reaction without isolation.

Reference Example 8

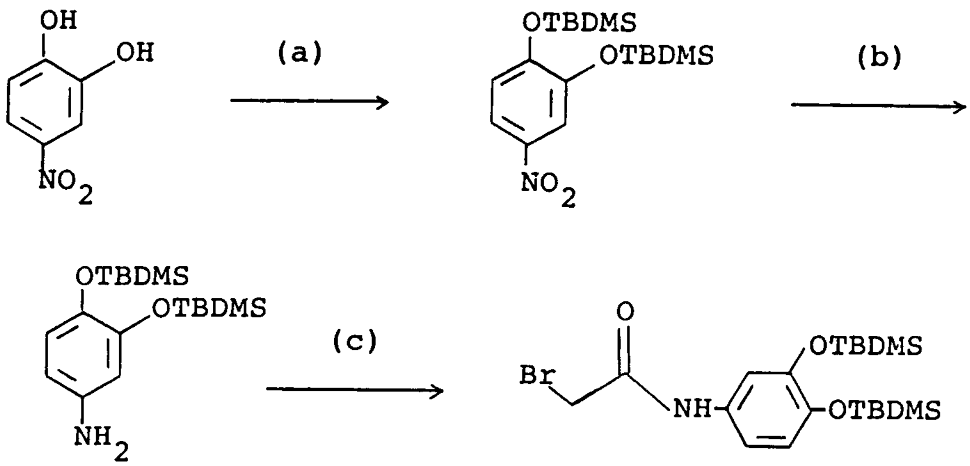


(a) To a solution of 1,2-dihydroxy-4-nitrobenzene (2.0 g) and imidazole (4.4 g) in dry dimethylformamide (10 ml), t-butyldimethylsilyl chloride (4.9 g) was added at room temperature, and the resultant mixture was stirred at the same temperature for 1 day. Stirring was continued at 60 °C for 3 hours. After cooling, the reaction mixture was diluted with benzene, washed with 0.1M phosphate buffer (pH, 7.0) and a saturated aqueous solution of sodium chloride and dried. After removal of the solvent, the precipitated crystals were collected, washed with methanol and dried under reduced pressure to give 1-nitro-3,4-di(t-butyldimethylsilyloxy)benzene (4.5 g).

$IR_{max}$ $cm^{-1}$ (KBr): 1583, 1515, 1338, 1301, 1268, 1236, 1088.

NMR δ ppm ($CDCl_3$): 0.15 (6H, s), 0.18 (6H, s), 0.96 (9H, s), 0.98 (9H, s), 6.16 (1H, d, J = 2.0 Hz), 6.21 (1H, dd, J = 8.3 & 2.0 Hz), 6.62 (1H, d, J = 8.3 Hz).

(b) 1-Nitro-3,4-di(t-butyldimethylsilyloxy)benzene (2.36 g) was dissolved in ethyl acetate (5 ml), and 10 % palladium-carbon (236 mg) was added thereto. Catalytic hydrogenation was performed at room temperature under atmospheric pressure for 1 hour. The catalyst was removed by filtration, and the filtrate was washed with ethyl acetate, followed by removal of the solvent to give 1-amino-3,4-di(t-butyldimethylsilyloxy)benzene.

$IR_{max}$ $cm^{-1}$ (neat): 3450, 3360, 1614, 1581, 1508, 1468, 1461, 1441, 1322, 1267, 1250, 1223, 1180, 1122.

NMR δ ppm ($CDCl_3$): 0.15 (6H, s), 0.19 (6H, s), 0.97 (9H, s), 0.98 (9H, s), 6.17 (1H, dd, J = 8.5 & 2.7 Hz), 6.23 (1H, d, J = 2.7 Hz), 6.63 (1H, d, J = 8.5 Hz).

(c) To a solution of 1-amino-3,4-di(t-butyldimethylsilyloxy)benzene (354 mg) in dry tetrahydrofuran (1.5 ml), a 1.55M solution of n-butyl lithium in hexane (0.71 ml) was added under nitrogen stream while ice-cooling. The resultant mixture was stirred for 15 minutes, and bromoacetyl bromide (0.087 ml) was added thereto at the same temperature, followed by stirring for 30 minutes. The reaction mixture was treated with an aqueous solution of sodium hydrogen carbonate (42 mg) and extracted with ethyl acetate. The extract was washed with an aqueous sodium chloride solution, dried over magnesium sulfate and concentrated. The residue was chromatographed on silica gel column to give 1-bromoacetylamino-3,4-di-(t-butyldimethylsilyloxy)benzene.

$IR_{max}$ $cm^{-1}$ (neat): 3280, 1655, 1607, 1507, 1420, 1405, 1294, 1250, 1225, 1206, 1120.

NMR δ ppm ($CDCl_3$): 0.18 (6H, s), 0.23 (6H, s), 0.98 (9H, s), 0.99 (9H, s), 4.00 (2H, s), 6.78 (1H, d, J = 8.6 Hz), 6.83 (1H, dd, J = 2.3 & 8.6 Hz), 7.25 (1H, d, J = 2.3 Hz), 7.95 (1H, br. s).

Reference Example 9

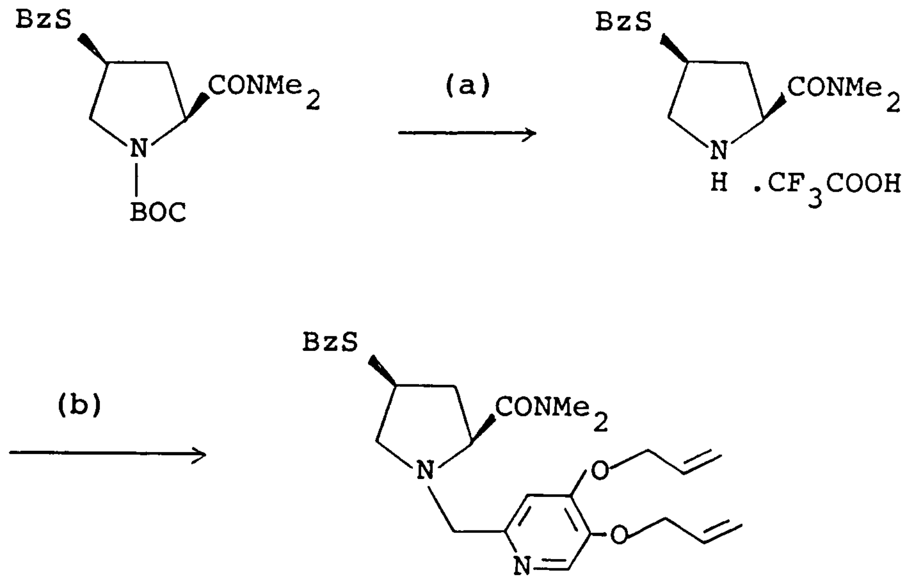


(a) A trifluoroacetic acid solution (36 ml) of anisole (4.32 g) was dropwise added under ice-cooling to (2S,4S)-1-t-butoxycarbonyl-2-dimethylaminocarbonyl-4-benzoylthiopyrrolidine (7.56 g), and the mixture was stirred at room temperature for 5.5 hours. The solvent was removed by distillation under reduced pressure, and the residue was combined with diethyl ether for crystallization. The crystals were collected by filtration and dried under reduced pressure to give (2S,4S)-2-dimethylaminocarbonyl-4-benzoylthiopyrrolidine trifluoroacetate (5.82 g).

$IR_{max}$ $cm^{-1}$ (KBr): 3060 (br), 1668, 1450, 1424, 1199, 1179, 1132.

NMR $\delta$ ppm ($CDCl_3$): 1.95 - 2.15 (1H, m), 3.06 (3H, s), 3.07 (3H, s), 3.35 - 3.55 (1H, m), 3.95 - 4.15 (1H, m), 4.38 (1H, m), 5.04 (1H, t, J = 8.4 Hz), 7.47 (2H, m), 7.62 (1H, m), 7.90 (2H, m).

(b) (2S,4S)-2-Dimethylaminocarbonyl-4-benzoylthiopyrrolidine trifluoroacetate (78 mg) and 4,5-diallyloxy-2-chloromethylpyridine (180 mg) were dissolved in dry methylene chloride (2 ml), and triethyamine (61 mg) was added thereto in nitrogen stream under ice-cooling. The resultant mixture was allowed to react at room temperature for 4 days. The reaction mixture wss washed with water, dried over sodium sulfate and concentrated. The residue was chromatographed on silica gel thin layer to give (2S,4S)-1-(4,5-diallyloxy-2-pyridyl)methyl-2-dimethylaminocarbonyl-4-benzoylthiopyrrolidine (212 mg).

$IR_{max}$ $cm^{-1}$ (neat): 1655, 1588, 1580, 1510, 1445, 1395, 1322, 1242, 1207, 1172.

NMR $\delta$ ppm ($CDCl_3$): 2.04 (1H, m), 2.7 - 3.3 (3H, m), 2.93 (3H, s), 3.01 (3H, s), 3.5 - 3.9 (3H, m), 4.22 (1H, m), 4.5 - 4.8 (4H, m), 5.2 - 5.5 (4H, m), 5.95 - 6.15 (2H, m), 7.20 (1H, s), 7.35 - 7.65 (3H, m), 7.91 (2H, m), 8.04 (1H, s).

Reference Examples 10 to 22

In the same manner as in Reference Example 9, the compounds as shown in Tables 8 and 9 were produced using the corresponding alkylating agents.

Table 8

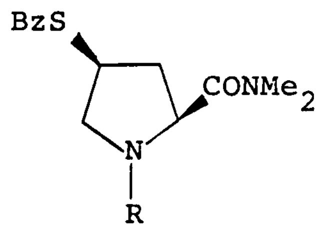

| Reference Example No. | R |
|---|---|
| 10 | 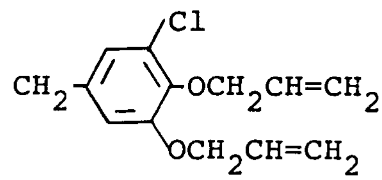 |
| 11 | 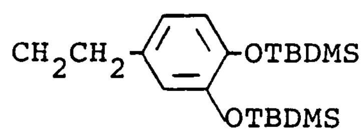 |
| 12 | 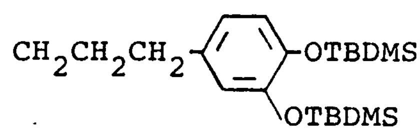 |
| 13 | 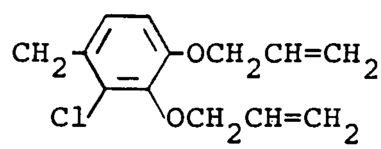 |

14 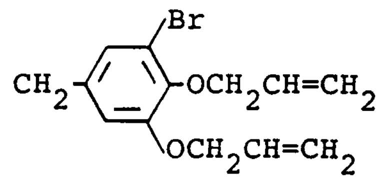

15 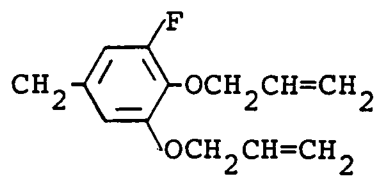

16 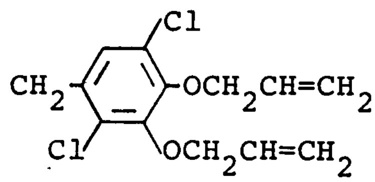

17 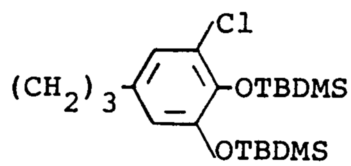

18 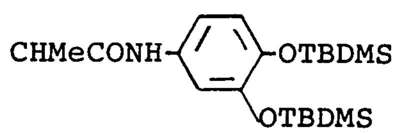

Physical property

Reference Example 10

| | |
|---|---|
| $IR_{max}$ $cm^{-1}$ (neat): | 1644, 1475, 1409, 1264, 1197. |
| NMR δ ppm ($CDCl_3$): | 2.01 (1H, dt, J = 13.0 & 6.0 Hz), 2.80 (1H, dt, J = 13.0 & 8.6 Hz), 2.93 (3H, s), 2.97 (3H, s), 3.02 (1H, dd, J = 9.9 & 7.3 Hz), 3.15 (1H, dd, J = 9.9 & 4.6 Hz), 3.47 (1H, d, J = 12.2 Hz), 3.65 (1H, dd, J = 8.6 & 6.0 Hz), 3.87 (1H, d, J = 12.2 Hz), 4.20 (1H, m), 4.57 (4H, m), 5.30 (4H, m), 6.08 (2H, m), 6.91 (2H, s), 7.43 (2H, m), 7.56 (1H, m), 7.91 (2H, m). |

Reference Example 11

| | |
|---|---|
| $IR_{max}$ $cm^{-1}$ (neat): | 1654, 1647 (sh), 1507, 1292, 1250, 1206. |
| NMR δ ppm ($CDCl_3$): | 0.17 (12H, s), 0.97 (18H, s), 2.00 (1H, m), 2.51 - 2.80 (4H, m), 2.85 - 3.06 (2H, m), 2.94 (3H, s), 3.04 (3H, s), 3.31 (1H, m), 3.56 (1H, m), 4.22 (1H, m), 6.59 - 6.65 (2H, m), 6.71 (1H, d, J = 7.9 Hz), 7.44 (2H, m), 7.57 (1H, m), 7.94 (2H, m). |

Reference Example 12

| | |
|---|---|
| $IR_{max}$ $cm^{-1}$ (neat): | 1658, 1640 (sh), 1511, 1292, 1253, 1208. |
| NMR δ ppm ($CDCl_3$): | 0.17 (12H, s), 0.97 (18H, s), 1.77 (2H, m), 1.99 (1H, m), 2.30 - 2.63 (3H, m), |

2.73 (2H, m), 2.92 (1H, m), 2.96 (3H, s), 3.11 (3H, s), 3.24 (1H, m), 3.64 (1H, m), 4.20 (1H, m), 6.56 - 6.63 (2H, m), 6.71 (1H, d, J = 7.9 Hz), 7.40 - 7.46 (2H, m), 7.56 (1H, m), 7.94 (2H, m).

Reference Example 13

IR$_{max}$ cm$^{-1}$ (neat): 1651, 1487, 1281, 1209, 1026.
NMR δ ppm ($CDCl_3$): 2.04 (1H, m), 2.83 (1H, m), 2.91 (3H, s), 3.02 (3H, s), 3.07 (1H, dd, J = 10.3 & 7.3 Hz), 3.18 (1H, dd, J = 10.3 & 4.6 Hz), 3.72 (1H, t, J = 7.5 Hz), 3.81 (1H, d, J = 13.2 Hz), 3.89 (1H, d, J = 13.2 Hz), 4.21 (1H, m), 4.56 (4H, m), 5.33 (4H, m), 6.12 (2H, m), 6.81 (1H, d, J = 8.6 Hz), 7.19 (1H, d, J = 8.6 Hz), 7.43 (2H, t, J = 7.5 Hz), 7.56 (1H, t, J = 7.5 Hz), 7.93 (2H, d, J = 7.5 Hz).

Reference Example 14

IR$_{max}$ cm$^{-1}$ (neat): 1656, 1481, 1418, 1270, 1206.
NMR δ ppm ($CDCl_3$): 2.02 (1H, m), 2.80 (1H, m), 2.93 (3H, s), 2.96 (3H, s), 3.03 (1H, dd, J = 10.2 & 7.3 Hz), 3.16 (1H, dd, J = 10.2 & 4.9 Hz), 3.48 (1H, d, J = 13.2 Hz), 3.64 (1H, t, J = 6.9 Hz), 3.87 (1H, d, J = 13.2 Hz), 4.22 (1H, m), 4.56 (4H, m), 5.32 (4H, m), 6.12 (2H, m), 6.95 (1H, d, J = 2.0 Hz), 7.06 (1H, d, J = 2.0 Hz), 7.43 (2H, t, J = 7.3 Hz), 7.56 (1H, t, J = 7.3 Hz), 7.92 (2H, d, J = 7.3 Hz).

Reference Example 15

IR$_{max}$ cm$^{-1}$ (neat): 1658, 1506, 1439, 1333, 1210, 1082.
NMR δ ppm ($CDCl_3$): 2.01 (1H, m), 2.80 (1H, m), 2.93 (3H, s), 2.98 (3H, s), 3.02 (1H, dd, J = 9.9 & 7.3 Hz), 3.14 (1H, dd, J = 9.9 & 5.0 Hz), 3.46 (1H, d, J = 13.5 Hz), 3.66 (1H, t, J = 7.5 Hz), 3.88 (1H, d, J = 13.5 Hz), 4.21 (1H, m), 4.57 (4H, m), 5.32 (4H, m), 6.07 (2H, m), 6.70 (1H, dd, J = 11.9 & 2.0 Hz), 6.76 (1H, s), 7.43 (2H, br. t, J = 7.6 Hz), 7.56 (1H, br. t, J = 7.6 Hz), 7.92 (2H, d, J = 7.6 Hz).

Reference Example 16

IR$_{max}$ cm$^{-1}$ (neat): 1659, 1448, 1409, 1352, 1289, 1209, 1175.
NMR δ ppm ($CDCl_3$): 2.03 (1H, m), 2.75 - 3.30 (3H, m), 2.95 (3H, s), 3.02 (3H, s), 3.7 - 4.0 (3H, m), 4.23 (1H, m), 4.56 (4H, m), 5.2 - 5.5 (4H, m), 6.0 - 6.2 (2H, m), 7.35 - 7.65 (4H, m), 7.9 - 8.0 (2H, m).

Reference Example 17

IR$_{max}$ cm$^{-1}$ (neat): 1660, 1560, 1482, 1419, 1309, 1254, 1208.
NMR δ ppm ($CDCl_3$): 0.17 (6H, s), 0.18 (6H, s), 0.95 (9H, s), 1.03 (9H, s), 1.78 (2H, m), 2.00 (1H, m), 2.48 (3H, m), 2.77 (2H, m), 2.93 (1H, m), 2.97 (3H, s), 3.12 (3H, s), 3.23 (1H, dd, J = 10.2 & 4.0 Hz), 3.51 (1H, m), 4.22 (1H, m), 6.53 (1H, d, J = 2.0 Hz), 6.75 (1H, d, J = 2.0 Hz), 7.43 (2H, d, J = 7.3 Hz), 7.56 (1H, t, J = 7.3 Hz), 7.93 (2H, d, J = 7.3 Hz).

Reference Example 18

IR$_{max}$ cm$^{-1}$ (neat): 3230, 1651, 1600, 1508, 1312, 1273 (sh), 1249, 1220 (sh), 1207, 1172, 1123.
NMR δ ppm ($CDCl_3$): 0.14, 0.15 and 0.16 (6H in total, each s), 0.22, 0.23 and 0.24 (6H in total, each s), 0.97, 0.98 and 0.99 (18H in total, each s), 1.30 and 1.34 (3H in total, each d, J = 6.9 & 7.3 Hz), 2.92 (1H, m), 2.97, 2.98, 3.02 and 3.04 (6H in total, each s), 3.2 - 3.9 (3H, m), 4.22 (1H, m), 6.6 - 7.3 (3H, m).

## Table 9

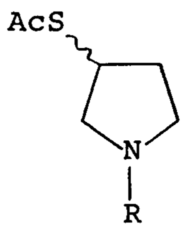

| Reference Example No. | R |
|---|---|
| 19 | 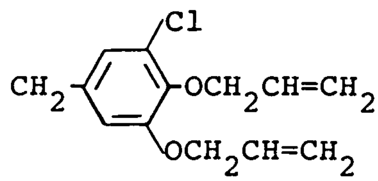 |
| 20 | 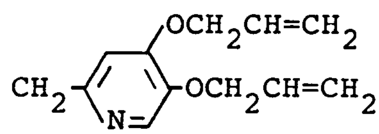 |
| 21 | 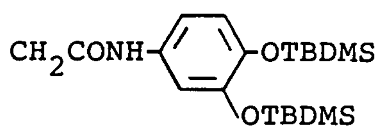 |
| 22 | 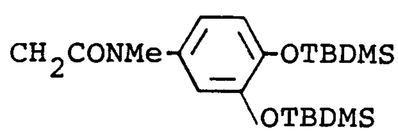 |

Physical property

Reference Example 19

| | |
|---|---|
| $IR_{max}$ $cm^{-1}$ (neat): | 1680, 1509, 1478, 1413, 1264, 1127. |
| NMR δ ppm ($CDCl_3$): | 1.76 (1H, m), 2.29 (3H, s), 2.40 (1H, m), 2.52 (2H, m), 2.65 (1H, m), 2.94 (1H, dd, J = 9.9 & 7,3 Hz), 3.49 (1H, d, J = 13.2 Hz), 3.53 (1H, d, J = 13.2 Hz), 3.92 (1H, m), 4.56 (4H, m), 5.33 (4H, m), 6.09 (2H, m), 6.81 (1H, d, J = 2.0 Hz), 6.91 (1H, d, J = 2.0 Hz). |

Reference Example 20

| | |
|---|---|
| $IR_{max}$ $cm^{-1}$ (neat): | 1684, 1584, 1508, 1318, 1240, 1220, 1160, 1132. |
| NMR δ ppm ($CDCl_3$): | 1.80 (1H, m), 2.30 (3H, s), 2.3 - 2.8 (4H, m), 3.02 (1H, dd, J = 7.3 & 9.9 Hz), 3.68 (2H, m), 3.96 (1H, m), 4.55 - 4.75 (4H, m), 5.25 - 5.55 (4H, m), 6.07 (2H, m), 6.97 (1H, s), 8.06 (1H, s). |

Reference Example 21

IR$_{max}$ cm$^{-1}$ (neat): 3300, 1688, 1588, 1512, 1310, 1273, 1252, 1223, 1124.
NMR δ ppm ($CDCl_3$): 0.18 (6H, s), 0.24 (6H, s), 0.98 (9H, s), 1.00 (9H, s), 1.84 (1H, m), 2.33 (3H, s), 2.43 (1H, m), 2.69 (2H, m), 2.98 (2H, m), 3.20 (1H, d, J = 16.5 Hz), 3.33 (1H, d, J = 16.5 Hz), 3.99 (1H, m), 6.77 (1H, d, J = 8.6 Hz), 6.87 (1H, dd, J = 2.6 & 8.6 Hz), 7.35 (1H, d, J = 2.6 Hz), 8.85 (1H, br. s).

Reference Example 22

IR$_{max}$ cm$^{-1}$ (neat): 1684, 1665 (sh), 1502, 1291, 1250, 1206, 1112.
NMR δ ppm ($CDCl_3$): 0.20 (6H, s), 0.22 (6H, s), 0.98 (9H, s), 1.00 (9H, s), 1.72 (2H, m), 2.28 (3H, s), 2.33 (1H, m), 2.54 - 2.75 (3H, m), 3.08 (2H, m), 3.20 (3H, s), 3.91 (1H, m), 6.62 - 6.65 (2H, m), 6.82 (1H dd, J = 1.7 & 6.9 Hz).

Reference Example 23

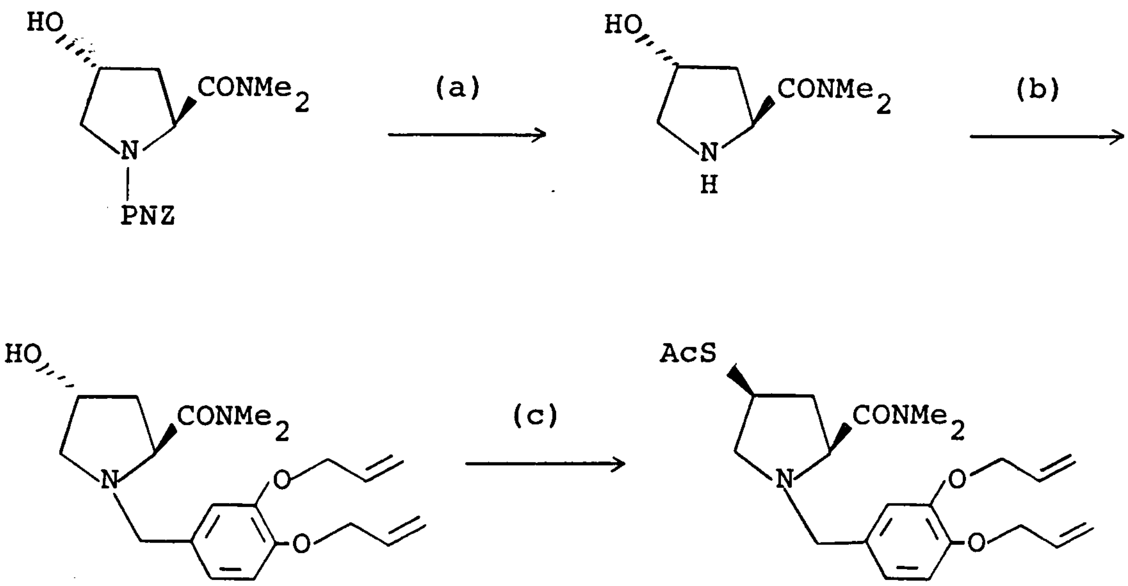


(a) (2S,4R)-1-p-Nitrobenzyloxycarbonyl-2-dimethylaminocarbonyl-4-hydroxypyrrolidine (500 mg) was dissolved in ethyl acetate (30 ml), and 10 % palladium-carbon (500 mg) was added thereto. Catalytic hydrogenation was performed at room temperature under atmospheric pressure for 30 minutes. The catalyst was removed by filtration, and the filtrate was concentrated under reduced pressure. The residue was crystallized from benzene, and the crystals were collected by filtration to give (2S,4R)-2-dimethylaminocarbonyl-4-hydroxypyrrolidine (195 mg).

IR$_{max}$ cm$^{-1}$ (KBr): 3279, 1636, 1387, 1102.

NMR δ ppm ($CDCl_3$): 1.84 (1H, m), 2.14 (1H, m), 2.89 (1H, d, J = 12.9 Hz), 2.98 (3H, s), 3.04 (3H, s), 3.31 (1H, dd, J = 4.6 & 11.6 Hz), 4.15 (1H, t, J = 8.1 Hz), 4.45 (1H, m).

(b) (2S,4R)-2-Dimethylaminocarbonyl-4-hydroxypyrrolidine (100 mg) and 3,4-diallyloxybenzaldehyde (137 mg) were dissolved in methanol (0.5 ml), and the resultant mixture was stirred at room temperature for 30 minutes. To the reaction mixture, a methanolic solution (0.5 ml) of sodium cyanoborohydride (13 mg) was added under ice-cooling, and the mixture was stirred under ice-cooling for 1 hour and at room temperature for 1 hour. The reaction mixture was diluted with benzene and extracted with a 2.5 % aqueous potassium phosphate solution. The aqueous layer was adjusted to pH 10 with 1N sodium hydroxide, extracted with methylene chloride and dried. Removal of the solvent gave (2S,4R)-1-(3,4-diallyloxybenzyl)-2-dimethylaminocarbonyl-4-hydroxypyrrolidine (86 mg).

NMR δ ppm ($CDCl_3$): 2.61 (1H, dd, J = 3.2 & 10.1 Hz), 2.83 (3H, s), 2.90 (3H, s), 3.46 (1H, dd, J = 5.4 & 10.1 Hz), 3.55 - 4.00 (3H, m), 4.45 - 4.75 (5H, m), 5.2 - 5.5 (4H, m), 6.0 - 6.2 (2H, m), 6.82 (2H, s), 6.95 (1H, s).

(c) To a solution of (2S,4R)-1-(3,4-diallyloxybenzyl)-2-dimethylaminocarbonyl-4-hydroxypyrrolidine (226

mg) and triphenylphosphine (247 mg) in dry tetrahydrofuran (0.5 ml), a solution of diethyl azodicarboxylate (164 mg) in dry tetrahydrofuran (0.2 ml) was dropwise added under nitrogen stream while ice-cooling, followed by stirring for 0.5 hour. A solution of thioacetic acid (72 mg) in dry tetrahydrofuran (0.2 ml) was dropwise added thereto, and the resultant mixture was stirred at room temperature for 1 hour. The reaction mixture was diluted with ethyl acetate, and an aqueous solution of sodium hydrogen carbonate (79 mg) was added thereto for extraction. The organic layer was washed with an aqueous sodium chloride solution and dried over sodium sulfate and concentrated. The residue was chromatographed on silica gel thin layer to give (2S,4S)-1-(3,4-diallyloxybenzyl)-2-dimethylaminocarbonyl-4-acetylthiopyrrolidine (108 mg).

$IR_{max}$ $cm^{-1}$ (neat): 1684, 1641, 1508, 1422, 1259, 1221, 1134, 1120 (sh).

NMR δ ppm ($CDCl_3$): 1.60 - 1.94 (2H, m), 2.28 (3H, s), 2.66 (1H, m), 2.91 (3H, s), 2.92 (3H, s), 2.98 (1H, m), 3.47 (1H, d, J = 13.1 Hz), 3.59 (1H, m), 3.84 (1H, d, J = 13.1 Hz), 3.99 (1H, m), 4.57 - 4.63 (4H, m), 5.24 - 5.30 (2H, m), 5.36 - 5.47 (2H, m), 6.00 - 6.17 (2H, m), 6.75 - 6.99 (3H, m).

Reference Examples 24 and 25

In the same manner as in Reference Example 23, the compounds as shown in Table 10 were produced using 3-hydroxypyrrolidine.

Table 10

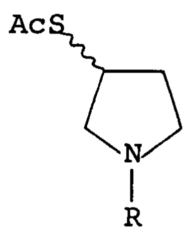

| Reference Example No. | R |
|---|---|
| 24 | 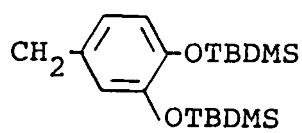 |
| 25 | 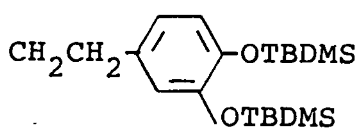 |

Physical property

Reference Example 24

$IR_{max}$ $cm^{-1}$ (neat): 1678, 1500, 1450, 1410, 1286, 1242, 1208, 1154.

NMR δ ppm ($CDCl_3$): 0.19 (12H, s), 0.98 (9H, s), 0.99 (9H, s), 1.73 (1H, m), 2.28 (3H, s), 2.3 - 2.7 (4H, m), 2.92 (1H, dd, J = 7.3 & 9.9 Hz), 3.49 (2H, q, J = 12.8 Hz), 3.91 (1H, m), 6.65 - 6.85 (3H, m).

Reference Example 25

$IR_{max}$ $cm^{-1}$ (neat): 1685, 1506, 1454, 1416, 1289, 1250, 1220, 1125.

NMR δ ppm ($CDCl_3$): 0.18 (6H, s), 0.18 (6H, s), 0.97 (9H, s), 0.98 (9H, s), 1.76 (1H, m), 2.31 (3H, s), 2.36 (1H, m), 2.51 - 2.78 (7H, m), 3.01 (1H, m), 3.94 (1H, m), 6.59 - 6.74 (3H, m).

Reference Example 26 to 28

In the same manner as in Reference Example 9 or 23, the compounds as shown in Table 11 were produced.

Table 11

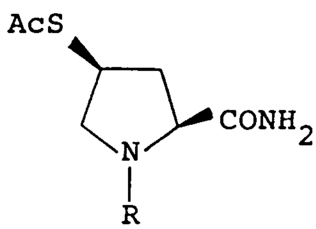

| Reference Example No. | R |
|---|---|
| 26 | 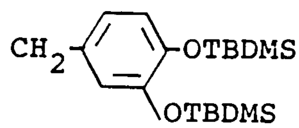 |
| 27 | 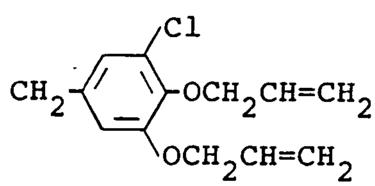 |
| 28 | 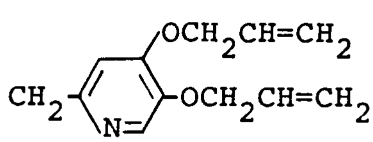 |

Physical property

Reference Example 26

$IR_{max}$ $cm^{-1}$ (neat): 1680, 1505, 1287, 1242, 1210.

NMR δ ppm ($CDCl_3$): 0.19 (3H, s), 0.20 (6H, s), 0.20 (3H, s), 0.98 (9H, s), 0.99 (9H, s), 2.28 (3H, s), 2.75 - 3.05 (3H, m), 3.23 (1H, dd, J = 5.5 & 10.4 Hz), 3.61 (2H, m), 3.98 (1H, m), 6.65 - 6.85 (3H, m).

Reference Example 27

$IR_{max}$ $cm^{-1}$ (neat): 3442, 3188, 1684, 1575, 1488, 1421, 1277, 1135, 1036.

NMR δ ppm ($CDCl_3$): 1.98 (1H, m), 2.84 (2H, m), 2.98 (1H, dd, J = 10.6 & 1.7 Hz), 3.24 (1H, dd, J = 9.9 & 6.9 Hz), 3.41 (1H, d, J = 13.5 Hz), 3.87 (1H, d, J = 13.5 Hz), 3.99 (1H, m), 4.57 (4H, m), 5.38 (4H, m), 6,13 (2H, m), 6.71 (1H, d, J = 2.0 Hz), 6.90 (1H, d, J = 2.0 Hz).

Reference Example 28

$IR_{max}$ $cm^{-1}$ (neat): 3338 (br), 1685, 1589, 1508, 1319, 1229.
NMR δ ppm ($CDCl_3$): 2.02 (1H, m), 2.29 (3H, s), 2.7 - 3.1 (3H, m), 3.38 (1H, m), 3.5 - 4.1 (4H, m), 4.55 - 4.80 (4H, m), 5.25 - 5.55 (4H, m), 5.77 (1H, br. s), 5.95 - 6.2 (2H, m), 6.76 (1H, s), 7.80 (1H, br. s), 8.11 (1H, s).

Reference Example 29

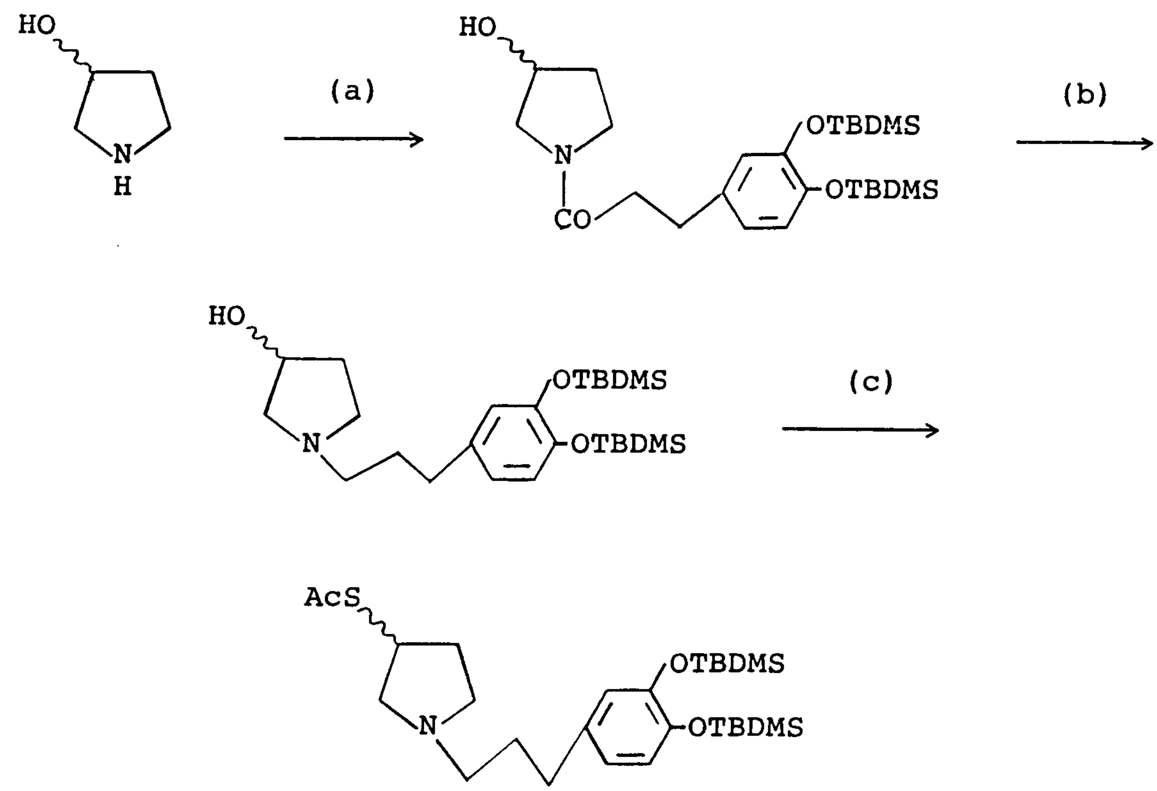


(a) To a solution of 3,4-di(t-butyldimethylsilyloxy)phenylpropionic acid (2.054 g) in dry tetrahydrofuran (20 ml), triethylamine (607 mg) and ethyl chloroformate (651 mg) were added in nitrogen stream while ice-cooling, followed by stirring for 1 hour. A solution of 3-hydroxypyrrolidine (436 mg) in dry tetrahydrofuran (5 ml) was added thereto, and stirring was continued for 1 hour. The reaction mixture was diluted with ethyl acetate, washed with an aqueous sodium chloride solution and dried over magnesium sulfate. After removal of the solvent by distillation, the residue was chromatographed on silica gel column to give 1-[3,4-di(t-butyldimethylsilyloxy)phenylpropionyl]-3-hydroxypyrrolidine(1.93 g).

$IR_{max}$ $cm^{-1}$ (neat): 3390 (br), 1620, 1508, 1459, 1421, 1294, 1252, 1226, 1158, 1122.

NMR δ ppm ($CDCl_3$): 0.18 (6H, s), 0.18 (6H, s), 0.98 (9H, s), 0.98 (9H, s), 1.95 (2H, m), 2.52 (2H, m), 2.85 (2H, t, J = 7.4 Hz), 3.2 - 3.7 (4H, m), 4.47 (1H, m), 6.64 (1H, d, J = 7.9 Hz), 6.68 (1H, s), 6.73 (1H, d, J = 7.9 Hz).

(b) To a suspension of lithium aluminum hydride (394 mg) in dry tetrahydrofuran (25 ml), a solution of 1-[3,4-di(t-butyldimethylsilyloxy)phenylpropionyl]-3-hydroxypyrrolidine(1.66 g) in dry tetrahydrofuran (25 ml) was dropwise added under nitrogen stream while ice-cooling. The resultant mixture was heated under reflux for 1 hour, cooled to room temperature and combined with water (3.36 ml) to decompose excess of the reducing agent. After addition of sodium sulfate (1.0 g), the resultant mixture was stirred for 15 minutes. Insoluble materials were removed by filtration, and the solvent was removed by distillation. The residue was chromatographed on silica gel column to give 1-[3,4-di(t-butyldimethylsilyloxy)-phenylpropyl]-3-hydroxypyrrolidine (736 mg).

$IR_{max}$ $cm^{-1}$ (neat): 3350 (br), 1509, 1471, 1460, 1420, 1290, 1250, 1222, 1157, 1127.

NMR δ ppm ($CDCl_3$): 0.18 (6H, s), 0.19 (6H, s), 0.98 (9H, s), 0.98 (9H, s), 1.7 - 1.9 (4H, m), 2.12 - 2.35 (2H, m), 2.45 - 2.56 (4H, m), 2.74 (1H, m), 2.93 (1H, m), 4.33 (1H, m), 6.60 (1H, dd, J = 2.0 & 7.9

Hz), 6.64 (1H, d, J = 2.0 Hz), 6.73 (1H, d, J = 7.9 Hz).

(c) To a solution of 1-[3,4-di(t-butyldimethylsilyloxy)phenylpropyl]-3-hydroxypyrrolidine (844 mg) in dry methylene chloride (17 ml), triethylamine (275 mg) and methenesulfonyl chloride (249 mg) were added in nitrogen stream while ice-cooling, followed by stirring for 1 hour. The reaction mixture was diluted with ethyl acetate, washed with water and an aqueous sodium chloride solution in order and dried over sodium sulfate, followed concentration. Potassium thioacetate (620 mg) was added to the residue, and the resultant mixture was stirred at 60 °C for 2 hours in dry dimethylformamide (14 ml). The reaction mixture was diluted with ethyl acetate, washed with water and an aqueous sodium chloride solution and dried over sodium sulfate. After removal of the solvent, the residue was chromatographed on silica gel column to give 1-[3,4-di(t-butyldimethylsilyloxy)phenylpropyl]-3-acetylthiopyrrolidine (455 mg).

$IR_{max}$ $cm^{-1}$ (neat): 1688, 1508, 1460, 1420, 1291, 1252, 1221.

NMR δ ppm ($CDCl_3$): 0.18 (6H, s), 0.18 (6H, s), 0.98 (9H, s), 0.98 (9H, s), 1.6 - 1.81 (3H, m), 2.30 (3H, s), 2.3 - 2.6 (6H, m), 2.64 (1H, m), 2.92 (1H, m), 3.92 (1H, m), 6.57 - 6.64 (2H, m), 6.72 (1H, d, J = 7.9 Hz).

## Claims

**1.** A compound of the formula:

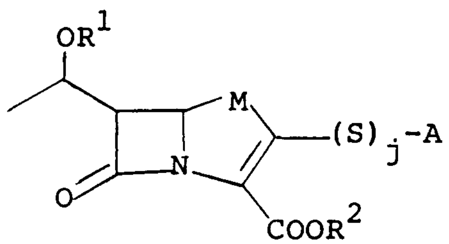

(I)

wherein:

j is an integer of 0 or 1;

$R^1$ is a hydrogen atom, a protective group for hydroxyl or either one of the below-mentioned groups (1) to (3);

$R^2$ is a hydrogen atom, a protective group for carboxyl or a negative charge when A has any moiety with a positive charge;

M is a sulfur atom, a methylene group or a methylene group substituted with lower alkyl; and

A varies depending on $R^1$ and represents the followings:

(i) when $R^1$ is a hydrogen atom or a protective group for hydroxyl, A is either one of the groups (a) to (g):

(a)

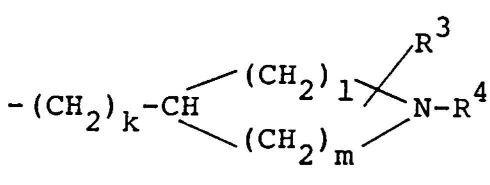

wherein k, l and m are each an integer of 0 to 3 but l and m are not simultaneously 0, $R^3$ is a hydrogen atom, a lower alkoxycarbonyl group, a carbamoyl group, a mono- or di(lower)-alkylaminocarbonyl group, a cyclic aminocarbonyl group, a lower alkyl group or a substituted lower alkyl group and $R^4$ is either one of the groups (1) to (3):

(1)

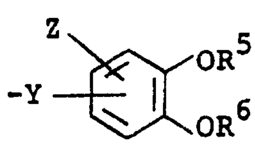

wherein y is a single bond, a lower alkylene group, a lower alkenylene group, a carbonyl group, a lower alkylene group having at least one linkage chosen from -CO-, -O- and -$NR^0$- (in which $R^0$ is a hydrogen atom or a lower alkyl group) in the alkylene chain or a lower alkenylene group having at least one linkage chosen from -CO-, -O- and -$NR^0$- (in which $R^0$ is as defined above) in the alkenylene chain, $R^5$ and $R^6$ are each a hydrogen atom or a protective group for hydroxyl, Z is a hydrogen atom, a halogen atom, a nitro group, a cyano group, a lower alkoxycarbonyl group, a carbamoyl group or a mono- or di(lower)alkylaminocarbonyl group,

(2)

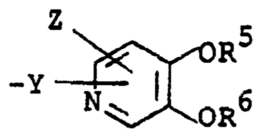

wherein Y, Z, $R^5$ and $R^6$ are each as defined above, and

(3)

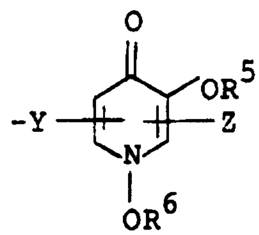

wherein Y, Z, $R^5$ and $R^6$ are each as defined above;
(b)

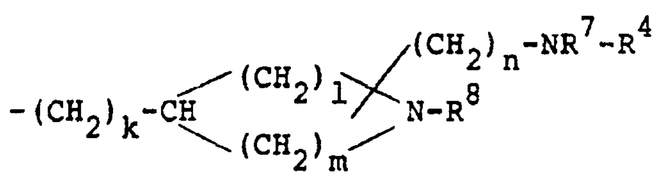

wherein k, l, m and $R^4$ are each as defined above, n is an integer of 0 to 3, $R^7$ is a hydrogen atom, a lower alkyl group or a substituted lower alkyl group and $R^8$ is a hydrogen atom, a protective group for amino, a lower alkyl group or a substituted lower alkyl group;
(c)

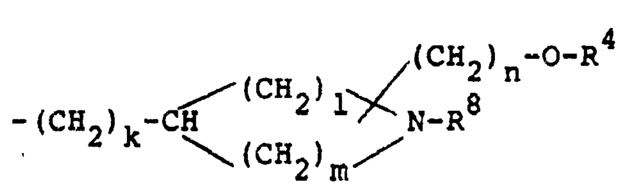

wherein k, l, m, n, $R^4$ and $R^8$ are each as defined above;
(d)

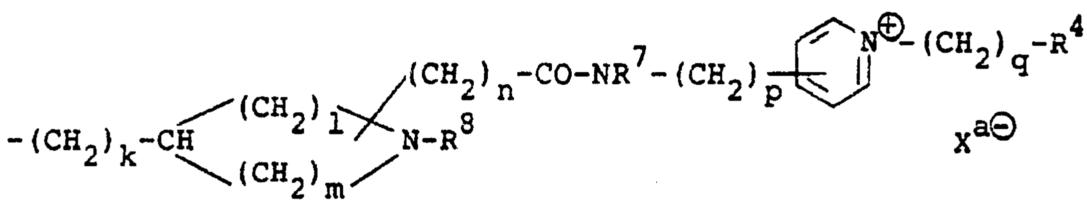

wherein k, l, m, n, $R^4$, $R^7$ and $R^8$ are each as defined above and p is an integer of 0 to 5, q is an integer of 1 to 5 and $Y^a$ is an acid residue or an intramolecular COO when $R^2$ is a negative charge;
(e)

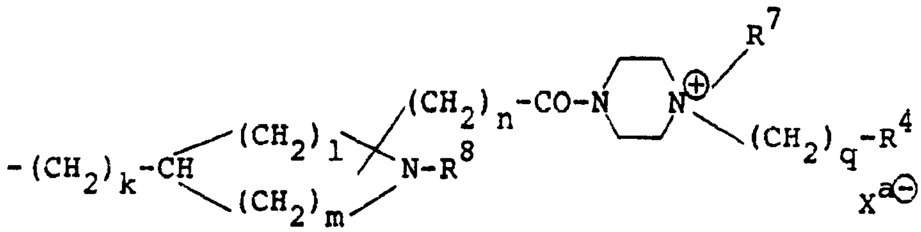

wherein k, l, m, n, q, $R^4$, $R^7$, $R^8$ and $X^a$ are each as defined above;
(f)

$-(CH_2)_k-O-R^4$

wherein k and $R^4$ are each as defined above;
(g)

$-(CH_2)_k-NR^7-R^4$

wherein k, $R^4$ and $R^7$ are each as defined above;
(ii) when $R^1$ is either one of the groups (1) to (3), A is either one of the groups (h) to (p) or any other organic group:
(h)

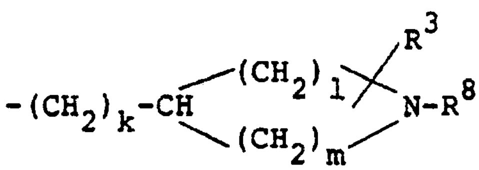

wherein k, l, m, $R^3$ and $R^8$ are each as defined above;
(i)

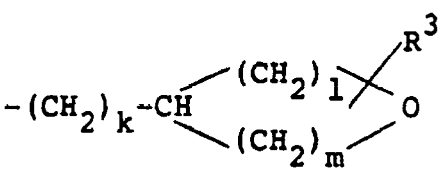

wherein k, l, m and $R^3$ are each as defined above;
(j)

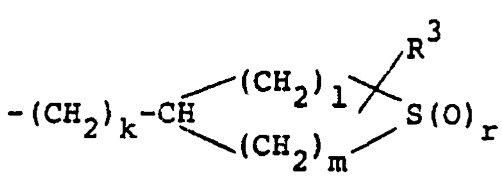

wherein k, l, m and $R^3$ are each as defined above and r is an integer of 0 to 2;

(k)

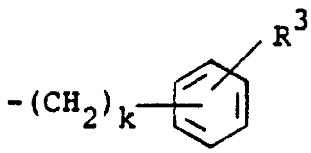

wherein k and $R^3$ are each as defined above;
(l)

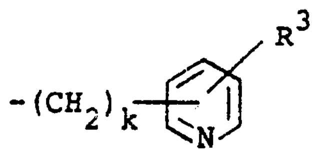

wherein k and $R^3$ are each as defined above;
(m)

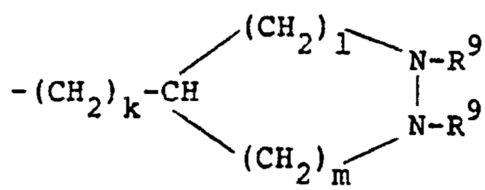

wherein k, l and m are each as defined above and $R^9$ is a hydrogen atom or a protective group for amino;
(n)

$-(CH_2)_k-R^{10}$

wherein k is as defined above and $R^{10}$ is a lower alkyl group or a substituted lower alkyl group;
(o)

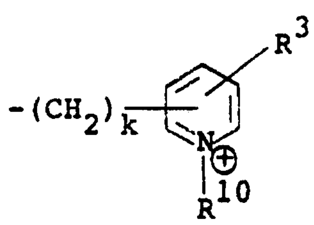

wherein k, $R^3$ and $R^{10}$ are each as defined above; and
(p)

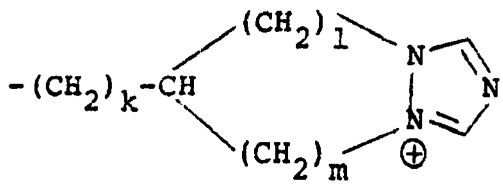

wherein k, l and m are each as defined above, and its salts.

**2.** The compound according to claim 1, wherein $R^2$ is a hydrogen atom or a negative charge in case of A comprising a positive charge, and a salt thereof.

**3.** The compound according to claim 2, wherein $R^1$ is a hydrogen atom and A is either one of the groups (a) to (e), and a salt thereof.

**4.** The compound according to claim 2, wherein $R^1$ is either one of the groups (1) to (3), and a salt thereof.

**5.** The compound according to claim 3 or 4, wherein A comprises a pyrrolidine ring, and a salt thereof.

**6.** The compound according to any one of claims 1-5, wherein M is a methylene group substituted with lower alkyl.

**7.** The compound according to claim 6 which has a (5R)-configuration in case of j = 0 or a (5S)-configuration in case of j = 1.

**8.** The compound according to any one of claims 1-5, wherein M is a sulfur atom or a methylene group and which has a (5R)-configuration.

**9.** The compound according to claim 7 which has a (6S,8R)-configuration.

**10.** The compound according to claim 8 which has a (5R,6S,8R)-configuration.

**11.** The compound according to claim 5, wherein k is 0, and a salt thereof.

**12.** The compound according to claim 3, wherein A is either one of the groups (b) to (e) and Y is a carbonyl group, and a salt thereof.

**13.** A process for preparing the compound (I) according to claim 1, or its salts, which comprises:
(A) reacting a compound of the formula:

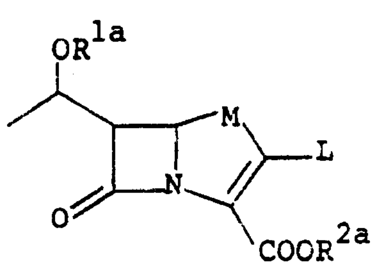

(II)

wherein M is as defined above, $R^{1a}$ is a hydrogen atom or a protective group for hydroxyl, $R^{2a}$ is a protective group for carboxyl and L is a reactive ester group of hydroxyl or a substituted or unsubstituted lower alkylsulfinyl group with a mercaptan compound of the formula:

$A^a$-SH (III-a)

wherein $A^a$ is either one of the groups (a) to (c) to give a compound of the formula:

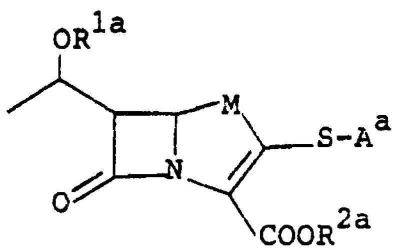

(I-a)

wherein $R^{1a}$, $R^{2a}$, $A^a$ and M are each as defined above, or reacting the compound (II) with a compound of the formula:

$Q^1$-SH (III-b-1)

wherein $Q^1$ is the group (h) to give a compound of the formula:

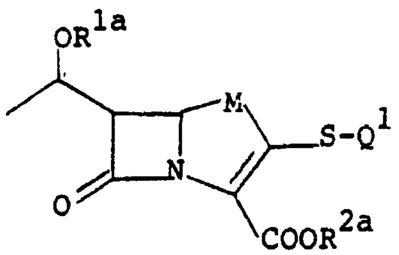

(IV-a)

wherein $R^{1a}$, $R^{2a}$, $Q^1$ and M are each as defined above and, in case of $R^8$ in the group (h) being a protective group for amino, then eliminating the protecting group and reacting the resultant compound with a compound of the formula:

$R^4$-$X^c$ (V)

wherein $R^4$ is as defined above and $X^c$ is an acid residue or an isocyanate group to give the compound (I-a);
(B) reacting the compound (II) with a mercaptan compound of the formula:

$Q^2$-SH (III-b-2)

wherein $Q^2$ is either one of the groups (d') and (e') of the formulas:

(d')

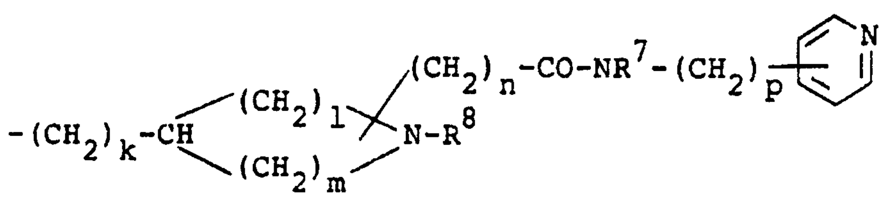

wherein k, l, m, n, p, $R^7$ and $R^8$ are each as defined above and

(e')

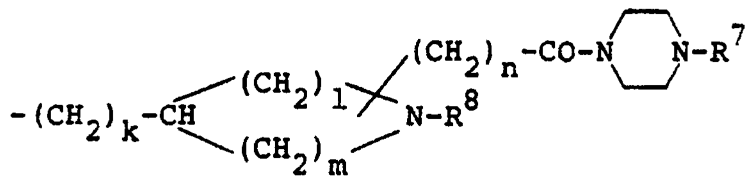

wherein k, l, m, n, $R^7$ and $R^8$ are each as defined and reacting the resultant compound of the formula:

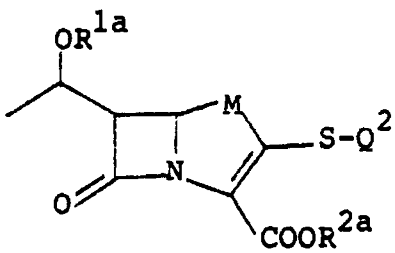

(IV-b)

wherein $R^{1a}$, $R^{2a}$, $Q^2$ and M are each as defined above with a compound of the formula:

$X^b$-$(CH_2)_q$-$R^4$ (VI)

wherein q and $R^4$ are each as defined above and $X^b$ is an acid residue to give a compound of the formula:

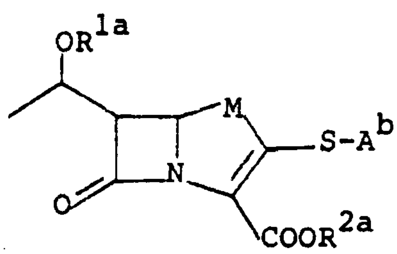

(I-b)

wherein $R^{1a}$, $R^{2a}$ and M are each as defined above and $A^b$ is either one of the groups (d) or (e);
(C) reacting a compound of the formula:

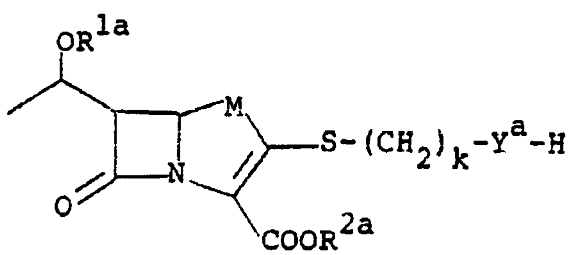

(VII)

wherein k, $R^{1a}$, $R^{2a}$ and M are each as defined above and $Y^a$ is an oxygen atom or -$NR^7$ (in which $R^7$ is as defined above) with a compound of the formula:

$R^4$-$X^c$ (V)

wherein $R^4$ and $X^c$ are each as defined above to give a compound of the formula:

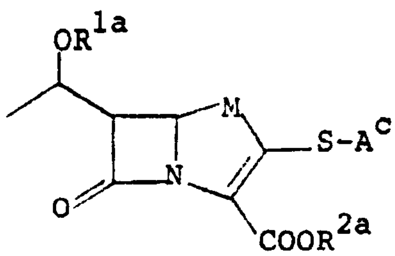

(I-c)

wherein $R^{1a}$, $R^{2a}$ and M are each as defined above and $A^c$ is either one of the groups (f) or (g);
(D) heating a compound of the formula:

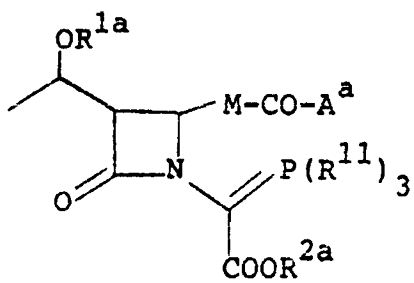

(VIII)

wherein $R^{1a}$, $R^{2a}$, M and $A^a$ are each as defined above and $R^{11}$ is a lower alkyl group, an aryl group or a lower alkoxy group to give a compound of the formula:

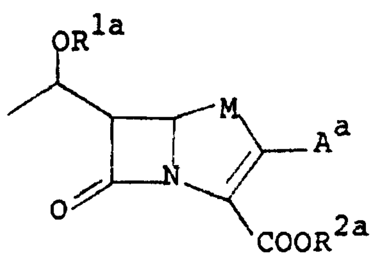

(I-d)

wherein $R^{1a}$, $R^{2a}$, M and $A^a$ are each as defined above;
(E) heating a compound of the formula:

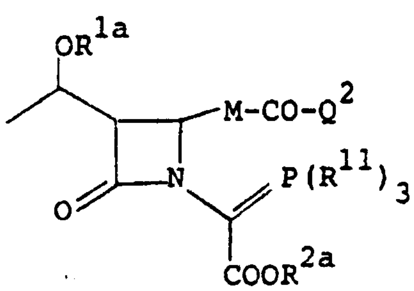

(IX)

wherein $R^{1a}$, $R^{2a}$, $R^{11}$, M and $Q^2$ are each as defined above to give a compound of the formula:

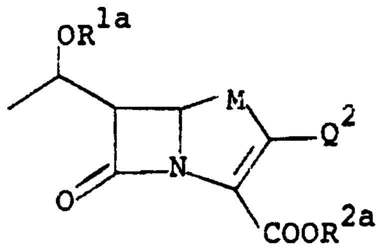

(X)

wherein $R^{1a}$, $R^{2a}$, M and $Q^2$ are each as defined above and reacting the latter with a compound of the formula:

$X^b-(CH_2)_q-R^4$ (VI)

wherein q, $R^4$ and $X^b$ are each as defined above to give a compound of the formula:

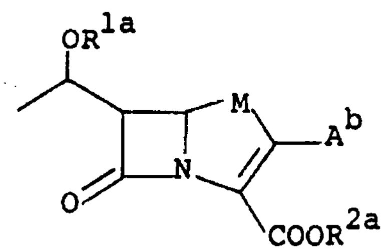

(I-e)

wherein $R^{1a}$, $R^{2a}$, M and $A^b$ are each as defined above;
(F) reacting a compound of the formula:

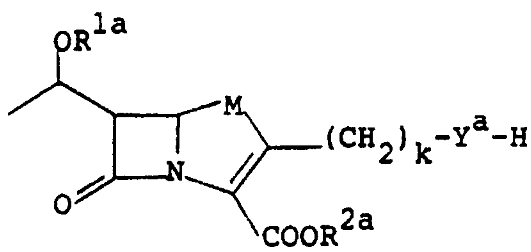

(XI)

wherein k, $R^{1a}$, $R^{2a}$ and M are each as defined above and $Y^a$ is an oxygen atom or $-NR^7$ (in which $R^7$ is as defined above) with a compound of the formula:

$R^4-X^c$ (V)

wherein $R^4$ and $X^c$ are each as defined above to give a compound of the formula:

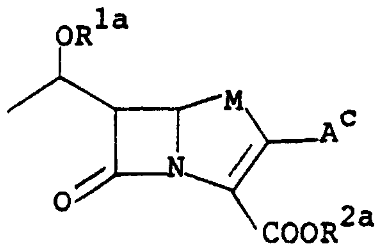

(I-f)

wherein $R^{1a}$, $R^{2a}$ and M are each as defined above and $A^c$ is either one of the groups (f) to (g);

(G) reacting the compound (II) with a mercaptan compound of the formula:

$A^d$-SH (III-c)

wherein $A^d$ is either one of the groups (h) to (n) to give a compound of the formula:

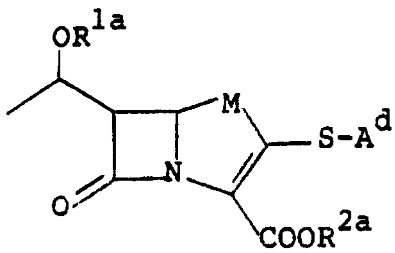

(XII)

wherein $R^{1a}$, $R^{2a}$, M and $A^d$ are each as defined above and, in case of $R^{1a}$ being a protective group for hydroxyl, then eliminating the protecting group and reacting the resultant compound of the formula:

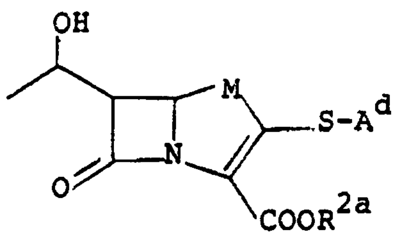

(XIII)

wherein $R^{2a}$, M and $A^d$ are each as defined above with a compound of the formula:

$R^{1b}$-$X^d$ (XIV)

wherein $R^{1b}$ is either one of the groups (1) to (3) and $X^d$ is an acid residue to give a compound of the formula:

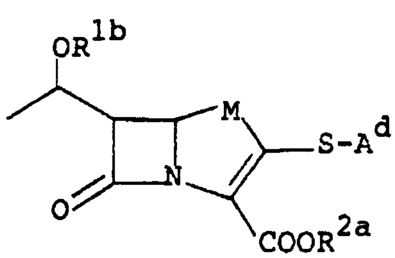

(I-g)

wherein $R^{1b}$, $R^{2a}$, M and $A^d$ are each as defined above;
(H) reacting a compound of the formula:

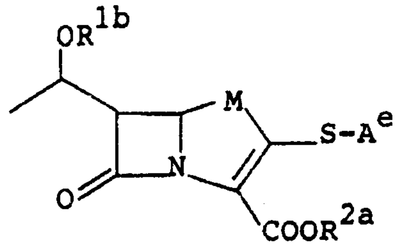

(I-g')

wherein $R^{1b}$, $R^{2a}$ and M are each as defined above and $A^e$ is either one of the groups (l) or (m) with a compound of the formula:

$R^{10}$-$X^e$ (XV)

wherein $R^{10}$ is as defined above and $X^e$ is an acid residue, or eliminating the amino-protecting group represented by $R^9$ and reacting the resultant compound with a $C_1$-$C_5$ alkyl ester of formimidic acid to give a compound of the formula:

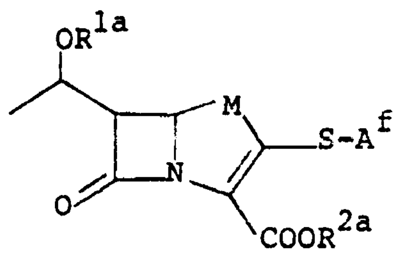

(I-h)

wherein $R^{1a}$, $R^{2a}$ and M are each as defined above and $A^f$ is either one of the groups (o) or (p);
(l) heating a compound of the formula:

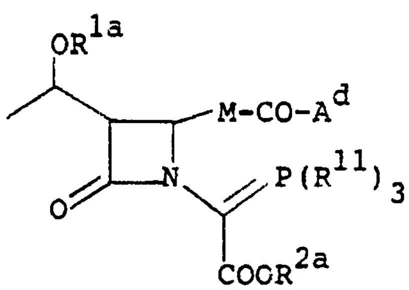

(XVI)

wherein $R^{1a}$, $R^{2a}$, M and $A^d$ are each as defined above and $R^{11}$ is a lower alkyl group or an aryl group or a lower alkoxy group to give a compound of the formula:

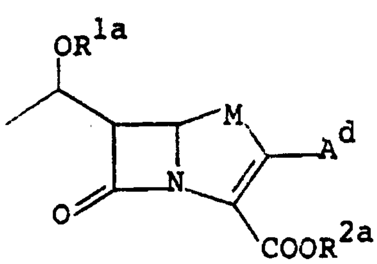

(XVII)

wherein $R^{1a}$, $R^{2a}$, M and $A^d$ are each as defined above and, in case of $R^{1a}$ being a protective group for hydroxyl, eliminating the protecting group, reacting the resultant compound of the formula:

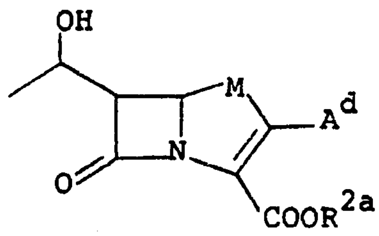

(XVIII)

wherein $R^{2a}$, M and $A^d$ are each as defined above with a compound of the formula:

$R^{1b}$-$X^d$ (XIV)

wherein $R^{1b}$ and $X^d$ are each as defined above to give a compound of the formula:

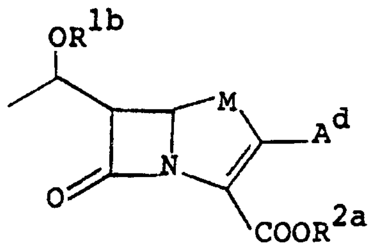

(I-i)

wherein $R^{1b}$, $R^{2a}$, M and $A^d$ are each as defined above; or
(J) reacting a compound of the formula:

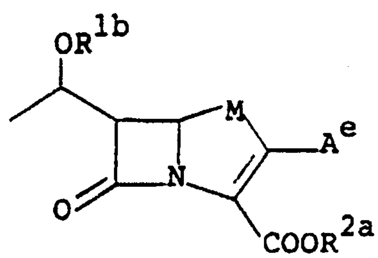

(I-i')

wherein $R^{1b}$, $R^{2a}$ and M are each as defined above and $A^e$ is either one of the groups (l) or (m) with a compound of the formula:

$R^{10}$-$X^e$ (XV)

wherein $R^{10}$ is as defined above and $X^e$ is an acid residue, or eliminating the amino-protecting group represented by $R^9$ and reacting the resultant compound with a $C_1$-$C_5$ alkyl ester of formimidic acid to give a compound of the formula:

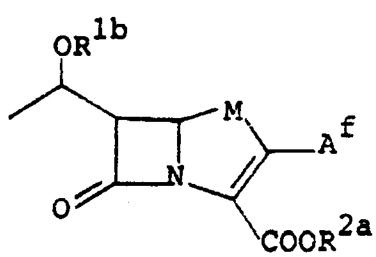

(I-j)

wherein $R^{1b}$, $R^{2a}$ and M are each as defined above and $A^f$ is either one of the groups (o) or (p), optionally followed by subjecting the compounds (I-a) to (I-j) to elimination of the carboxyl-protecting group represented by $R^{2a}$, the amino-protecting group represented by $R^8$ or $R^9$ and/or the hydroxyl-protecting group represented by $R^{1a}$, $R^5$ or $R^6$ to give the compound (I) wherein at least one of $R^8$, $R^9$, $R^{1a}$, $R^5$ and $R^6$ is a hydrogen atom and $R^{2a}$ is a hydrogen atom or a negative charge.

**14.** An antimicrobial composition which comprises an antimicrobially effective amount of the compound (I) according to any one of claims 1 - 12 or its pharmaceutically acceptable salt, and a pharmaceutically acceptable carrier or diluent.

**15.** A compound according to any one of claims 1 - 12 for use in the prevention or treatment of a bacterial infection in a living body.

**16.** The use of a compound according to any one of claims 1 - 12 for preparing an antimicrobial composition.

European Patent Office

# EUROPEAN SEARCH REPORT

Application Number

EP 91113384.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - B - 0 138 539 (PFIZER INC.) * Claims * | 1,14-16 | C 07 D 499/00<br>C 07 D 487/04<br>C 07 D 477/00<br>A 61 K 31/43 |
| A | EP - B - 0 070 204 (SUMITOMO PHARMACEUTICALS COMPANY, LIMITED) * Claims * | 1,3, 14-16 | |
| A | EP - B - 0 072 710 (SANKYO COMPANY LIMITED) * Claims * | 1,3, 14-16 | |
| A | EP - A - 0 161 541 (MERCK & CO. INC.) * Claims * | 1,14-16 | |
| A | EP - A - 0 168 707 (MERCK & CO. INC.) * Claims * | 1,14-16 | TECHNICAL FIELDS SEARCHED (Int. Cl.5) |
| A | CHEMICAL ABSTRACTS, vol. 111, no. 19, November 6, 1989, Columbus, Ohio, USA K. TAMURA et al. "Preparation of carbapenem compounds and their intermediates." page 704, abstract no. 173 901h & Jpn. Kokai Tokkyo Koho JP 01 93,586 (89 93,586) | 1,3 | C 07 D 499/00<br>C 07 D 487/00<br>C 07 D 477/00<br>C 07 D 519/00<br>A 61 K |
| A | CHEMICAL ABSTRACTS, vol. 110, no. 9, February 27, 1989, Columbus, Ohio, USA M. TAKEMURA et al. "Preparation of 6-(1-hydroxy-ethyl)-2-penem-3-carboxylate Derivatives as anti-bacterials." | 1,3, 14-16 | |

The present search report has been drawn up for all claims

| Place of search | Date of completion of the search | Examiner |
|---|---|---|
| VIENNA | 28-11-1991 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-
EP 91113384.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | page 617, abstract-no. 75 160a & Jpn. Kokai Tokkyo Koho JP 63,154,691 (88,154,691) -- | | |
| A | CHEMICAL ABSTRACTS, vol. 108, no. 17, April 25, 1988, Columbus, Ohio, USA SUMITOMO CHEMICAL CO., LTD. "Beta-Lactams" page 713, abstract-no. 150 152p & Jpn. Kokai Tokkyo Koho JP 60,233,076 (85,233,076) -- | 1,14-16 | |
| A | CHEMICAL ABSTRACTS, vol. 105, no. 11, September 15, 1986, Columbus, Ohio, USA M. SATO et al. "Penem-derivatives" page 636, abstract-no. 97 455z & Jpn. Kokai Tokkyo Koho JP 60,222,487 (85,222,487) -- | 1,3, 14-16 | TECHNICAL FIELDS SEARCHED (Int. Cl.5) |
| A | CHEMICAL ABSTRACTS, vol. 105, no. 7, August 18, 1986, Columbus, Ohio, USA T. MIYADERA et al. "Carbapenem-3-carboxylic acid derivatives." page 601, abstract-no. 60 472b & Jpn. Kokai Tokkyo Koho JP 60,260,580 (85,260,580) -- | 1,3, 14-16 | |
| A | CHEMICAL ABSTRACTS, vol. 104, no. 21, May 26, 1986, Columbus, Ohio, USA T. SHIBATA et al. "Synthetic | 1,3, 14-16 | |

The present search report has been drawn up for all claims

| Place of search | Date of completion of the search | Examiner |
|---|---|---|
| VIENNA | 28-11-1991 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application Number



EP 91113384.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | study of 1-substituted carbapenem antibiotics."<br>page 611, abstract-no. 186 187k<br>& Tetrahedron Lett. 1985, 26(39), 4739-42<br>-- | | |
| A | CHEMICAL ABSTRACTS, vol. 104, no. 21, May 26, 1986, Columbus, Ohio, USA<br>G. EMMER et al. "Syntheses and biological activities of new penem derivatives with side chains derived from 4-hydroxyproline."<br>page 611, abstract-no. 186 191g<br>& J. Antibiot. 1985, 38(10), 1371-86<br>---- | 1,3, 14-16 | |

TECHNICAL FIELDS SEARCHED (Int. Cl.5)

The present search report has been drawn up for all claims

| Place of search | Date of completion of the search | Examiner |
|---|---|---|
| VIENNA | 28-11-1991 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)